# EUROPÄISCHE PATENTSCHRIFT

(11) **EP 1 877 396 B1**
(45) Veröffentlichungstag und Bekanntmachung des Hinweises auf die Patenterteilung: **18.02.2009**
(21) Anmeldenummer: 06724361.8
(22) Anmeldetag: 15.04.2006
(51) Int. Cl.: C07D 401/14

(54) **DIPYRIDYL-DIHYDROPYRAZOLONE UND IHRE VERWENDUNG 4- (PYRIDIN-3-YL) -2- (PYRIDIN-2-YL) -1,2-DIHYDRO-3H-PYRAZOL-3-ON DERIVATE ALS SPEZIFISCHE HEMMSTOFFE DER HIF-PROLYL-4-HYDROXYLASEN ZUR BEHANDLUNG KARDIOVASKULÄRER UND HÄMATOLOGISCHER ERKRANKUNGEN**
4-(PYRIDIN-3-YL)-2-(PYRIDIN-2-YL)-1,2-DIHYDRO-3H-PYRAZOL-3-ONE DERIVATIVES AS SPECIFIC HIF-PROLYL-4-HYDROXYLASE INHIBITORS FOR TREATING CARDIOVASCULAR AND HAEMATOLOGICAL DISEASES
DERIVES DE 4-(PYRIDIN-3-YL)-2-(PYRIDIN-2-YL)-1,2-DIHYDRO-3H-PYRAZOL-3-ONE UTILISES EN TANT QU'INHIBITEURS SPECIFIQUES DES HIF-PROLYL-4-HYDROXYLASES POUR TRAITER DES MALADIES CARDIOVASCULAIRES ET HEMATOLOGIQUES

(30) Priorität: 28.04.2005 DE 102005019712
(43) Veröffentlichungstag der Anmeldung: 16.01.2008
(73) Patentinhaber: Bayer HealthCare AG, 51368 Leverkusen (DE)
(72) Erfinder: FLAMME, Ingo, 51580 Reichshof (DE); ERGÜDEN, Jens-Kerim, 42489 Wülfrath (DE); OEHME, Felix, 42115 Wuppertal (DE); THEDE, Kai, 42115 Wuppertal (DE); KARIG, Gunter, 51069 Köln (DE); KUHL, Alexander, 58093 Hagen (DE); WILD, Hanno, 42113 Wuppertal (DE); SCHUHMACHER, Joachim, 42113 Wuppertal (DE); KOLKHOF, Peter, 42113 Wuppertal (DE); BÄRFACKER, Lars, 46047 Oberhausen (DE); HÜTTER, Joachim, 42349 Wuppertal (DE)
(86) Internationale Anmeldenummer: PCT/EP2006/003488
(87) Internationale Veröffentlichungsnummer: WO 2006/114213

(56) Entgegenhaltungen:
- EP-A- 0 183 159
- WO-A-02/092573
- WO-A-20/04052284
- WO-A-20/04087066
- WO-A-20/05030121

## Beschreibung

Die vorliegende Anmeldung betrifft neue Dipyridyl-dihydropyrazolone, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Eine mangelhafte Versorgung des menschlichen Organismus oder seiner Teile mit Sauerstoff, die entweder durch ihre Dauer und/oder ihr Ausmaß eine regelrechte Funktion des Organismus oder seiner Teile beeinträchtigt oder ihre Funktion völlig zum Erliegen bringt, wird als Hypoxie bezeichnet. Eine Hypoxie kann verursacht werden durch eine Verminderung des verfügbaren Sauerstoffs in der Atemluft (z.B. bei Aufenthalten in großer Höhe), durch Störungen der äußeren Atmung (z.B. infolge gestörter Funktion der Lungen oder Verlegung der Atemwege), durch eine Verminderung des Herzzeitvolumens (z.B. bei einer Herzinsuffizienz, einer akuten Rechtsherzüberlastung bei Lungenembolie), durch eine zu geringe Sauerstoff-Transportkapazität des Blutes (z.B. infolge einer Blutarmut (Anämie) oder Intoxikation z.B. mit Kohlenmonoxid), örtlich begrenzt durch eine Minderdurchblutung infolge von Gefäßverschlüssen (Ischämie-Zustände typischerweise z.B. des Herzens, der unteren Extremitäten oder des Gehirns, diabetische Makro-und Mikroangiopathie) oder auch durch erhöhten Sauerstoffbedarf des Gewebes (z.B. infolge erhöhter Muskelarbeit oder lokaler Entzündungen) [Eder, Gedigk (Hrsg.), Allgemeine Pathologie und pathologische Anatomie, 33. Aufl., Springer Verlag, Berlin, 1990].

Der menschliche Organismus ist bedingt fähig, sich an Situationen verminderter Sauerstoffversorgung akut und chronisch anzupassen. Neben einer Sofortantwort, welche u.a. durch vegetativnervöse Kontrollmechanismen eine Erhöhung des Herzzeitvolumens und des Atemzeitvolumens sowie eine lokale Erweiterung von Blutgefäßen einschließt, zieht die Hypoxie eine Veränderung der Transkription zahlreicher Gene nach sich. Die Funktion der Genprodukte dient dabei der Kompensation des Sauerstoffmangels. So werden mehrere Enzyme der Glykolyse und der Glukosetransporter 1 verstärkt exprimiert, wodurch die anaerobe ATP-Gewinnung gesteigert und ein Überleben des Sauerstoffmangels ermöglicht wird [Schmidt, Thews (Hrsg.), Physiologie des Menschen, 27. Aufl., Springer Verlag, Berlin, 1997; Löffler, Petrides (Hrsg.), Biochemie und Pathobiochemie, 7. Aufl., Springer Verlag, Berlin, 2003].

Ferner führt Hypoxie zur verstärkten Expression des vaskulären Endothelzellwachstumsfaktors, VEGF, wodurch in hypoxischen Geweben die Neubildung von Blutgefäßen (Angiogenese) angeregt wird. Hierdurch wird langfristig die Durchblutung ischämischer Gewebe verbessert. Bei verschiedenen Herzkreislauferkrankungen und Gefäßverschluß-Erkrankungen erfolgt diese Gegenregulation offenbar nur sehr unzureichend [Übersicht bei: Simons und Ware, Therapeutic angiogenesis in cardiovascular disease, Nat. Rev. Drug. Discov. 2 (11), 863-71 (2003)].

Ferner wird bei systemischer Hypoxie das vorwiegend in den interstitiellen Fibroblasten der Nieren gebildete Peptidhormon Erythropoietin verstärkt exprimiert. Hierdurch wird die Bildung roter Blutzellen im Knochenmark angeregt und damit die Sauerstoff-Transportkapazität des Blutes gesteigert. Dieser Effekt wurde und wird von Leistungssportlern beim sogenannten Höhentraining genutzt. Eine Abnahme der Sauerstoff-Transportkapazität des Blutes z.B. infolge einer Blutungsanämie verursacht normalerweise eine Zunahme der Erythropoietin-Produktion in der Niere. Bei bestimmten Formen der Anämie kann dieser Regelmechanismus gestört oder sein Sollwert niedriger eingestellt sein. So wird z.B. bei Patienten, die unter einer Niereninsuffizienz leiden, Erythropoietin im Nierenparenchym zwar produziert, jedoch bezogen auf die Sauerstoff-Transportkapazität des Blutes in deutlich verminderten Mengen, was eine sogenannte renale Anämie zur Folge hat. Insbesondere die renale Anämie, aber auch Tumor- und HIV-Infektion-bedingte Anämien werden üblicherweise durch parenterale Verabfolgung von rekombinantem humanen Erythropoietin (rhEPO) behandelt. Derzeit existiert zu dieser kostspieligen Therapie keine alternative Therapie mit einem oral verfügbaren Arzneistoff [Übersichten bei: Eckardt, The potential of erythropoietin and related strategies to stimulate erythropoiesis, Curr. Opin. Investig. Drugs 2(8), 1081-5 (2001); Berns, Should the target hemoglobin for patients with chronic kidney disease treated with erythropoietic replacement therapy be changed?, Semin. Dial. 18 (1), 22-9 (2005)]. Jüngere Untersuchungen belegen, dass Erythropoetin neben seiner die Erythropoese steigernden Wirkung auch eine hiervon unabhängige, schützende (anti-apoptotische) Wirkung auf hypoxische Gewebe, insbesondere das Herz und das Gehirn ausübt. Ferner mindert eine Therapie mit Erythropoetin neueren Studien zufolge an herzinsuffizienten Patienten den durchschnittlichen Morbiditäts-Schweregrad [Übersichten bei: Caiola und Cheng, Use of erythropoietin in heart failure management, Ann. Pharmacother. 38 (12), 2145-9 (2004); Katz, Mechanisms and treatment of anemia in chronic heart failure, Congest. Heart. Fail. 10 (5), 243-7 (2004)].

Den oben beschriebenen durch Hypoxie induzierten Genen ist gemeinsam, dass die Steigerung ihrer Expression unter Hypoxie durch den sogenannten Hypoxie-induzierbaren Transkriptionsfaktor (HIF) hervorgerufen wird. Bei HIF handelt es sich um einen heterodimeren Transkriptionsfaktor, der aus einer alpha- und einer beta-Untereinheit besteht. Es wurden drei HIF-alpha-Isoformen beschrieben, von denen HIF-1 alpha und HIF-2 alpha hoch homolog und von Bedeutung für die Hypoxie-induzierte Genexpression sind. Während die auch als ARNT (aryl hydrocarbon receptor nuclear translocator) bezeichnete beta-Untereinheit (von der 2 Isoformen beschrieben wurden) konstitutiv exprimiert ist, hängt die Expression der alpha-Untereinheit vom Sauerstoffgehalt in der Zelle ab. Unter Normoxie wird das HIF-alpha-Protein poly-ubiquitiniert und anschließend proteasomal degradiert. Unter Hypoxie ist diese Degradierung gehemmt, so dass HIF-alpha mit ARNT dimerisieren und seine Zielgene aktivieren kann. Das HIF-Dimer bindet hierbei an sogenannte Hypoxie-responsible Elemente (HRE) in den regulatorischen Sequenzen seiner Zielgene. Die HRE sind durch eine Konsensus-Sequenz definiert. Funktionelle HRE wurden in den regulatorischen Elementen zahlreicher Hypoxie-induzierter Gene nachgewiesen [Übersichten bei: Semenza, Hypoxia-inducible factor 1: oxygen homeostasis and disease pathophysiology, Trends Mol. Med. 7 (8), 345-50 (2001); Wenger und Gassmann, Oxygen(es) and the hypoxia-inducible factor-1, Biol. Chem. 378 (7), 609-16 (1997)].

Der molekulare Mechanismus, der dieser Regulation von HIF-alpha zugrunde liegt, wurde durch die Arbeiten mehrerer voneinander unabhängiger Forschergruppen aufgeklärt. Der Mechanismus ist Spezies-übergreifend konserviert: HIF-alpha wird durch eine als PHD oder EGLN bezeichnete Subklasse von Sauerstoff-abhängigen Prolyl-4-Hydroxylasen an zwei spezifischen Prolyl-Resten hydroxyliert (P402 und P564 der humanen HIF-1-alpha-Untereinheit). Bei den HIF-Prolyl-4-Hydroxylasen handelt es sich um Eisen-abhängige, 2-Oxoglutarat-umsetzende Dioxygenasen [Epstein et al., C. elegans EGL-9 and mammalian homologs define a family of dioxygenases that regulate HIF by prolyl hydroxylation, Cell 107 (1), 43-54 (2001); Bruick und McKnight, A conserved family of prolyl-4-hydroxylases that modify HIF, Science 294 (5545), 1337-40 (2001); Ivan et al., Biochemical purification and pharmacological inhibition of a mammalian prolyl hydroxylase acting on hypoxia-inducible factor, Proc. Natl. Acad. Sci. U.S.A. 99 (21), 13459-64 (2002)]. Die Enzyme wurden 2001 erstmals als Prolyl-Hydroxylasen annotiert [Aravind und Koonin, The DNA-repair protein AlkB, EGL-9, and leprecan define new families of 2-oxoglutarate- and irondependent dioxygenases, Genome Biol. 2 (3), research0007.1-0007.8, Epub 2001 Feb 19].

An die prolylhydroxylierte HIF-alpha-Untereinheit bindet das pVHL Tumor Suppressor Protein, welches zusammen mit Elongin B und C den sogenannten VBC-Komplex bildet, der die HIF-alpha-Untereinheit an eine E3 Ubiquitin-Ligase adaptiert. Da die Prolyl-4-Hydroxylierung der HIF-alpha-Untereinheit und ihre nachfolgende Degradierung in Abhängigkeit von der intrazellulären Konzentration des Sauerstoffs erfolgt, wurden die HIF-Prolyl-4-Hydroxylasen auch als zellulärer Sauerstoffsensor bezeichnet. Es wurden drei Isoformen dieser Enzyme identifiziert: EGLN1/PHD2, EGLN2/PHD1 und EGLN3/PHD3. Zwei dieser Enzyme (EGLN2/PHD1 und EGLN3/PHD3) werden selbst unter Hypoxie transkriptionell induziert und sind möglicherweise für die unter chronischer Hypoxie zu beobachtende Absenkung der HIF-alpha-Spiegel verantwortlich [Übersicht bei: Schofield und Ratcliffe, Oxygen sensing by HIF hydroxylases, Nat. Rev. Mol. Cell. Biol. 5 (5), 343-54 (2004)].

Eine selektive pharmakologische Hemmung der HIF-Prolyl-4-Hydroxylasen zieht die Steigerung der Genexpression HIF-abhängiger Zielgene nach sich und ist damit für die Therapie zahlreicher Krankheitsbilder von Nutzen. Insbesondere bei Erkrankungen des Herz-Kreislaufsystems ist von der Induktion neuer Blutgefäße sowie der Änderung der Stoffwechselsituation ischämischer Organe von aerober hin zu anaerober ATP-Gewinnung eine Besserung des Krankheitsverlaufs zu erwarten. Eine Verbesserung der Vaskularisierung chronischer Wunden fördert den Heilungsprozess, insbesondere bei schwer heilenden Ulcera cruris und anderen chronischen Hautwunden. Die Induktion von körpereigenem Erythropoietin bei bestimmten Krankheitsformen, insbesondere bei Patienten mit renaler Anämie, ist ebenfalls ein anzustrebendes therapeutisches Ziel.

Die bislang in der wissenschaftlichen Literatur beschriebenen HIF-Prolyl-4-Hydroxylase-Inhibitoren erfüllen nicht die an ein Arzneimittel zu stellenden Anforderungen. Es handelt sich hierbei entweder um kompetitive Oxoglutarat-Analoga (wie z.B. N-Oxalylglycin), die durch ihre sehr geringe Wirkstärke charakterisiert sind und daher in *in vivo*-Modellen bislang keine Wirkung im Sinne einer Induktion von HIF-Zielgenen gezeigt haben. Oder es handelt sich um Eisen-Komplexbildner (Chelatoren) wie Desferroxamin, die als unspezifische Hemmstoffe Eisen-haltiger Dioxygenasen wirken und, obwohl sie eine Induktion der Zielgene wie z.B. Erythropoetin *in vivo* herbeiführen, durch Komplexierung des verfügbaren Eisens offenbar der Erythropoese entgegenwirken.

Aufgabe der vorliegenden Erfindung ist die Bereitstellung neuer Verbindungen, die für die Behandlung von Erkrankungen, insbesondere kardiovaskulärer und hämatologischer Erkrankungen, eingesetzt werden können.

Im Rahmen der vorliegenden Erfindung werden nunmehr Verbindungen beschrieben, die als spezifische Hemmstoffe der HIF-Prolyl-4-Hydroxylasen wirken und aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbeiführen.

2-Heteroaryl-4-aryl-1,2-dihydropyrazolone mit bakterizider und/oder fungizider Wirkung werden in EP 165 448 und EP 212 281 offenbart. Die Verwendung von 2-Heteroaryl-4-aryl-1,2-dihydropyrazolonen als Lipoxygenase-Inhibitoren zur Behandlung von Atemwegs-, Herz-Kreislauf und Entzündungserkrankungen wird in EP 183 159 beansprucht. 2,4-Diphenyl-1,2-dihydropyrazolone mit herbizider Aktivität werden in DE 2 651 008 beschrieben. Über die Herstellung und pharmakologischen Eigenschaften bestimmter 2-Pyridyl-1,2-dihydropyrazolone wird in Helv. Chim. Acta 49 (1), 272-280 (1966) berichtet. In WO 96/12706, WO 00/51989 und WO 03/074550 werden Verbindungen mit Dihydropyrazolon-Partialstruktur zur Behandlung unterschiedlicher Erkrankungen beansprucht.

Gegenstand der vorliegenden Erfindung sind Verbindungen der allgemeinen Formel (I) 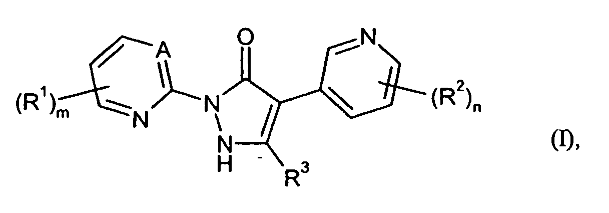 in welcher
- A: für CH oder N steht,
- R¹: für einen Substituenten ausgewählt aus der Reihe (C₁-C₆)-Alkyl, Trifluormethyl, Halogen, Cyano, Nitro, Hydroxy, (C₁-C₆)-Alkoxy, Amino, (C₁-C₆)-Alkoxycarbonyl, Hydroxycarbonyl und -C(=O)-NH-R⁴ steht, worin
(C₁-C₆)-Alkyl seinerseits mit Hydroxy, (C₁-C₄)-Alkoxy, Amino, Mono-(C₁-C₄)-alkylamino, Di-(C₁-C₄)-alkylamino oder einer Gruppe der Formel -NH-C(=O)-R⁵, -NH-C(=O)-NH-R⁶ oder -NH-SO₂R⁷ substituiert sein kann, worin
R⁵ (C₁-C₆)-Alkyl, das mit Hydroxy, (C₁-C₄)-Alkoxy, Phenyl oder 5- oder 6-gliedrigem Heteroaryl substituiert sein kann, oder Phenyl bedeutet,
wobei Phenyl und Heteroaryl ihrerseits jeweils ein- bis dreifach, gleich oder verschieden, mit Halogen, Cyano, (C₁-C₄)-Alkyl, Hydroxy, (C₁-C₄)-Alkoxy, Trifluormethyl oder Trifluormethoxy substituiert sein können,
R⁶ (C₁-C₆)-Alkyl, das mit Hydroxy oder (C₁-C₄)-Alkoxy substituiert sein kann, bedeutet
und
R⁷ (C₁-C₆)-Alkyl bedeutet, und
- R⁴: Wasserstoff oder (C₁-C₆)-Alkyl, das mit Hydroxy, (C₁-C₄)-Alkoxy oder Phenyl substituiert sein kann, bedeutet,
wobei Phenyl seinerseits mit Halogen, Cyano, (C₁-C₄)-Alkyl, (C₁-C₄)-Alkoxy, Trifluormethyl oder Trifluormethoxy substituiert sein kann,
- R²: für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Hydroxycarbonyl und -C(=O)-NH-R⁸ steht, worin
(C₁-C₆)-Alkyl und (C₁-C₆)-Alkoxy ihrerseits mit Hydroxy substituiert sein können
und
R⁸ Wasserstoff oder (C₁-C₄)-Alkyl bedeutet,
- m: für die Zahl 0, 1 oder 2 steht,
- n: für die Zahl 0, 1, 2 oder 3 steht,
wobei für den Fall, dass Rinder R² mehrfach auftreten, ihre Bedeutungen jeweils gleich oder verschieden sein können,
und
- R³: für Wasserstoff, (C₁-C₆)-Alkyl oder (C₃-C₇)-Cycloalkyl steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

(C₁-C₆-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.

(C₃-C₇)-Cycloalkyl und (C₃-C₆)-Cycloalkyl stehen im Rahmen der Erfindung für eine gesättigte monocyclische Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

(C₁-C₆-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy und tert.-Butoxy.

(C₁-C₆)-Alkoxycarbonyl und (C₁C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Mono- (C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, n-Propylamino, Isopropylamino, n-Butylamino und tert.-Butylamino.

Di-(C₁C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-methylamino, *N,N*-Diisopropylamino, *N*-n-Butyl-*N*-methylamino, *N*-tert.-Butyl-*N*-methylamino.

5- oder 6-gliedriges Heteroaryl steht im Rahmen der Erfindung für einen aromatischen Heterocyclus (Heteroaromaten) mit insgesamt 5 oder 6 Ringatomen und bis zu drei gleichen oder verschiedenen Ring-Heteroatomen aus der Reihe N, O und/oder S, der über ein Ring-Kohlenstoffatom oder gegebenenfalls ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl. Bevorzugt ist ein 5-gliedriger Heteroaryl-Rest mit bis zu zwei Ring-Heteroatomen aus der Reihe N, O und/oder S wie beispielsweise Furyl, Pyrrolyl, Thienyl, Pyrazolyl, Imidazolyl, Thiazolyl, Oxazolyl, Isoxazolyl, Isothiazolyl.

Halogen schließt im Rahmen der Erfindung Fluor, Chlor, Brom und Iod ein. Bevorzugt sind Fluor, Chlor oder Brom.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- A: für CH oder N steht,
- R¹: für einen Substituenten ausgewählt aus der Reihe (C₁-C₆)-Alkyl, Trifluormethyl, Cyano, Nitro, Hydroxy, (C₁-C₆)-Alkoxy, Amino, (C₁-C₆)-Alkoxycarbonyl und Hydroxycarbonyl steht,
- R²: für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino und Hydroxycarbonyl steht, worin (C₁-C₆)-Alkyl und (C₁-C₆)-Alkoxy ihrerseits mit Hydroxy substituiert sein können,
- m: für die Zahl 0, 1 oder 2 steht,
- n: für die Zahl 0, 1, 2 oder 3 steht,
wobei für den Fall, dass R¹ oder R² mehrfach auftreten, ihre Bedeutungen jeweils gleich oder verschieden sein können,
und
- R³: für Wasserstoff, (C₁-C₆)-Alkyl oder (C₃-C₇)-Cycloalkyl steht,
sowie ihre Salze, Solvate und Solvate der Salze.

Gleichfalls bevorzugt sind Verbindungen der Formel (I), in welcher
- A: für CH steht,
- R¹: für einen Substituenten ausgewählt aus der Reihe (C₁-C₆)-Alkyl, Fluor, Chlor, Brom und -C(=O)-NH-R⁴ steht, worin (C₁-C₆)-Alkyl seinerseits mit Hydroxy, (C₁-C₄)-Alkoxy, Amino, Mono-(C₁C₄)-alkylamino, Di-(C₁-C₄)-alkylamino oder einer Gruppe der Formel -NH-C(=O)-R⁵, -NH-C(=O)-NH-R⁶ oder -NH-SO₂R⁷ substituiert sein kann, worin
R⁵ (C₁-C₆)-Alkyl, das mit Hydroxy, Methoxy, Ethoxy, Phenyl oder 5-gliedrigem Heteroaryl substituiert sein kann, oder Phenyl bedeutet,
wobei Phenyl und Heteroaryl ihrerseits jeweils ein- bis dreifach, gleich oder verschieden, mit Fluor, Chlor, Brom, Cyano, Methyl, Hydroxy, Methoxy, Ethoxy, Trifluormethyl oder Trifluormethoxy substituiert sein können,
und
R⁶ und R⁷ unabhängig voneinander (C₁-C₆)-Alkyl bedeuten, und
- R⁴: (C₁-C₆)-Alkyl, das mit Hydroxy, Methoxy, Ethoxy oder Phenyl substituiert sein kann, bedeutet,
wobei Phenyl seinerseits mit Fluor, Chlor, Brom, Cyano, Methyl, Methoxy, Ethoxy, Trifluormethyl oder Trifluormethoxy substituiert sein kann,
- R²: für einen Substituenten ausgewählt aus der Reihe Fluor, Chlor, Brom, Cyano, (C₁-C₆)-Alkyl, Trifluormethyl, Hydroxycarbonyl und -C(=O)-NH-R⁸ steht, worin
(C₁-C₆)-Alkyl seinerseits mit Hydroxy substituiert sein kann
und
R⁸ (C₁-C₄)-Alkyl bedeutet,
- m: für die Zahl 0, 1 oder 2 steht,
n für die Zahl 0, 1 oder 2 steht,
wobei für den Fall, dass R¹ oder R² mehrfach auftreten, ihre Bedeutungen jeweils gleich oder verschieden sein können, und
- R³: für Wasserstoff steht,
sowie ihre Salze, Solvate und Solvate der Salze.

Besonders bevorzugt sind Verbindungen der Formel (I), in welcher
- A: für CH steht,
- R¹: für einen Substituenten ausgewählt aus der Reihe (C₁-C₄)-Alkyl, Trifluormethyl, Nitro, (C₁-C₄)-Alkoxy, Amino und (C₁-C₄)-Alkoxycarbonyl steht,

- R²: für einen Substituenten ausgewählt aus der Reihe Chlor, Brom, Cyano, (C₁-C₄)-Alkyl, Trifluormethyl, Hydroxy, (C₁-C₄)-Alkoxy, Trifluormethoxy und Amino steht, worin (C₁-C₄)-Alkyl und (C₁-C₄)-Alkoxy ihrerseits mit Hydroxy substituiert sein können,
- m: für die Zahl 0 oder 1 steht,
- n: für die Zahl 0, 1, 2 oder 3 steht,
wobei für den Fall, dass R² mehrfach auftritt, seine Bedeutungen gleich oder verschieden sein können,
und
- R³: für Wasserstoff oder Methyl steht,
sowie ihre Salze, Solvate und Solvate der Salze.

Gleichfalls besonders bevorzugt sind Verbindungen der Formel (I), in welcher
- A: für CH steht,
- R¹: für einen Substituenten ausgewählt aus der Reihe (C₁-C₄)-Alkyl, Fluor, Chlor, Brom und -C(=O)-NH-R⁴ steht, worin
(C₁-C₄)-Alkyl seinerseits mit Hydroxy, Amino oder einer Gruppe der Formel -NH-C(=O)-R⁵ oder -NH-C(=O)-NH-R⁶ substituiert sein kann, worin
R⁵ (C₁-C₄)-Alkyl, das mit Phenyl oder Pyrazolyl substituiert sein kann, oder Phenyl bedeutet,
wobei Phenyl und Pyrazolyl ihrerseits jeweils ein- bis dreifach, gleich oder verschieden, mit Fluor, Chlor, Methyl oder Trifluormethyl substituiert sein können, und
- R⁶: (C₁-C₄)-Alkyl bedeutet,
und
- R⁴: (C₁-C₄)-Alkyl, das mit Phenyl substituiert sein kann, bedeutet,
- R²: für einen Substituenten ausgewählt aus der Reihe Chlor, Brom, Cyano, (C₁-C₄)-Alkyl und Trifluormethyl steht, worin (C₁-C₄)-Alkyl seinerseits mit Hydroxy substituiert sein kann,
- m: für die Zahl 0, 1 oder 2 steht,
- n: für die Zahl 0, 1 oder 2 steht,
wobei für den Fall, dass R¹ oder R² mehrfach auftreten, ihre Bedeutungen jeweils gleich oder verschieden sein können,
und
- R³: für Wasserstoff steht,
sowie ihre Salze, Solvate und Solvate der Salze.

Von besonderer Bedeutung sind Verbindungen der Formel (I-A) 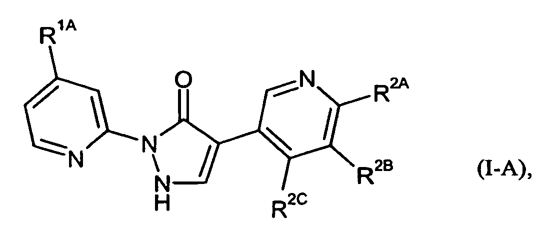 in welcher
- R^{1A}: für Wasserstoff, Methyl oder Trifluormethyl steht
und
- R^{2A} , R^{2B} und R^{2C}: gleich oder verschieden sind und unabhängig voneinander für Wasserstoff, Chlor, Brom, Cyano, Methyl, Hydroxymethyl, Methoxy oder Ethoxy stehen,
sowie ihre Salze, Solvate und Solvate der Salze.

Von besonderer Bedeutung sind ebenso Verbindungen der Formel (I-B) 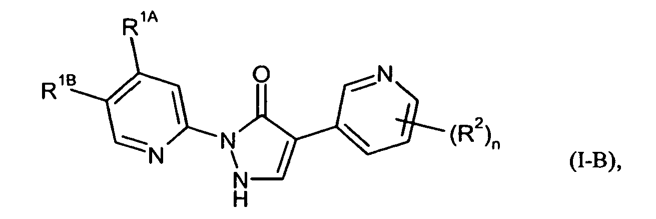 in welcher
- R^{1A} und R^{1B}: gleich oder verschieden sind und unabhängig voneinander für Wasserstoff, Fluor, Chlor, (C₁-C₄)-Alkyl oder -C(=O)-NH-R⁴ stehen, worin
(C₁-C₄)-Alkyl seinerseits mit Hydroxy, Amino oder einer Gruppe der Formel -NH-C(=O)-R⁵ substituiert sein kann, worin
R⁵ (C₁-C₄)-Alkyl, das mit Phenyl oder Pyrazolyl substituiert sein kann, oder Phenyl bedeutet,
wobei Phenyl und Pyrazolyl ihrerseits jeweils ein- bis dreifach, gleich oder verschieden, mit Fluor, Chlor, Methyl oder Trifluormethyl substituiert sein können, und
- R⁴: (C₁-C₄)-Alkyl, das mit Phenyl substituiert sein kann, bedeutet,
- R²: für einen Substituenten ausgewählt aus der Reihe Chlor, Brom, Cyano, Methyl, Hydroxymethyl oder Trifluormethyl steht
und
- n: für die Zahl 0, 1 oder 2 steht,
wobei für den Fall, dass R² mehrfach auftritt, seine Bedeutungen gleich oder verschieden sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,2-Dihydropyrazol-3-on-Derivate der Formel (I) können auch in der tautomeren 1*H*-Pyrazol-5-ol-Form (I') vorliegen (siehe nachfolgendes Schema 1); beide tautomeren Formen werden von der vorliegenden Erfindung ausdrücklich umfasst.

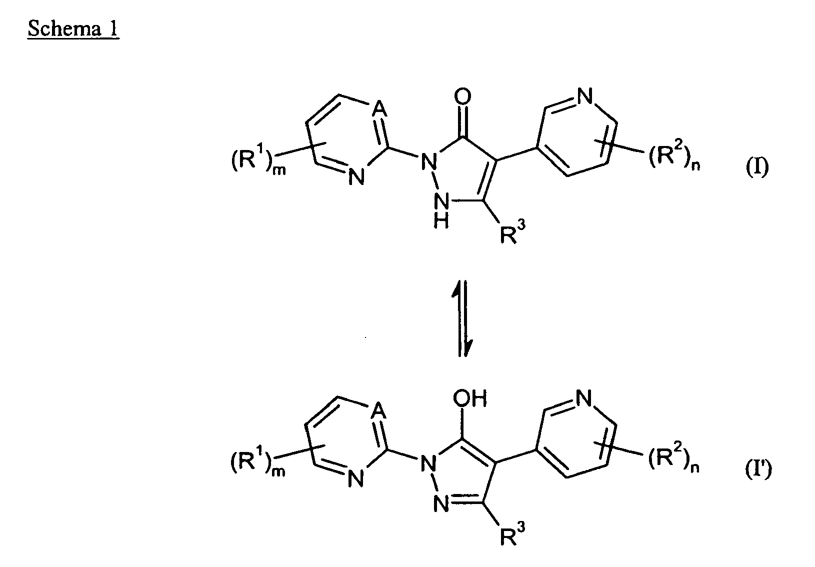

Weiterer Gegenstand der Erfindung ist ein Verfahren zur Herstellung der erfindungsgemäßen Verbindungen der Formel (I), dadurch gekennzeichnet, dass man Verbindungen der Formel (II) 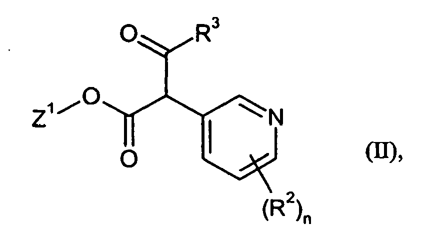 in welcher R², R³ und n die oben angegebenen Bedeutungen aufweisen und
- Z¹: für Methyl oder Ethyl steht,
in einem inerten Lösungsmittel gegebenenfalls in Gegenwart einer Säure mit einer Verbindung der Formel (III) 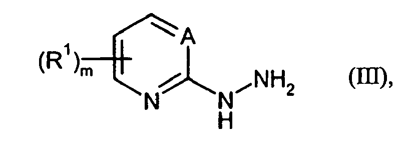 in welcher A, R¹ und m die oben angegebenen Bedeutungen aufweisen,
zu Verbindungen der Formel (IV) 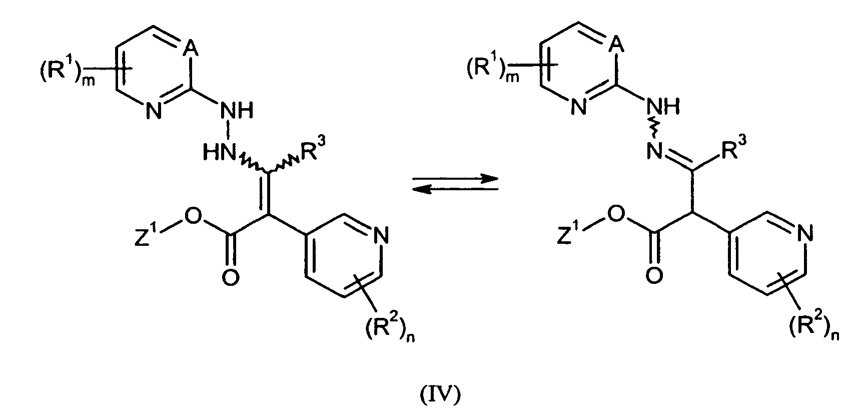 in welcher Z¹, A, R¹, R², R³, m und n die oben angegebenen Bedeutungen aufweisen,
umsetzt und diese dann in einem inerten Lösungsmittel in Gegenwart einer Base cyclisiert
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

Die erfindungsgemäßen Verbindungen der Formel (I), in welcher R³ für Wasserstoff steht, können auch hergestellt werden, indem man zunächst Verbindungen der Formel (V) 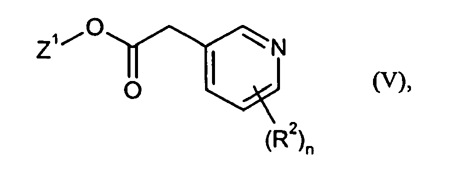 in welcher Z¹, R² und n die oben angegebenen Bedeutungen aufweisen,
mit einer Verbindung der Formel (VI) 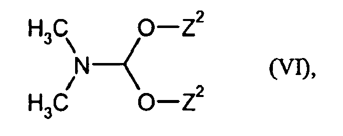 in welcher
- Z²: für Methyl oder Ethyl steht,
zu Verbindungen der Formel (VII) 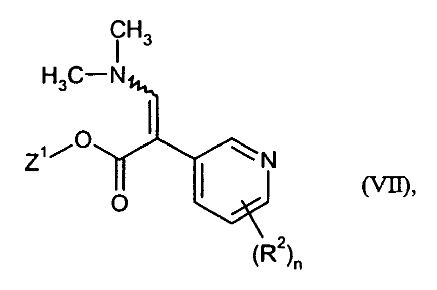 in welcher Z¹, R² und n die oben angegebenen Bedeutungen aufweisen,
kondensiert, anschließend in Gegenwart einer Säure mit einer Verbindung der Formel (III) zu Verbindungen der Formel (IV-A) 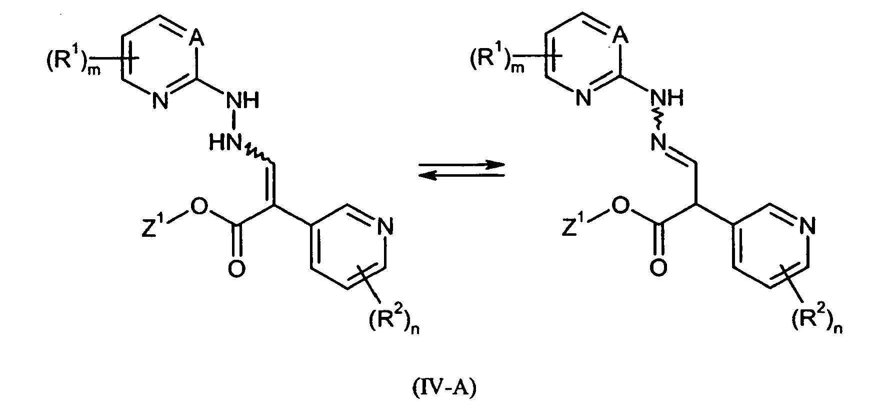 in welcher Z¹, A, R¹, R², m und n die oben angegebenen Bedeutungen aufweisen,
umsetzt und diese dann in einem inerten Lösungsmittel in Gegenwart einer Base cyclisiert.

Die erfindungsgemäßen Verbindungen können gegebenenfalls auch hergestellt werden durch weitere Umwandlungen von funktionellen Gruppen einzelner Substituenten, insbesondere den unter R¹ und R² aufgeführten, ausgehend von den nach obigem Verfahren erhaltenen Verbindungen der Formel (I). Diese Umwandlungen werden nach üblichen Methoden durchgeführt und umfassen beispielsweise Reaktionen wie nukleophile oder elektrophile Substitution, Oxidation, Reduktion, Hydrierung, Veresterung, Esterspaltung, Veretherung, Etherspaltung, Bildung von Carbonamiden, Sulfonamiden und Harnstoffen, sowie die Einführung und Entfernung temporärer Schutzgruppen.

Als inerte Lösungsmittel für die Verfahrensschritte (II) + (III) → (IV), (IV) → (I) und (IV-A) → (I) eignen sich insbesondere Alkohole wie Methanol, Ethanol, n-Propanol, iso-Propanol, n-Butanol oder tert.-Butanol. Bevorzugt wird Ethanol verwendet.

Der Verfahrensschritt (II) + (III) → (IV) kann gegebenenfalls vorteilhaft unter Zusatz einer Säure durchgeführt werden. Hierfür eignen sich übliche anorganische oder organische Säuren wie beispielsweise Chlorwasserstoff, Essigsäure, Trifluoressigsäure, Methansulfonsäure oder para-Toluolsulfonsäure. Bevorzugt wird Essigsäure verwendet.

Die Umsetzung (II) + (III) → (IV) erfolgt im Allgemeinen in einem Temperaturbereich von 0°C bis +100°C, bevorzugt von +10°C bis +40°C.

Als Base für den Cyclisierungsschritt (IV) → (I) bzw. (IV-A) → (I) eignen sich übliche anorganische oder organische Basen. Hierzu gehören insbesondere Alkalihydroxide wie beispielsweise Natrium- oder Kaliumhydroxid, Alkali- oder Erdalkalicarbonate wie Natrium-, Kalium-, Calcium-oder Cäsiumcarbonat, Alkali-Alkoholate wie Natrium- oder Kaliummethanolat, Natrium- oder Kaliumethanolat oder Natrium- oder Kalium-tert.-butylat, oder Alkalihydride wie Natriumhydrid. Bevorzugt wird Natriumethanolat verwendet.

Die Umsetzung (IV) → (I) bzw. (IV-A) → (I) erfolgt im Allgemeinen in einem Temperaturbereich von 0°C bis +60°C, bevorzugt von 0°C bis +30°C.

Die Verfahrenssequenz (II) + (III) → (IV) → (I) kann unter zweistufiger Reaktionsführung oder auch als Eintopf-Reaktion, ohne Isolierung der Zwischenstufe (IV), durchgeführt werden.

Der Verfahrensschritt (V) + (VI) → (VII) wird bevorzugt ohne Lösungsmittel in Gegenwart eines Überschusses an (VI) unter Mikrowellen-Bestrahlung durchgeführt. Die Umsetzung erfolgt im Allgemeinen in einem Temperaturbereich von +20°C bis +150°C, bevorzugt von +80°C bis +120°C [vgl. auch J.P. Bazureau et al., Synthesis 1998, 967; ibid. 2001 (4), 581].

Der Verfahrensschritt (VII) + (III) → (IV-A) wird vorteilhafterweise unter Zusatz einer Säure durchgeführt. Hierfür eignen sich übliche anorganische oder organische Säuren wie beispielsweise Chlorwasserstoff, Essigsäure, Trifluoressigsäure, Methansulfonsäure oder para-Toluolsulfonsäure. Bevorzugt wird Essigsäure verwendet. Als inerte Lösungsmittel für diesen Verfahrensschritt können Alkohole wie Methanol, Ethanol, n-Propanol, iso-Propanol, n-Butanol oder tert.-Butanol eingesetzt werden. Besonders bevorzugt wird die Umsetzung in Essigsäure selbst, ohne Zusatz eines weiteren Lösungsmittels, durchgeführt.

Die Umsetzung (VII) + (III) → (IV-A) erfolgt im Allgemeinen in einem Temperaturbereich von 0°C bis +60°C, bevorzugt von +10°C bis +30°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können nach literaturbekannten Methoden zur C-Acylierung von Carbonsäureestern aus Verbindungen der Formel (V) hergestellt werden. Die Verbindungen der Formeln (III), (V) und (VI) sind kommerziell erhältlich, literaturbekannt oder können in Analogie zu literaturbekannten Verfahren hergestellt werden.

Die Herstellung der erfindungsgemäßen Verbindungen kann durch die folgenden Syntheseschemata 2-4 veranschaulicht werden:

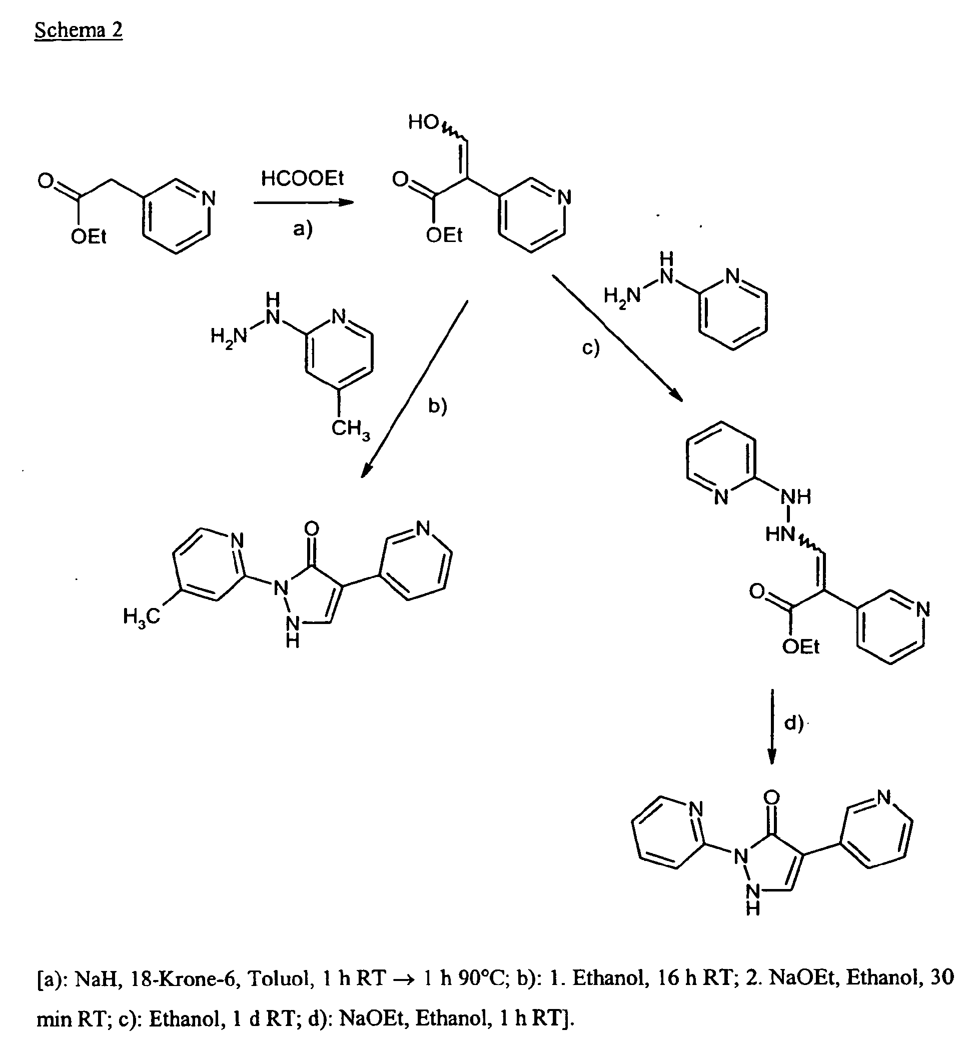

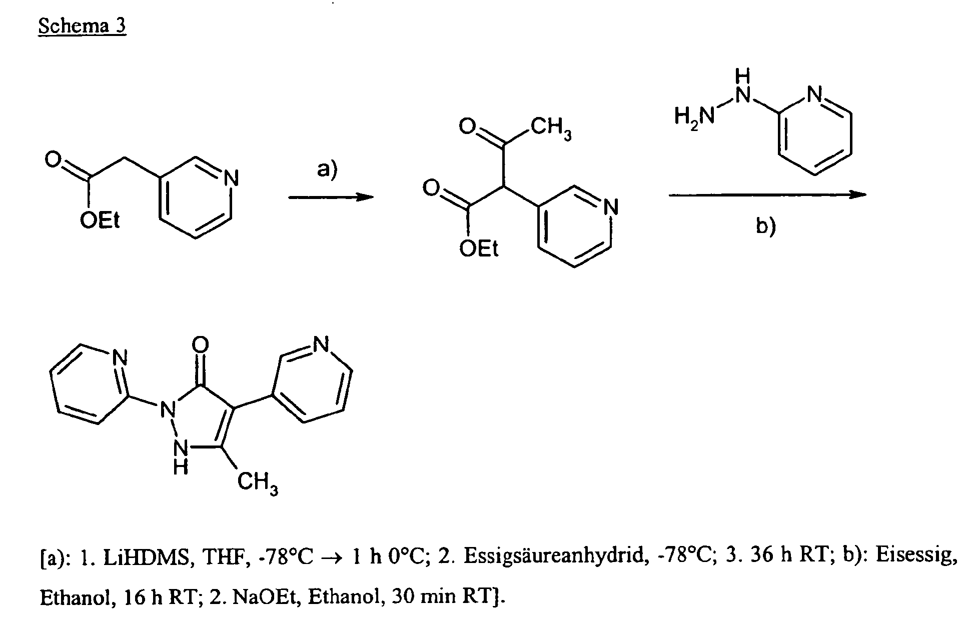

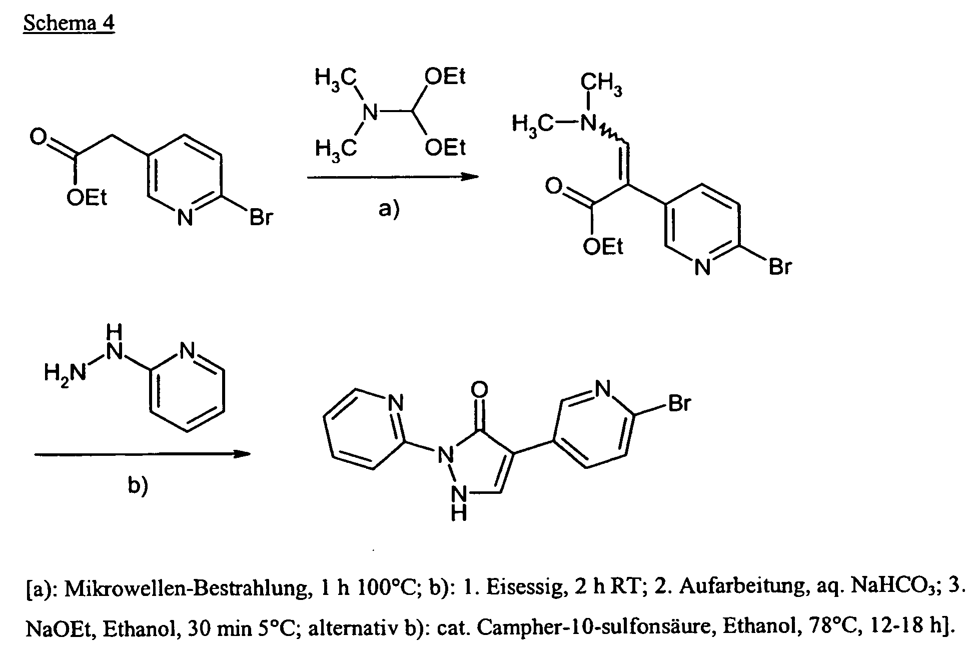

Die erfindungsgemäßen Verbindungen zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen zeichnen sich als spezifische Hemmstoffe der HIF-Prolyl-4-Hydroxylasen aus.

Die erfindungsgemäßen Verbindungen können aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden. Die Verbindungen eignen sich ferner zur Behandlung und/oder Prophylaxe von Störungen der Blutbildung, wie z.B. idiopathischen Anämien, renaler Anämie, Anämien in Begleitung einer Tumor-Erkrankung, einer Infektion oder einer anderen entzündlichen Erkrankung wie z.B. rheumatoider Arthritis.

Die Verbindungen sind ferner geeignet zur Steigerung des Hämatokrits mit dem Ziel der Gewinnung von Blut zur Eigenblutspende vor Operationen.

Die erfindungsgemäßen Verbindungen können außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die Verbindungen eignen sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die Verbindungen können ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die Verbindungen eignen sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin können die erfindungsgemäßen Verbindungen eingesetzt werden zur Behandlung und/oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignen sich die Verbindungen zur Behandlung und/oder Prophylaxe einer insbesondere im höheren Lebensalter gehäuft auftretenden allgemeinen körperlichen Schwäche bis hin zur Kachexie.

Ferner eignen sich die Verbindungen zur Behandlung und/oder Prophylaxe sexueller Dysfunktion.

Außerdem sind die Verbindungen zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignen sich die erfindungsgemäßen Verbindungen zur Behandlung und/oder Prophylaxe von fibrotischen Erkrankungen z.B. des Herzens, der Lunge und der Leber.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Herstellung eines Arzneimittels zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Mineralocorticoid-Rezeptor-Antagonisten, Aspirin, Diuretika, Eisen-Supplements, Vitamin B12- und Folsäure-Supplements, Calcium-Antagonisten, Statine und Digitalis (Digoxin)-Derivate.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

| | |
|---|---|
| aq. | wässrige Lösung |
| cat. | katalytisch |
| d | Tag(e) |
| DCI | direkte chemische Ionisation (bei MS) |
| DMF | Dimethylformamid |
| DMSO | Dimethylsulfoxid |
| d. Th. | der Theorie (bei Ausbeute) |
| EI | Elektronenstoß-Ionisation (bei MS) |
| ESI | Elektrospray-Ionisation (bei MS) |
| Et | Ethyl |
| GC-MS | Gaschromatographie-gekoppelte Massenspektroskopie |
| h | Stunde(n) |
| HPLC | Hochdruck-, Hochleistungsflüssigchromatographie |
| konz. | konzentriert |
| LC-MS | Flüssigchromatographie-gekoppelte Massenspektroskopie |
| LiHMDS | Lithiumhexamethyldisilazid |
| min | Minute(n) |
| MS | Massenspektroskopie |
| MTBE | Methyl-tert.-butylether |
| NMR | Kernresonanzspektroskopie |
| Rₜ | Retentionszeit (bei HPLC) |
| RT | Raumtemperatur |
| TFA | Trifluoressigsäure |
| THF | Tetrahydrofuran |

### LC-MS-, HPLC- und GC-MS-Methoden:

### Methode 1:

Instrument: Micromass Platform LCZ mit HPLC Agilent Serie 1100; Säule: Thermo Hypersil GOLD 3 µ, 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 2:

Instrument: Micromass Platform LCZ mit HPLC Agilent Serie 1100; Säule: Thermo HyPURITY Aquastar 3 µ, 50 mm x 2.1 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 3:

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 6:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 7:

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 8:

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Ausgangsverbindungen und Intermediate:

### Beispiel 1A

### 2-Hydrazino-4-methylpyridin

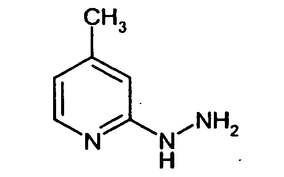

3.33 g (30.0 mmol) 2-Fluor-4-methylpyridin werden in 40 ml 2-Ethoxyethanol vorgelegt, die Lösung mit 14.6 ml (15.0 g, 300 mmol) Hydrazinhydrat versetzt und der Ansatz in der Siedehitze (150°C Badtemperatur) 16 h lang gerührt. Die Reaktionslösung wird am Rotationsverdampfer eingeengt, der Rückstand auf 100 ml Wasser gegeben und mit Essigsäureethylester (dreimal je 100 ml) extrahiert. Die vereinigten organischen Phasen werden über Natriumsulfat getrocknet, filtriert und eingeengt. Der erhaltene Rückstand wird im Vakuum getrocknet.
Ausbeute: 1.90 g (51% d. Th.)
¹H-NMR (400 MHz, DMSO-d₆): δ = 7.83 (d, 1H), 7.22 (s, 1H), 6.51 (s, 1H), 6.38 (d, 1H), 4.04 (s, 2H), 2.17 (s, 3H)
LC-MS (Methode 1): Rₜ = 0.80 min; MS (ESIpos): m/z = 124 [M+H]⁺.

### Beispiel 2A

### 3-Hydroxy-2-pyridin-3-yl-acrylsäureethylester

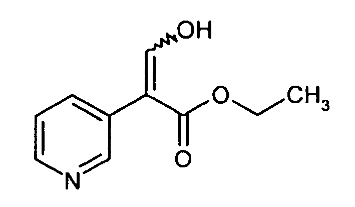

1.65 g (10.0 mmol) 3-Pyridylessigsäureethylester werden unter Argon in 20 ml wasserfreiem Toluol vorgelegt. Man versetzt die Lösung portionsweise mit 410 mg (10.3 mmol) Natriumhydrid-Suspension (60%-ig in Paraffinöl) und 130 mg (0.50 mmol) 18-Krone-6 und rührt den Ansatz 30 min bei RT, anschließend 30 min bei 85°C (Badtemperatur). Nach dieser Zeit wird abgekühlt und bei ca. 20°C 1.48 g (20.0 mol) Ameisensäureethylester zugetropft. Man rührt zunächst 60 min bei RT, dann 60 min bei 90°C (Badtemperatur). Nach Abkühlung wird die Reaktionslösung auf ca. 50 ml gesättigte Ammoniumchlorid-Lösung gegeben und mit Essigsäureethylester (fünfmal je 40 ml) extrahiert. Die vereinigten organischen Phasen werden mit 50 ml gesättigter Natriumchlorid-Lösung gewaschen, über Natriumsulfat getrocknet, filtriert und eingeengt. Der erhaltene Feststoff wird mit Pentan gewaschen und im Vakuum getrocknet.
Ausbeute: 1.3 g (67% d. Th.)
¹H-NMR (400 MHz, DMSO-d₆): δ = 11.38 (br. s, 1H), 8.50 (d, 1H), 8.39 (dd, 1H), 7.97 (s, 1H), 7.71 (d, 1H), 7.35 (dd, 1H), 4.12 (q, 2H), 1.97 (t, 3H).
MS (DCI]: m/z = 194 [M+H]⁺.

### Beispiel 3A

### 2-Pyridin-3-yl-3-(pyridin-2-ylhydrazono)propionsäureethylester

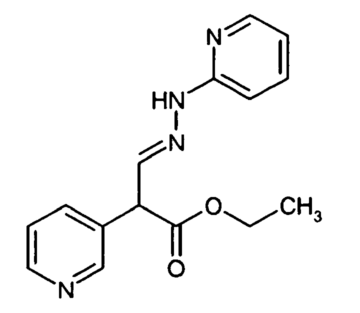

2.90 g (15.0 mmol) der Verbindung aus Beispiel 2A und 1.72 g (15.8 mmol) 2-Pyridylhydrazin werden in 75 ml Ethanol gelöst und der Ansatz 4 d bei RT gerührt. Die Reaktionsmischung wird am Rotationsverdampfer vom Lösungsmittel befreit und der Rückstand über Kieselgel 60 (Laufmittel: Dichlormethan → Dichlormethan/Methanol 10:1 →Dichlormethan/Methanol 2:1) chromatographiert. Die Produktfraktionen werden vereinigt und das Lösungsmittel am Rotationsverdampfer entfernt. Man erhält nach Trocknen im Vakuum 3.95 g (93% d. Th.) der Titelverbindung.
LC-MS (Methode 2): Rₜ = 2.10 min; MS (ESIpos): m/z = 285 [M+H]⁺.

### Beispiel 4A

### (6-Brompyridin-3-yl)methanol

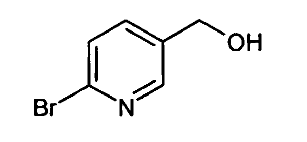

1.34 ml (1.34 mmol) einer 1 M Lösung von Lithiumaluminiumhydrid in THF werden in 5 ml trockenem THF unter Argon vorgelegt und eine Lösung von 500 mg (2.69 mmol) 6-Brom-3-pyridincarbaldehyd in 3 ml trockenem THF bei 0°C zugetropft. Man rührt 1 h bei RT nach, versetzt den Ansatz dann unter Eisbadkühlung mit 25 ml Essigsäureethylester und hydrolysiert langsam mit 50 ml gesättigter Natriumhydrogencarbonat-Lösung. Die wässrige Phase wird mit Essigsäureethylester extrahiert (dreimal je 20 ml). Die vereinigten organischen Phasen werden mit gesättigter Natriumchlorid-Lösung gewaschen, über Natriumsulfat getrocknet, filtriert und am Rotationsverdampfer eingeengt. Nach Entfernen von Lösungsmittelresten im Vakuum erhält man 375 mg (74% d. Th.) der Titelverbindung.
LC-MS (Methode 3): Rₜ = 1.02 min; MS (ESIpos): m/z = 189 [M+H]⁺.

### Beispiel 5A

### (6-Chlor-5-methylpyridin-3-yl)methanol

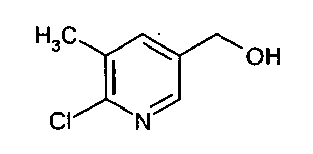

Die Titelverbindung wird durch Umsetzung von 3.11 g (20.0 mmol) 6-Chlor-5-methylnicotinaldehyd [Herstellung beschrieben in DE 4429465-A1, Beispiel 7] mit 1.51 g (40.0 mmol) Natriumborhydrid in 30 ml Wasser und anschließende Extraktion der wässrigen Phase mit Dichlormethan erhalten. Das nach Entfernen des Lösungsmittels am Rotationsverdampfer erhaltene Produkt wird im Vakuum getrocknet und direkt weiterverwendet.

### Beispiel 6A

### 2-Brom-5-(chlormethyl)pyridin

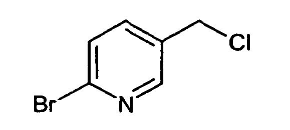

3.69 g (19.7 mmol) der Verbindung aus Beispiel 4A werden unter Argon vorgelegt und bei -60°C (Badtemperatur) tropfenweise mit 25 ml Thionylchlorid versetzt. Der Ansatz wird 1 h bei -60°C gerührt. Man engt bei RT am Rotationsverdampfer ein und versetzt den Rückstand mit 50 ml gesättigter Natriumhydrogencarbonat-Lösung und 50 ml Essigsäureethylester. Die wässrige Phase wird mit Essigsäureethylester extrahiert (viermal je 25 ml). Die vereinigten organischen Phasen werden über Natriumsulfat getrocknet, filtriert, das Lösungsmittel am Rotationsverdampfer entfernt und der Rückstand im Vakuum getrocknet.
Ausbeute: 3.71 g (91% d. Th.)
LC-MS (Methode 1): Rₜ = 3.28 min; MS (ESIpos): m/z = 208 [M+H]⁺.

### Beispiel 7A

### 2-Chlor-5-(chlormethyl)-3-methylpyridin

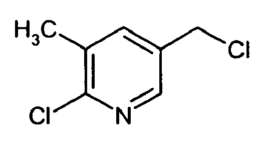

Die Herstellung erfolgt analog zu Beispiel 6A aus 1.00 g (6.35 mmol) (6-Chlor-5-methylpyridin-3-yl)methanol und 5 ml Thionylchlorid. Es werden 1.26 g der Titelverbindung erhalten, welche ohne weitere Reinigung umgesetzt wird.
LC-MS (Methode 4): Rₜ = 1.92 min; MS (ESIpos): m/z = 176 [M]⁺.

### Beispiel 8A

### (6-Brompyridin-3-yl)acetonitril

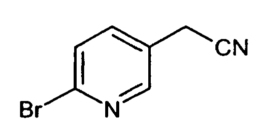

3.75 g (18.2 mmol) der Verbindung aus Beispiel 6A werden in 20 ml DMF vorgelegt, 979 mg (20.0 mmol) Natriumcyanid zugegeben und der Ansatz für 2 d bei RT gerührt. Die Reaktionsmischung wird auf ein Gemisch aus 250 ml gesättigter Ammoniumchlorid-Lösung und 200 ml Essigsäureethylester gegeben und die wässrige Phase mit Essigsäureethylester (dreimal je 100 ml) extrahiert. Die vereinigten organischen Phasen werden über Natriumsulfat getrocknet, filtriert, eingeengt und der Rückstand im Vakuum getrocknet. Das so erhaltene Produkt wird ohne weitere Aufreinigung umgesetzt.
Ausbeute: 3.23 g (90% d. Th.)
LC-MS (Methode 3): Rₜ = 1.46 min; MS (ESIpos): m/z = 197 [M+H]⁺.

### Beispiel 9A

### (6-Chlor-5-methylpyridin-3-yl)acetonitril

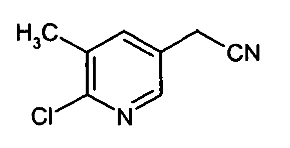

Die Synthese erfolgt analog zu Beispiel 8A aus 1.26 g (7.14 mmol) der Verbindung aus Beispiel 7A. Das erhaltene Rohprodukt wird über Kieselgel 60 (Laufmittel: Dichlormethan → Dichlormethan/Methanol 50:1) chromatographisch gereinigt.
Ausbeute: 215 mg (18% d. Th.)
LC-MS (Methode 1): Rₜ = 2.95 min; MS (ESIpos): m/z = 167 [M+H]⁺.

### Beispiel 10A

### (2-Chlorpyridin-3-yl)essigsäureethylester

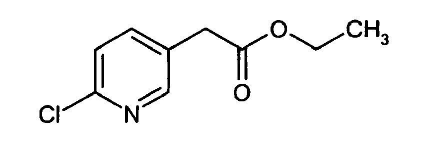

Zu einer Mischung aus 270 ml Ethanol und 101 ml konz. Schwefelsäure werden 22.0 g (144 mmol) (6-Chlorpyridin-3-yl)acetonitril gegeben und der Ansatz 24 h unter Rückfluß gerührt. Man tropft das Reaktionsgemisch langsam unter Rühren zu einer Mischung aus 350 g Natriumhydrogencarbonat und 1 Liter Wasser. Die wässrige Phase wird mit Dichlormethan (fünfmal je 400 ml) extrahiert. Die vereinigten organischen Phasen werden über Natriumsulfat getrocknet, filtriert und am Rotationsverdampfer vom Lösungsmittel befreit. Man erhält 23.1 g (80% d. Th.) der Titelverbindung, welche ohne weitere Reinigung umgesetzt wird.
¹H-NMR (400 MHz, DMSO-d₆): δ = 8.32 (d, 1H), 7.78 (dd, 1H), 7.49 (d, 1H), 4.10 (q, 2H), 3.77 (s, 2H), 1.19 (t, 3H).
LC-MS (Methode 3): Rₜ = 1.91 min; MS (ESIpos): m/z = 200 [M+H]⁺.

In analoger Weise zu Beispiel 10A werden die in Tabelle 1 aufgeführten Verbindungen aus den entsprechenden Edukten erhalten:

**Tabelle 1**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **11A** | 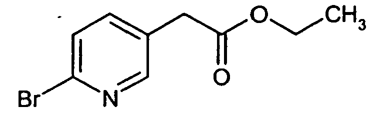 | 246 | 2.02 (5) | 55 |
| **12A** | 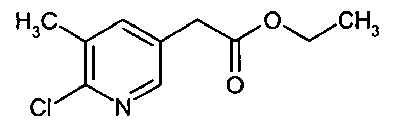 | 214 | 2.14 (5) | 86 |

### Beispiel 13A

### (5-Brompyridin-3-yl)essigsäureethylester

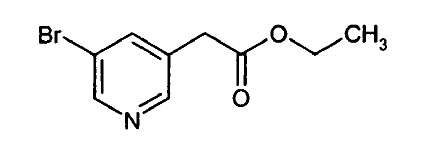

1.00 g (4.63 mmol) (5-Brompyridin-3-yl)essigsäure werden in 20 ml Ethanol vorgelegt, mit 2 ml konz. Schwefelsäure versetzt und der Ansatz über Nacht unter Rückfluß gerührt. Die Reaktionslösung wird unter Rühren auf eine Mischung aus 100 ml gesättigter Natriumhydrogencarbonat-Lösung und 100 ml Essigsäureethylester gegeben und die wässrige Phase mit Essigsäureethylester (dreimal je 50 ml) extrahiert. Die vereinigten organischen Phasen werden über Natriumsulfat getrocknet, filtriert, eingeengt und der Rückstand über Nacht im Vakuum von Lösungsmittelresten befreit.
Ausbeute: 1.06 g (94% d. Th.)
¹H-NMR (400 MHz, DMSO-d₆): δ = 8.61 (d, 1H), 8.48 (d, 1H), 8.01 (dd, 1H), 4.10 (q, 2H), 3.78 (s, 2H), 1.20 (t, 3H).
LC-MS (Methode 5): Rₜ = 2.06 min; MS (ESIpos): m/z = 246 [M+H]⁺.

### Beispiel 14A

### 2-(6-Brompyridin-3-yl)-3-(dimethylamino)acrylsäureethylester

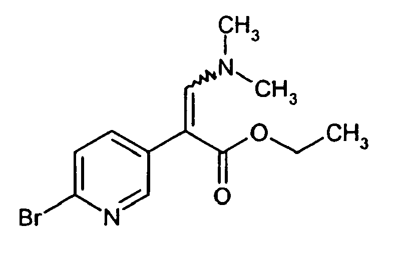

1.30 g (2.98 mmol) der Verbindung aus Beispiel 11A werden in 6 ml Dimethylformamid-diethylacetal gelöst und der Ansatz unter Mikrowellenbestrahlung 60 min bei 100°C gerührt. Der Ansatz wird am Rotationsverdampfer eingeengt und der Rückstand über Kieselgel 60 (Laufmittel: Cyclohexan → Cyclohexan/Essigsäureethylester 1:3) chromatographiert.
Ausbeute: 854 mg (96% d.Th.)
¹H-NMR (400 MHz, DMSO-d₆): δ = 8.11 (d, 1H), 7.61 (s, 1H), 7.54 (d, 1H), 7.48 (dd, 1H), 4.01 (q, 2H), 2.70 (br. s, 6H), 1.12 (t, 3H).
MS (DCI, NH₃): m/z = 316 [M+NH₄]⁺
LC-MS (Methode 4): Rₜ = 1.88 min; MS (ESIpos): m/z = 299 [M+H]⁺.

Die in Tabelle 2 aufgeführten Verbindungen werden aus den entsprechenden Edukten analog zu Beispiel 14A hergestellt:

**Tabelle 2**

| **Beispiel Nr.** | **Struktur** | **Ausbeute (% d. Th.)** | **MS (ESI) [M+H]⁺; LC-MS Rₜ [min] (Methode)** | **¹H-NMR** |
|---|---|---|---|---|
| **15A** | 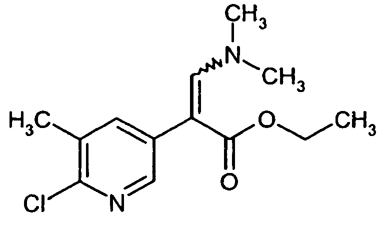 | 70 | m/z = 269; 1.97 min (4) | (400 MHz, DMSO-d₆): δ=7.97 (d, 1H), 7.60 (s, 1H), 7.53 (d, 1H), 4.00 (q, 2H), 2.70 (s, 6H), 2.31 (s, 3H), 1.12 (t, 3H). |
| **16A** | 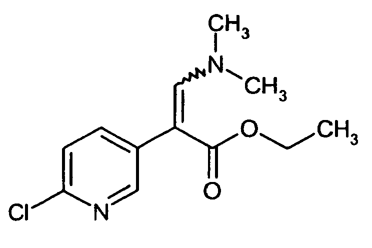 | 90 | m/z = 255; 1.98 min (3) | (300 MHz, DMSO-d₆): δ = 8.13 (d, 1H), 7.61 (s, 1H), 7.58 (dd, 1H), 7.41 (d, 1H), 4.01 (q, 2H), 2.70 (s, 6H), 1.12 (t, 3H). |
| **17A** | 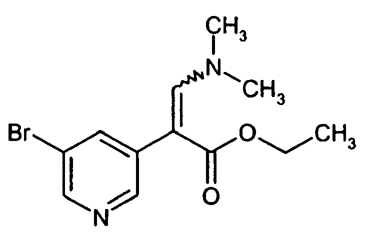 | 80 | m/z = 301; 1.83 min (4) | (300 MHz, DMSO-d₆): δ = 8.49 (d, 1H), 8.30 (d, 1H), 7.78 (dd, 1H), 7.62 (s, 1H), 4.02 (q, 2H), 2.71 (s, 6H), 1.13 (t, 3H). |

### Beispiel 18A

### 3-Oxo-2-pyridin-3-yl-butansäureethylester

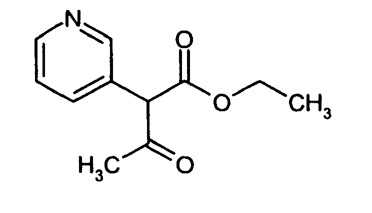

Unter Argon werden 500 mg (3.0 mmol) Ethyl-pyridin-3-acetat in 5 ml wasserfreiem THF vorgelegt und eine Lösung von Lithiumhexamethyldisilazid (6.7 ml, 1M in THF) bei -78°C zugetropft. Nach 15 min erwärmt man auf 0°C, rührt 1h nach und kühlt wieder auf -78°C. Nach Zugabe von 340 mg (3.3 mmol) Essigsäureanhydrid wird der Ansatz 36 h lang bei RT gerührt. Man versetzt mit wässriger Ammoniumchlorid-Lösung, extrahiert mit Dichlormethan, trocknet die organische Phase über Magnesiumsulfat und engt im Vakuum ein. Es werden 488 mg der Titelverbindung mit 70%-iger Reinheit erhalten, die ohne weitere Reinigung umgesetzt werden.
LC-MS (Methode 1): Rₜ = 2.24 min; MS (ESIpos): m/z = 208 [M+H]⁺.

### Beispiel 19A

### (5-Methylpyridin-3-yl)acetonitril

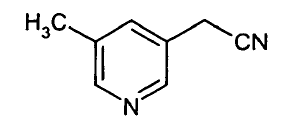

Die Herstellung der Titelverbindung ist in DE 2 854 210-C2 (Tabelle 2, Beispiel 37) beschrieben.

### Beispiel 20A

### 5-(Cyanomethyl)pyridin-2-carbonsäureethylester

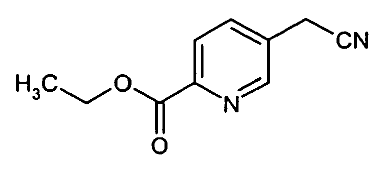

10.5 g (52.6 mmol) 5-(Chlormethyl)pyridin-2-carbonsäureethylester [Herstellung nach H. Barth et al., Liebigs Ann. Chem. 1981, 2164-2179] werden in 75 ml wasserfreiem DMF vorgelegt und bei RT innerhalb von 3 h portionsweise mit 2.58 g (52.6 mmol) Natriumcyanid versetzt. Anschließend wird der Ansatz auf 500 ml gesättigte Ammoniumchlorid-Lösung gegeben und mit Dichlormethan (viermal je 100 ml) extrahiert. Die vereinigten organischen Phasen werden über Natriumsulfat getrocknet, eingeengt und der Rückstand über Kieselgel chromatographisch gereinigt (Laufmittel: Cyclohexan → Cyclohexan/Essigsäureethylester 1:4). Man erhält 3.80 g (38% d. Th.) der Titelverbindung.
¹H-NMR (300 MHz, DMSO-d₆): δ = 8.69 (d, 1H), 8.10 (d, 1H), 7.99 (dd, 1H), 4.36 (q, 2H), 4.24 (s, 2H), 1.34 (t, 3H).
LC-MS (Methode 3): Rₜ = 1.45 min; MS (ESIpos): m/z = 191 [M+H]⁺.

In analoger Weise zu Beispiel 10A werden die in Tabelle 3 aufgeführten Verbindungen aus den entsprechenden Edukten erhalten:

**Tabelle 3**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **21A** | 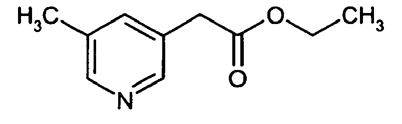 | 180 | 2.16(1) | 93 |
| **22A** | 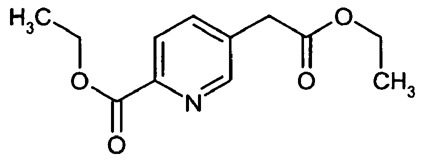 | 238 | 1.79 (3) | 82 |

### Beispiel 23A

### (4-Methylpyridin-3-yl)essigsäureethylester

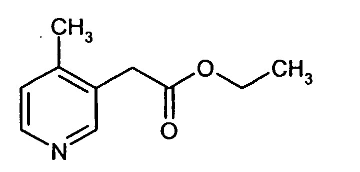

Die Synthese der Titelverbindung erfolgt analog zu Beispiel 13A aus 200 mg (1.32 mmol) (4-Methylpyridin-3-yl)essigsäure.
Ausbeute: 235 mg (99% d. Th.)
¹H-NMR (300 MHz, DMSO-d₆): δ = 8.32 (d, 1H), 7.56 (dd, 1H), 7.20 (dd, 1H), 4.08 (q, 2H), 3.67 (s, 2H), 2.44 (s, 3H), 1.18 (t, 3H).
LC-MS (Methode 1): Rₜ = 1.86 min; MS (ESIpos): m/z = 180 [M+H]⁺.

### Beispiel 24A

### (6-Methylpyridin-3-yl)essigsäureethylester

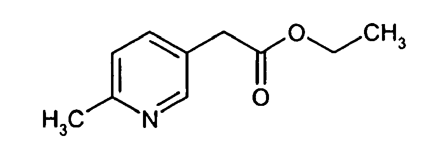

Die Synthese der Titelverbindung erfolgt analog zu Beispiel 13A aus 493 mg (3.26 mmol) (6-Methylpyridin-3-yl)essigsäure [Herstellung: N. Sperber et al., J. Am. Chem. Soc. 81, 704-709 (1959)].
Ausbeute: 580 mg (99% d. Th.)
¹H-NMR (300 MHz, DMSO-d₆): δ = 8.32 (d, 1H), 7.57 (dd, 1H), 7.20 (d, 1H), 4.08 (q, 2H), 3.67 (s, 2H), 2.44 (s, 3H), 1.18 (t, 3H).
LC-MS (Methode 1): Rₜ = 1.85 min; MS (ESIpos): m/z = 180 [M+H]⁺.

Die in Tabelle 4 aufgeführten Verbindungen werden aus den entsprechenden Edukten analog zu Beispiel 14A hergestellt:

**Tabelle 4**

| **Beispiel Nr.** | **Struktur** | **Ausbeute (% d. Th.)** | **MS (ESI) [M+H]⁺; LC-MS Rₜ [min] (Methode)** | **¹H-NMR** |
|---|---|---|---|---|
| **25A** | 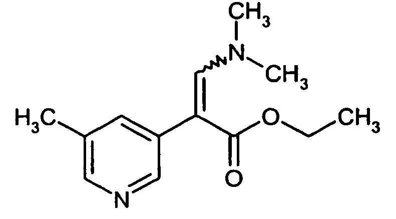 | 84 | m/z = 235; 1.08 min (5) | (400 MHz, DMSO-d₆): δ = 8.21 (d, 1H), 8.11 (d, 1H), 7.57 (s, 1H), 7.32 (s, 1H), 4.00 (q, 2H), 2.66 (s, 6H), 2.28 (s, 3H), 1.11 (t, 3H). |
| **26A** | 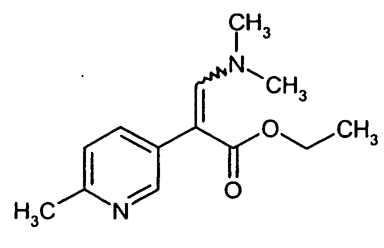 | 50 | m/z = 235; 2.17 min (1) | |
| **27A** | 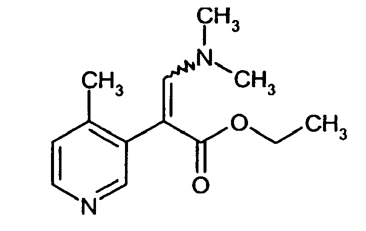 | 35 | m/z = 235; 2.21 min (1) | |
| **28A** | 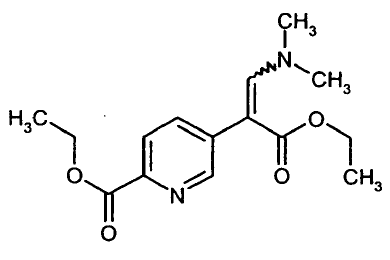 | 81 | m/z = 293; 2.02 min (5) | (300 MHz, DMSO-d₆): δ = 8.44 (d, 1H), 7.96 (d, 1H), 7.68 (s, 1H), 7.66 (dd, 1H), 4.33 (q, 2H), 4.03 (q, 2H), 2.71 (br. s, 6H), 1.33 (t, 3H), 1.13 (t, 3H). |

Die in Tabelle 5 aufgeführten Verbindungen werden aus den entsprechenden 2-Chlorpyridinen analog zu Beispiel 1A erhalten. Anstelle der unter Beispiel 1A beschriebenen Aufarbeitung wird hier die Reaktionslösung eingeengt und der Rückstand mit einer Mischung aus Diethylether und Dichlormethan verrührt. Man filtriert das überschüssige, kristalline Hydrazin-Hydrochlorid ab, engt das Filtrat ein, trocknet dieses im Vakuum und setzt das Produkt ohne weitere Aufreinigung um.

**Tabelle 5**

| **Beispiel Nr.** | **Struktur** | **MS (ESI) [M+H]⁺; LC-MS Rₜ [min] (Methode)** | **¹H-NMR** (400 MHz, DMSO-d₆) |
|---|---|---|---|
| **29A** | 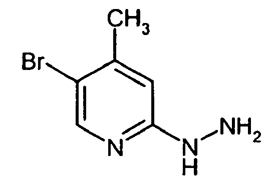 | m/z = 202; 2.14 min (1) | δ = 7.99 (s, 1H), 7.52 (s, 1H), 6.70 (s, 1H), 4.14 (s, 2H), 2.22 (s, 3H). |
| **30A** | 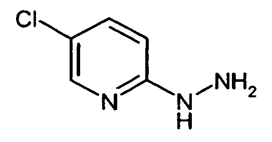 | m/z = 144; 0.78 min (1) | δ = 7.96 (d, 1H), 7.65 (s, 1H), 7.50 (dd, 1H), 6.73 (d, 1H), 4.18 (s, 2H). |
| **31A** | 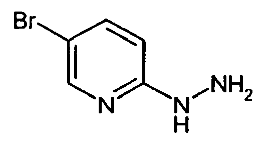 | m/z = 188; 1.00 min (1) | |
| **32A** | 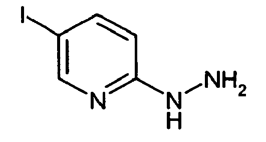 | m/z = 236; 1.32 min (1) | δ= 8.11 (d, 1 H), 7.67 (dd, 1H), 7.62 (s, 1H), 6.60 (d, 1H), 4.14 (s, 2H). |

### Beispiel 33A

### 2-Hydrazino-isonicotinsäurenitril

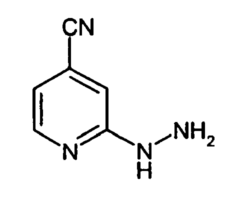

20.0 g (144 mmol) 2-Chlorisonicotinsäurenitril werden in 150 ml 1-Butanol vorgelegt, mit 303 ml (303 mmol) einer 1 M Lösung von Hydrazinhydrat in THF versetzt und 16 h lang erhitzt (110°C Badtemperatur). Es wird eingeengt und der Rückstand mittels Flash-Chromatographie an Kieselgel (Laufmittel: Dichlormethan/Methanol 10:1) gereinigt.
Ausbeute: 9.48 g (49% d. Th.)
¹H-NMR (400 MHz, DMSO-d₆): δ = 8.15 (d, 1H), 8.05 (s, 1H), 7.01 (s, 1H), 6.83 (dd, 1H), 4.30 (s, 2H).
LC-MS (Methode 1): Rₜ = 0.52 min; MS (ESIpos): m/z = 135 [M+H]⁺.

### Beispiel 34A

### (6-Hydrazinopyridin-3-yl)methanol

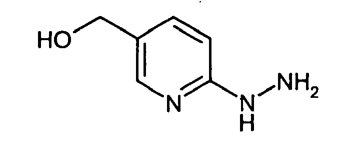

20.0 g (139 mmol) (6-Chlorpyridin-3-yl)methanol werden in 400 ml einer 35%-igen wässrigen Hydrazinhydrat-Lösung über Nacht unter Rückfluss erhitzt. Es wird eingeengt, mit Toluol versetzt, erneut eingeengt und der Rückstand mit einer Mischung aus Dichlormethan, Methanol und Diethylether verrührt. Man filtriert den kristallinen Rückstand (Hydrazin-Hydrochlorid) ab, engt das Filtrat ein und trocknet im Vakuum.
Ausbeute: 19.3 g (99% d. Th.)
¹H-NMR (400 MHz, DMSO-d₆): δ = 7.91 (d, 1H), 7.40 (dd, 1H), 7.29 (s, 1H), 6.66 (d, 1H), 4.93 (s, 1H), 4.31 (s, 2H), 4.14 (s, 2H).
LC-MS (Methode 1): Rₜ = 0.50 min; MS (ESIpos): m/z = 140 [M+H]⁺.

### Beispiel 35A

### Benzophenon-(4-methoxypyridin-2-yl)hydrazon

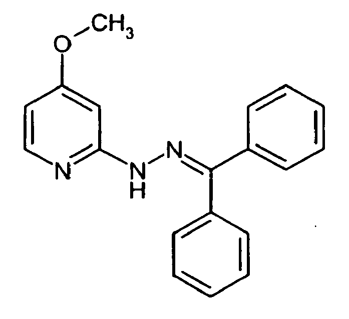

Unter Argon werden 500 mg (3.48 mmol) 2-Chlor-4-methoxypyridin, 752 mg (3.83 mmol) Benzophenonhydrazon, 469 mg (4.88 mmol) Natrium-*tert*.-butylat, 15.6 mg (0.07 mmol) Palladium(II)-acetat, 21.2 mg (0.17 mmol) Phenylboronsäure und 43.4 mg (0.07 mmol) racemisches 2,2'- Bis-(diphenylphosphino)-1,1'-binaphthyl in entgastem Toluol bei 90°C über Nacht erhitzt. Nach dem Abkühlen gießt man die Reaktionsmischung in Wasser, extrahiert die wässrige Phase mehrfach mit Essigsäureethylester, trocknet die vereinigten organischen Phasen über Natriumsulfat, filtriert und engt ein. Der Rückstand wird mittels präparativer HPLC gereinigt (RP18-Säule; Laufmittel: Acetonitril/Wasser-Gradient).
Ausbeute: 872 mg (83% d. Th.)
¹H-NMR (400 MHz, DMSO-d₆): δ = 10.8 (s, 1H), 8.07 (d, 1H), 7.70-7.64 (m, 5H), 7.50-7.38 (m, 5H), 6.94 (d, 1H), 6.78 (dd, 1H), 3.92 (s, 3H).
LC-MS (Methode 3): Rₜ = 1.70 min; MS (ESIpos): m/z = 304 [M+H]⁺.

### Beispiel 36A

### 2-Hydrazino-4-methoxypyridin

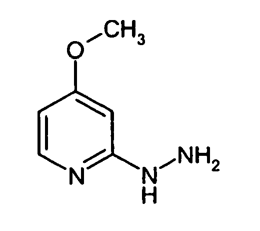

850 mg (2.80 mmol) der Verbindung aus Beispiel 35A werden in konzentrierter Salzsäure über Nacht bei 65°C erhitzt. Nach dem Abkühlen wird die Reaktionsmischung mit Dichlormethan gewaschen und eingeengt. Man erhält 470 mg des Rohproduktes als Hydrochlorid. 250 mg hiervon werden mit Polymer-gebundenem Tris(2-aminoethyl)amin in Dichlormethan über Nacht bei RT gerührt. Man filtriert, engt das Filtrat ein und trocknet im Hochvakuum.
Ausbeute: 170 mg (39% d. Th.)
¹H-NMR (400 MHz, DMSO-d₆): δ = 7.78 (d, 1H), 7.26 (s, 1H), 6.25 (d, 1H), 6.15 (dd, 1H), 4.09 (s, 2H), 3.73 (s, 3H).
LC-MS (Methode 1): Rₜ = 0.89 min; MS (ESIpos): m/z = 140 [M+H]⁺.

### Beispiel 37A

### 3-(Chlormethyl)-2-methylpyridin

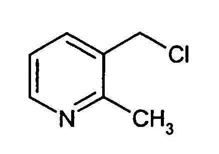

1.00 g (8.12 mmol) 3-(Hydroxymethyl)-2-methylpyridin werden vorgelegt und bei 0°C langsam mit 5.9 ml (81.2 mmol) Thionylchlorid versetzt. Der Ansatz wird 3 h unter Rückfluss erhitzt. Man engt ein, versetzt den Rückstand mit gesättigter Natriumhydrogencarbonat-Lösung und extrahiert mehrmals mit Diethylether. Die vereinigten organischen Phasen werden mit gesättigter Natriumchlorid-Lösung gewaschen, über Natriumsulfat getrocknet, filtriert und eingeengt.
Ausbeute: 0.98 g (85% d. Th.)
GC-MS (Methode 7): Rₜ = 4.85 min; MS (EIpos): m/z = 141 [M]⁺.

### Beispiel 38A

### (2-Methylpyridin-3-yl)acetonitril

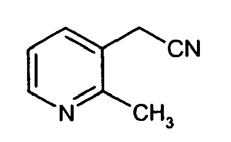

970 mg (6.85 mmol) der Verbindung aus Beispiel 37A werden in 10 ml DMF vorgelegt, mit 336 mg (6.85 mmol) Natriumcyanid versetzt und der Ansatz über Nacht bei 45°C gerührt. Die Reaktionsmischung wird auf 75 ml gesättigte Ammoniumchlorid-Lösung gegeben und mehrmals mit Dichlormethan extrahiert. Die vereinigten organischen Phasen werden über Natriumsulfat getrocknet, filtriert und eingeengt. Man reinigt den Rückstand mittels Flash-Chromatographie an Kieselgel (Laufmittel: Dichlormethan/Methanol 20:1).
Ausbeute: 795 mg (88% d. Th.)
GC-MS (Methode 7): Rₜ = 6.14 min; MS (EIpos): m/z = 132 [M]⁺.

### Beispiel 39A

### (2-Methylpyridin-3-yl)essigsäureethylester

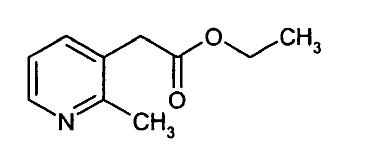

790 mg (5.98 mmol) der Verbindung aus Beispiel 38A werden in 10 ml Ethanol vorgelegt, langsam mit 4 ml konzentrierter Schwefelsäure versetzt und 6 h unter Rückfluss erhitzt. Nach dem Abkühlen neutralisiert man mit 6.00 g Natriumhydrogencarbonat und gesättigter Natriumhydrogencarbonat-Lösung. Die wässrige Phase wird mehrfach mit Essigsäureethylester extrahiert, und die vereinigten organischen Phasen werden über Natriumsulfat getrocknet, filtriert und eingeengt. Man setzt den Rückstand ohne weitere Aufreinigung um.
Ausbeute: 614 mg (57% d. Th.)
¹H-NMR (400 MHz, DMSO-d₆): δ = 8.34 (dd, 1H), 7.57 (dd, 1H), 7.18 (dd, 1H), 4.10 (q, 2H), 3.73 (s, 2H), 2.40 (s, 3H), 1.18 (t, 3H).
LC-MS (Methode 1): Rₜ = 1.84 min; MS (ESIpos): m/z = 180 [M+H]⁺.

### Beispiel 40A

### (6-Trifluormethylpyridin-3-yl)-essigsäureethylester

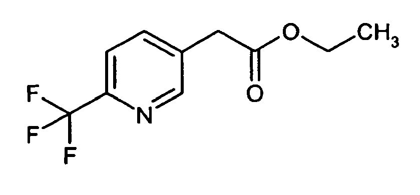

4.23 g (20.6 mmol) (6-Trifluormethylpyridin-3-yl)essigsäure [erhältlich aus [6-(Trifluormethyl)-pyridin-3-yl]methanol analog zur Reaktionssequenz der Beispiele 37A, 38A und 41] werden unter Argon in 200 ml Ethanol vorgelegt, mit 0.2 ml konzentrierter Schwefelsäure versetzt und 5 h lang unter Rückfluss erhitzt. Nach dem Abkühlen engt man die Reaktionslösung ein, nimmt den Rückstand in Essigsäureethylester auf und wäscht mit gesättigter Natriumhydrogencarbonat-Lösung. Die wässrige Phase wird mehrfach mit Essigsäureethylester rückextrahiert, und die vereinigten organischen Phasen werden über Natriumsulfat getrocknet, filtriert und eingeengt. Man reinigt den Rückstand mittels Flash-Chromatographie an Kieselgel (Laufmittel: Gradient Cyclohexan → Cyclohexan/Essigsäureethylester 1:1).
Ausbeute: 3.24 g (67% d. Th.)
¹H-NMR (400 MHz, DMSO-d₆): δ = 8.69 (s, 1H), 8.02 (d, 1H), 7.89 (d, 1H), 4.13 (q, 2H), 3.91 (s, 2H), 1.21 (t, 3H).
LC-MS (Methode 3): Rₜ = 2.12 min; MS (ESIpos): m/z = 234 [M+H]⁺.

### Beispiel 41A

### 3-Hydroxy-2-(6-trifluormethylpyridin-3-yl)-acrylsäureethylester

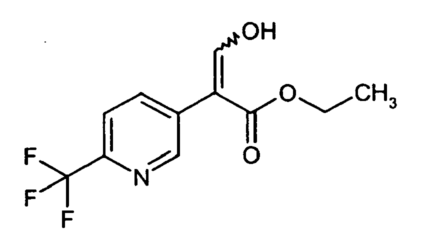

3.46 g (14.8 mmol) der Verbindung aus Beispiel 40A werden unter Argon in 50 ml wasserfreiem Toluol vorgelegt, portionsweise mit 712 mg (17.8 mmol) Natriumhydrid-Suspension (60%-ig in Paraffinöl) versetzt und 1 h bei RT und anschließend 20 min bei 80°C gerührt. Nach dem Abkühlen gibt man 392 mg (1.48 mmol) 18-Krone-6 und anschließend unter Eiskühlung 2.20 g (29.7 mol) Ameisensäureethylester tropfenweise hinzu. Man rührt zunächst 1 h bei 0°C, dann 1 h bei RT. Anschließend gibt man eine Mischung aus 100 ml Essigsäureethylester und 150 ml 0.1 M Salzsäure hinzu, trennt die Phasen, extrahiert die wässrige Phase mehrfach mit Essigsäureethylester, trocknet die vereinigten organischen Phasen über Natriumsulfat, filtriert und engt ein. Man reinigt den Rückstand mittels Flash-Chromatographie an Kieselgel (Laufmittel: Cyclohexan/Essigsäureethylester-Gradient).
Ausbeute: 3.9 g (100% d. Th.)
¹H-NMR (400 MHz, DMSO-d₆): δ = 11.8 (s, 1H), 8.70 (d, 1H), 8.03 (s, 1H), 8.00 (d, 1H), 7.87 (d, 1H), 4.15 (q, 2H), 1.21 (t, 3H).

### Beispiel 42A

### 3-(Dimethylamino)-2-pyridin-3-yl-acrylsäureethylester

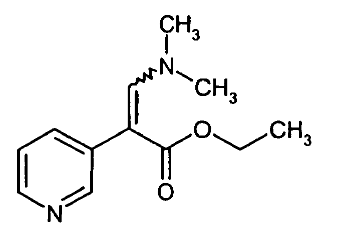

37.4 g (226 mmol) Pyridin-3-ylessigsäureethylester werden in 100 g (679 mmol) Dimethylformamid-diethylacetal über Nacht bei 100°C erhitzt. Nach dem Abkühlen engt man ein und reinigt den Rückstand mittels Flash-Chromatographie an Kieselgel vor (Laufmittel: Gradient Cyclohexan/Essigsäureethylester 1:1 → Essigsäureethylester/Ethanol 9:1). Das erhaltene Produkt wird durch Vakuumdestillation (1 mbar, 200°C Badtemperatur) feingereinigt.
Ausbeute: 35.0 g (70% d. Th.)
¹H-NMR (400 MHz, DMSO-d₆): δ = 8.37 (dd, 1H), 8.31 (dd, 1H), 7.59 (s, 1H), 7.51 (dt, 1H), 7.29 (ddd, 1H), 4.00 (q, 2H), 2.67 (s, 6H), 1.11 (t, 3H).
LC-MS (Methode 1): Rₜ = 2.38 min; MS (ESIpos): m/z = 221 [M+H]⁺.

### Beispiel 43A

### 3-(Dimethylamino)-2-(2-methylpyridin-3-yl)-acrylsäureethylester

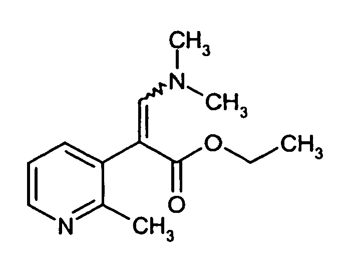

600 mg (3.35 mmol) der Verbindung aus Beispiel 39A werden in 1.7 ml (10.0 mmol) Dimethylformamid-diethylacetal über Nacht bei 100°C erhitzt. Nach dem Abkühlen engt man ein und reinigt den Rückstand mittels präparativer HPLC (RP18-Säule; Laufmittel: Acetonitril/Wasser-Gradient).
Ausbeute: 619 mg (79% d. Th.)
¹H-NMR (400 MHz, DMSO-d₆): δ = 8.30 (dd, 1H), 7.54 (s, 1H), 7.38 (dd, 1H), 7.13 (dd, 1H), 4.05-3.92 (m, 2H), 2.62 (s, 6H), 2.30 (s, 3H), 1.08 (t, 3H).
LC-MS (Methode 1): Rₜ = 2.19 min; MS (ESIpos): m/z = 235 [M+H]⁺.

### Ausführungsbeispiele:

### Beispiel 1

### 4-Pyridin-3-yl-2-pyrimidin-2-yl-1,2-dihydro-3H-pyrazol-3-on

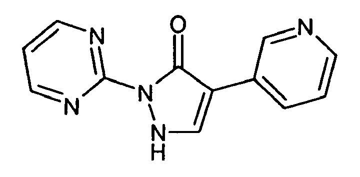

193 mg (1 mmol) der Verbindung aus Beispiel 2A und 116 mg (1.05 mmol) 2-Hydrazinopyrimidin werden in 2 ml wasserfreiem Ethanol unter Argon vorgelegt und der Ansatz 20 h bei RT gerührt. Anschließend gibt man portionsweise 40 mg (1 mmol) Natriumhydrid-Suspension (60%-ig in Paraffinöl) hinzu, wobei eine deutliche Trübung der Reaktionslösung entsteht. Es wird 10 min bei RT nachgerührt. Das dunkle Reaktionsgemisch wird dann mit 1 ml 1 M Salzsäure versetzt, wobei ein Niederschlag ausfällt. Man saugt ab, wäscht den Rückstand mit Wasser (2 x 1 ml) und trocknet im Vakuum. Man erhält 173 mg (72% d. Th.) der Titelverbindung.
¹H-NMR (300 MHz, DMSO-d₆): δ = 12.8 (br. s, 1H), 9.04 (d, 1H), 8.92 (d, 2H), 8.38 (m, 2H), 8.19 (d, 1H), 7.50 (dd, 1H), 7.42 (dd, 1H).
LC-MS (Methode 5): Rₜ = 0.59 min; MS (ESIpos): m/z = 240 [M+H]⁺.

### Beispiel 2

### 2-(4-Methylpyridin-2-yl)-4-pyridin-3-yl-1,2-dihydro-3H-pyrazol-3-on

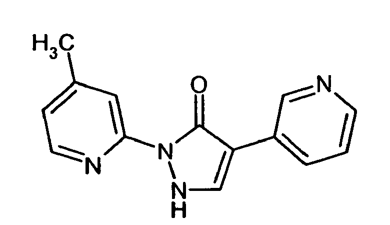

1.53 g (7.90 mmol) der Verbindung aus Beispiel 2A und 2.92 g (23.7 mmol) der Verbindung aus Beispiel 1A werden in 2 ml wasserfreiem Ethanol unter Argon gelöst und der Ansatz 16 h bei RT gerührt. Man gibt 537 mg (7.90 mmol) Natriumethanolat hinzu, wobei sich die Reaktionslösung dunkelrot färbt. Es wird 30 min bei RT nachgerührt und anschließend mit 7.9 ml 1 M Salzsäure versetzt. Die Lösung wird teilweise eingeengt, wobei ein Niederschlag ausfällt. Dieser wird abfiltriert, mit Wasser (zweimal je 5 ml) und mit MTBE (5 ml) gewaschen und im Vakuum getrocknet. Man erhält 435 mg (22% d. Th.) der Titelverbindung.
¹H-NMR (400 MHz, DMSO-d₆): δ = 9.06 (d, 1H), 8.35 (m, 3H), 8.20 (d, 1H), 8.08 (s, 1H), 7.37 (dd, 1H), 7.21 (d, 1H), 2.45 (s, 3H).
LC-MS (Methode 5): Rₜ = 1.42 min; MS (ESIpos): m/z = 253 [M+H]⁺.

### Beispiel 3

### 4-Pyridin-3-yl-2-[5-(trifluormethyl)pyridin-2-yl]-1,2-dihydro-3H-pyrazol-3-on

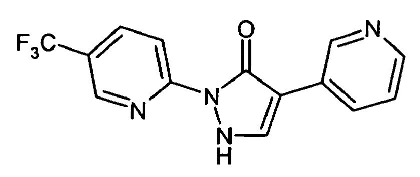

Die Verbindung wird analog zu Beispiel 2 aus 580 mg (3.00 mmol) der Verbindung aus Beispiel 2A und 558 mg (3.00 mmol) 2-Hydrazino-5-(trifluormethyl)pyridin hergestellt.
Ausbeute: 55 mg (6% d. Th.)
HPLC (Methode 6): Rₜ= 3.7 min.
MS (ESIpos): m/z = 307 [M+H]⁺
¹H-NMR (des Ethanol-Addukts) (300 MHz, DMSO-d₆): δ = 13.3 (s, 1H), 9.14 (d, 1H), 8.86 (d, 1H), 8.66 (d, 1H), 8.60 (s, 1H), 8.31-8.41 (m, 3H), 7.40 (dd, 1H), 4.36 (s, 1H), 3.45 (q, 2H), 1.05 (t, 3H).

### Beispiel 4

### 2-(5-Oxo-4-pyridin-3-yl-2,5-dihydro-1H-pyrazol-1-yl)isonicotinsäure-tert.-butylester

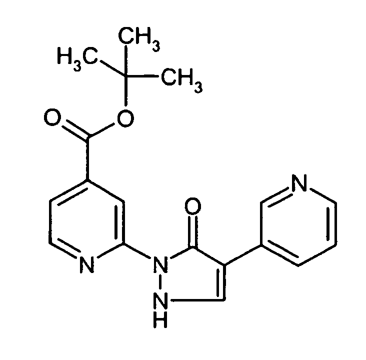

Die Verbindung wird analog zu Beispiel 2 aus 500 mg (2.59 mmol) der Verbindung aus Beispiel 2A und 662 mg (2.85 mmol) 2-Hydrazino-isonicotinsäure-*tert.*-butylester hergestellt. Man erhält 288 mg (33% d. Th.) der Titelverbindung als gelben Feststoff.
¹H-NMR (300 MHz, CDCl₃): δ = 12.7 (br. s, 1H), 8.97 (d, 1H), 8.47-8.41 (m, 3H), 8.03 (dt, 1H), 7.91 (s, 1H), 7.76 (dd, 1H), 7.32 (dd, 1H), 1.64 (s, 9H).
LC-MS (Methode 5): Rₜ = 1.75 min; MS (ESIpos): m/z = 339 [M+H]⁺.

### Beispiel 5

### 4-Pyridin-3-yl-2-[4-(trifluormethyl)pyridin-2-yl]-1,2-dihydro-3H-pyrazol-3-on

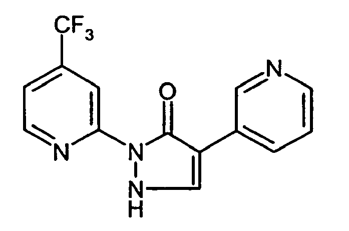

Die Verbindung wird analog zu Beispiel 2 aus 18 mg (0.09 mmol) der Verbindung aus Beispiel 2A und 18 mg (0.10 mmol) 2-Hydrazino-4-(trifluormethyl)pyridin [R.A. Evans, C. Wentrup, J. Chem. Soc. Chem. Commun. 15, 1062-1064 (1992)] hergestellt. Man erhält 11.7 mg (41% d. Th.) der Titelverbindung als gelben Feststoff.
¹H-NMR (400 MHz, CDCl₃): δ = 12.6 (br. s, 1H), 8.97 (s, 1H), 8.53 (d, 1H), 8.46 (d, 1H), 8.25 (s, 1H), 8.01 (d, 1H), 7.92 (s, 1H), 7.45 (d, 1H), 7.32 (dd, 1H).
LC-MS (Methode 5): Rₜ = 1.50 min; MS (ESIpos): m/z = 307 [M+H]⁺.

### Beispiel 6

### 2-Pyridin-2-yl-4-pyridin-3-yl-1,2-dihydro-3H-pyrazol-3-on

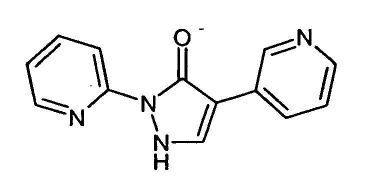

3.95 g (13.9 mmol) der Verbindung aus Beispiel 3A werden in 80 ml wasserfreiem Ethanol unter Argon vorgelegt und bei RT portionsweise mit 945 mg (13.9 mmol) Natriumethanolat versetzt. Nach 30 min. Rühren werden 13.9 ml 1 M Salzsäure zugetropft. Der ausgefallene Niederschlag wird abgesaugt, mit kaltem Ethanol (20 ml) und mit Wasser (zweimal je 20 ml) gewaschen und im Vakuum getrocknet. Man erhält 2.80 g (85% d. Th.) der Titelverbindung.
¹H-NMR (400 MHz, CDCl₃): δ = 13.5 (br. s, 1H), 8.97 (d, 1H), 8.44 (dd, 1H), 8.34 (d, 1H), 8.05-7.91 (m, 3H), 7.87 (s, 1H), 7.31 (dd, 1H), 7.25 (m, 1H).
HPLC (Methode 6): Rₜ = 3.00 min.
MS (DCI): m/z = 239 [M+H]⁺.

### Beispiel 7

### 4-(6-Chlorpyridin-3-yl)-2-pyridin-2-yl-1,2-dihydro-3H-pyrazol-3-on

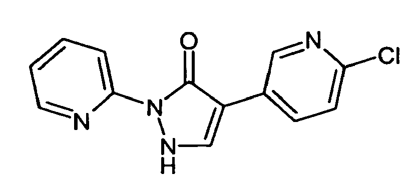

5.06 g (19.9 mmol) der Verbindung aus Beispiel 16A und 4.34 g (39.7 mmol) 2-Hydrazinopyridin werden in 100 ml Eisessig 2 h bei RT gerührt. Der Ansatz wird eingeengt, der Rückstand in 300 ml Essigsäureethylester aufgenommen und mit gesättigter Natriumhydrogencarbonat-Lösung (zweimal je 100 ml) gewaschen. Die organische Phase wird über Natriumsulfat getrocknet, filtriert und eingeengt. Der erhaltene Rückstand wird in 100 ml Ethanol aufgenommen, unter Eisbadkühlung portionsweise mit 1.49 g (21.9 mmol) Natriumethanolat versetzt und der Ansatz 30 min bei 0°C nachgerührt. Man versetzt die Reaktionslösung bei 0°C mit 22 ml 1 M Salzsäure und rührt für weitere 30 min bei dieser Temperatur. Der entstandene Niederschlag wird abgesaugt und mit kaltem Ethanol gewaschen. Nach Trocknen im Vakuum erhält man 3.18 g (59% d. Th.) der Titelverbindung.
¹H-NMR (300 MHz, DMSO-d₆): δ = 8.93 (s, 1H), 8.50 (d, 2H), 8.34 (d, 2H), 8.05 (dd, 1H), 7.51 (d, 1H), 7.36 (dd, 1H).
LC-MS (Methode 3): Rₜ = 2.27 min; MS (ESIpos): m/z = 273 [M+H]⁺.

Die in Tabelle 6 aufgeführten Beispiele werden analog zu Beispiel 7 aus den entsprechenden Edukten hergestellt:

**Tabelle 6**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **8** | 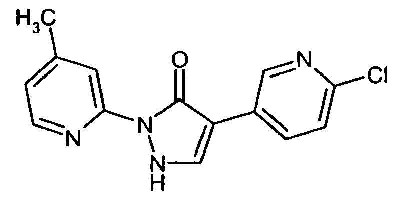 | 287 | 2.37 (3) | 49 |
| **9** | 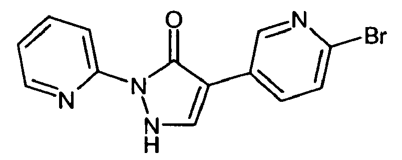 | 317 | 2.32 (5) | 63 |
| **10** | 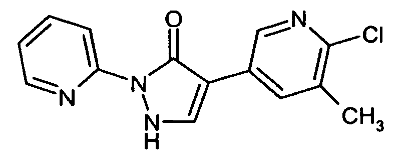 | 287 | 2.38 (3) | 97 |

### Beispiel 11

### 5-[2-(4-Methylpyridin-2-yl)-3-oxo-2,3-dihydro-1H-pyrazol-4-yl]pyridin-2-carbonitril

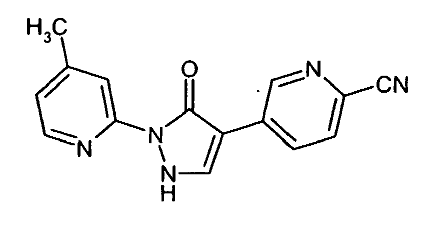

100 mg (0.35 mmol) der Verbindung aus Beispiel 8, 81.9 mg (0.70 mmol) Zinkcyanid und 40.3 mg (0.03 mmol) Tetrakis(triphenylphosphin)palladium(0) werden unter Argon in 4 ml wasserfreiem DMF vorgelegt und das Reaktionsgemisch 90 min lang bei 190°C unter Mikrowellenbestrahlung (Single-Mode-Gerät Explorer der Fa. CEM) gerührt. Man saugt die Reaktionsmischung über Kieselgur ab, wäscht den Rückstand mit DMF nach und engt das Filtrat am Rotationsverdampfer ein. Der so erhaltene Rückstand wird über präparative HPLC (Säule: YMC GEL ODS-AQ S-5/15 µm; Gradient: Acetonitril/Wasser + 0.2% TFA 10:90 → 95:5) chromatographiert. Der aus den vereinigten Produktfraktionen erhaltene Feststoff wird mit 6 ml Dichlormethan gewaschen, abgesaugt und im Vakuum getrocknet.
Ausbeute: 7 mg (7% d. Th.)
¹H-NMR (400 MHz, DMSO-d₆): δ = 9.23 (d, 1H), 8.55 (s, 1H), 8.43 (dd, 1H), 8.35 (d, 1H), 8.15 (s, 1H), 7.94 (d, 1H), 7.25 (d, 1H), 2.47 (s, 3H).
LC-MS (Methode 3): Rₜ = 2.01 min; MS (ESIpos): m/z = 278 [M+H]⁺.

### Beispiel 12

### 5-(3-Oxo-2-pyridin-2-yl-2,3-dihydro-1H-pyrazol-4-yl)pyridin-2-carbonitril

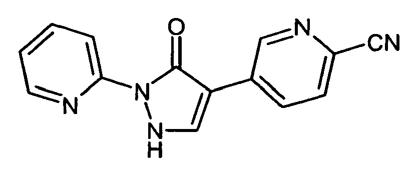

Die Darstellung erfolgt analog zu Beispiel 11 aus 100 mg (0.37 mmol) der Verbindung aus Beispiel 7.
Ausbeute: 12 mg (12% d. Th.)
¹H-NMR (400 MHz, DMSO-d₆): δ = 9.27 (s, 1H), 8.66 (s, 1H), 8.50 (m, 2H), 8.35 (d, 1H), 8.08 (dd, 1H), 7.97 (d, 1H), 7.38 (dd, 1H).
LC-MS (Methode 3): Rₜ = 1.70 min; MS (ESIpos): m/z = 264 [M+H]⁺.

### Beispiel 13

### 4-(5-Brompyridin-3-yl)-2-pyridin-2-yl-1,2-dihydro-3H-pyrazol-3-on

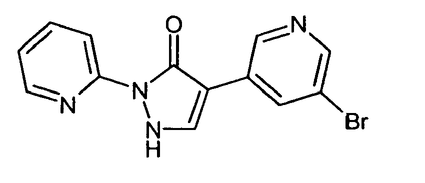

Die Synthese der Titelverbindung erfolgt analog zu Beispiel 7 aus 1.40 g (3.50 mmol) der Verbindung aus Beispiel 17A.
Ausbeute: 345 mg (31% d. Th.)
LC-MS (Methode 5): Rₜ = 2.24 min; MS (ESIpos): m/z = 319 [M+H]⁺.

### Beispiel 14

### 5-(3-Oxo-2-pyridin-2-yl-2,3-dihydro-1H-pyrazol-4-yl)nicotinonitril

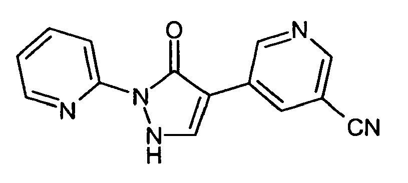

Die Darstellung erfolgt analog zu Beispiel 11 aus 50 mg (0.16 mmol) der Verbindung aus Beispiel 13.
Ausbeute: 20 mg (48% d. Th.)
¹H-NMR (300 MHz, DMSO-d₆): δ = 9.40 (d, 1H), 8.76 (d, 1H), 8.72 (m, 1H), 8.61 (s, 1H), 8.51 (d, 1H), 8.36 (d, 1H), 8.07 (dd, 1H), 7.37 (dd, 1H).
LC-MS (Methode 3): Rₜ = 1.65 min; MS (ESIpos): m/z = 264 [M+H]⁺.

### Beispiel 15

### 5-Methyl-2-pyridin-2-yl-4-pyridin-3-yl-1,2-dihydro-3H-pyrazol-3-on

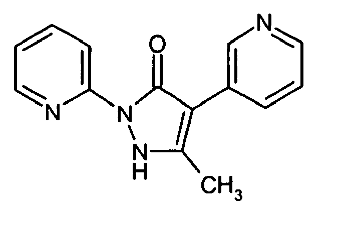

Zu einer Lösung von 58 mg (0.28 mmol) der Verbindung aus Beispiel 18A und 34 mg (0.31 mmol) 2-Hydrazinopyridin in 0.4 ml absolutem Ethanol werden 22 µl Eisessig gegeben und die Mischung über Nacht bei RT gerührt. Man versetzt mit 19 mg Natriumethylat, rührt 30 min bei RT nach und neutralisiert dann mit 1 N Salzsäure. Nach Zugabe von Wasser wird mit Dichlormethan extrahiert, die organische Phase über Magnesiumsulfat getrocknet, filtriert und eingeengt. Der Rückstand wird mit Diisopropylether verrührt und der Feststoff abgesaugt. Nach Trocknen erhält man 13.5 mg (19% d. Th.) der Titelverbindung.
¹H-NMR (400 MHz, CDCl₃): δ = 13.28 (br. s, 1H), 8.83 (s, 1H), 8.47 (d, 1H), 8.30 (d, 1H), 7.95-7.86 (m, 3H), 7.33 (dd, 1H), 7.18 (t, 1H), 2.44 (s, 3H).
LC-MS (Methode 1): Rₜ = 2.1 min; MS (ESIpos): m/z = 253 [M+H]⁺.

### Beispiel 16

### 4-(6-Hydroxypyridin-3-yl)-2-pyridin-2-yl-1,2-dihydro-3H-pyrazol-3-on

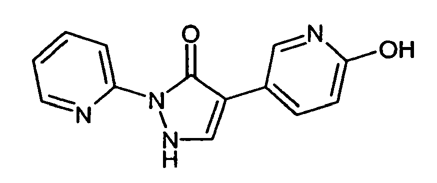

50.0 mg (0.18 mmol) der Verbindung aus Beispiel 7 und 600 mg (7.74 mmol) Ammoniumacetat werden als Suspension in 3 ml Eisessig in einer single mode-Mikrowelle (Explorer der Fa. CEM) für 2 h auf 180°C erhitzt. Nachdem per analytischer HPLC ein kompletter Umsatz festgestellt wird, wird Toluol zugegeben und die flüchtigen Komponenten azeotrop abdestilliert. Der Rückstand wird in Wasser aufgenommen und der verbleibende Feststoff abfiltriert. Das leicht bräunliche Pulver wird anschließend mit Wasser und dann mit MTBE gewaschen. Nach dem Trocknen werden 39 mg (84% d. Th.) der Titelverbindung erhalten.
¹H-NMR (300 MHz, DMSO-d₆): δ = 12.7 (br. s, 1H), 11.58 (br. s, 1H), 8.47 (d, 1H), 8.32 (m, 1H), 8.21 (s, 1H), 8.01 (m, 2H), 7.87 (dd, 1H), 7.32 (dd, 1H), 6.39 (d, 1H).
LC-MS (Methode 3): Rₜ = 1.31 min; MS (ESIpos): m/z = 255 [M+H]⁺.

Die in Tabelle 7 aufgeführten Beispiele werden analog zu Beispiel 7 aus den entsprechenden Edukten hergestellt:

**Tabelle 7**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **17** | 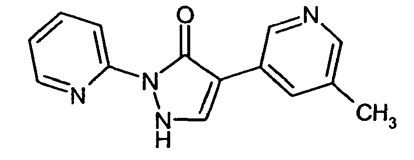 | 253 | 1.06 (5) | 9* |
| **18** | 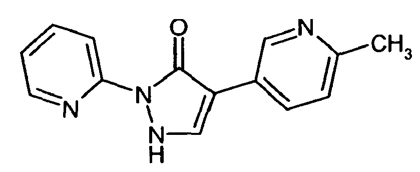 | 253 | 2.20 (1) | 13* |
| **19** | 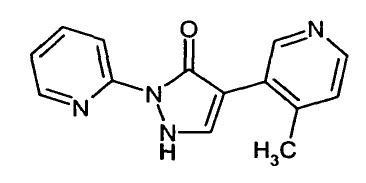 | 253 | 1.01 (5) | 30* |
| **20** | 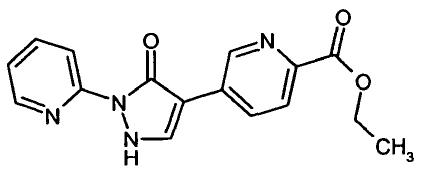 | 311 | 1.90 (3) | 31 |

| | | | | |
|---|---|---|---|---|
| [* Reinigung des Rohprodukts über präparative HPLC (Säule: YMC Gel ODS-AQ S-5 / 15 µm; Gradient: Acetonitril/Wasser + 0.2% Trifluoressigsäure 10:90 → 95:5)]. | | | | |

### Beispiel 21

### 4-[6-(Hydroxymethyl)pyridin-3-yl]-2-pyridin-2-yl-1,2-dihydro-3H-pyrazol-3-on

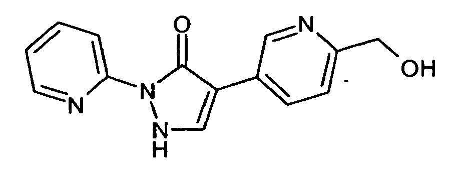

In 20 ml Ethanol werden 73 mg (1.93 mmol) Natriumborhydrid vorgelegt und bei 0°C 115 mg (1.03 mmol) Calciumchlorid zugesetzt. Die Verbindung aus Beispiel 20 (400 mg, 1.29 mmol) wird portionsweise zugegeben und der Ansatz 1 h bei 0°C, danach 4 h bei RT gerührt. Zur Vervollständigung der Reaktion werden weitere 73 mg (1.93 mmol) Natriumborhydrid zugefügt und der Ansatz 20 h bei RT gerührt. Man hydrolysiert mit 5 ml Wasser und stellt mit 1 N Salzsäure schwach sauer. Anschließend wird das Gemisch 1 h bei RT gerührt. Man engt ein, nimmt den Rückstand in ca. 16 ml DMSO/Wasser-Gemisch (1:1) auf und reinigt portionsweise über präparative HPLC (Säule: YMC Gel ODS-AQ S-5 / 15 µm; Gradient: Acetonitril/Wasser + 0.2% Trifluoressigsäure 10:90 → 95:5).
Ausbeute: 332 mg (96% d. Th.)
¹H-NMR (400 MHz, DMSO-d₆): δ = 9.19 (s, 1H), 8.71 (d, 1H), 8.64 (s, 1H), 8.51 (d, 1H), 8.37 (d, 1H), 8.09 (dd, 1H), 7.81 (d, 1H), 7.38 (dd, 1H), 4.77 (s, 2H).
LC-MS (Methode 3): Rₜ = 0.65 min; MS (ESIpos): m/z = 269 [M+H]⁺.

### Beispiel 22

### 2-Pyridin-2-yl-4-(6-trifluormethylpyridin-3-yl)-1,2-dihydro-3H-pyrazol-3-on

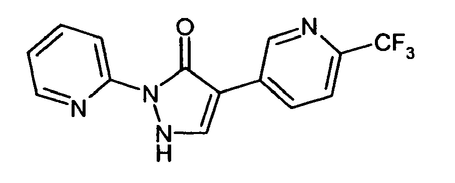

261 mg (1.00 mmol) der Verbindung aus Beispiel 41A und 115 mg (1.05 mmol) 2-Hydrazinopyridin werden in 5 ml wasserfreiem Ethanol unter Argon gelöst und der Ansatz 20 h bei RT gerührt. Man gibt 68 mg (1.00 mmol) Natriumethanolat hinzu, rührt 30 min bei RT und fügt dann 1 ml 1 M Salzsäure und wenig Ethanol zu, wobei ein Niederschlag ausfällt. Dieser wird abfiltriert, mit wenig Ethanol gewaschen und im Vakuum getrocknet.
Ausbeute: 180 mg (59% d. Th.)
¹H-NMR (400 MHz, DMSO-d₆): δ = 9.26 (s, 1H), 8.63 (s, 1H), 8.55 (d, 1H), 8.51 (d, 1H), 8.35 (d, 1H), 8.05 (t, 1H), 7.87 (d, 1H), 7.37 (dd, 1H).
LC-MS (Methode 4): Rₜ = 2.15 min; MS (ESIpos): m/z = 307 [M+H]⁺.

### Beispiel 23

### 2-(5-Hydroxymethyl-pyridin-2-yl)-4-pyridin-3-yl-1,2-dihydro-3H-pyrazol-3-on-Hydrochlorid

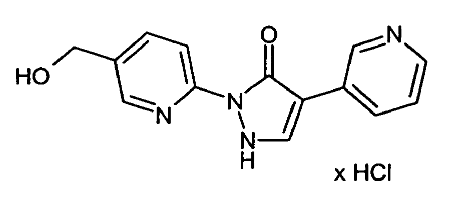

4.00 g (18.2 mmol) der Verbindung aus Beispiel 42A, 2.70 g (19.4 mmol) der Verbindung aus Beispiel 34A und 450 mg (1.94 mmol) Campher-10-sulfonsäure werden in 120 ml wasserfreiem Ethanol gelöst und der Ansatz über Nacht unter Rückfluss erhitzt. Nach dem Abkühlen wird der entstandene Niederschlag abgesaugt, mit Ethanol und Diethylether gewaschen, in Methanol suspendiert, mit einem Überschuss einer 4 N Lösung von Chlorwasserstoff in 1,4-Dioxan versetzt und erneut eingeengt. Der Rückstand wird mit einer Mischung aus Methanol und Dichlormethan verrührt, abgesaugt und getrocknet.
Ausbeute: 3.23 g (66% d. Th.)
¹H-NMR (400 MHz, DMSO-d₆): δ = 9.36 (s, 1H), 8.91 (d, 1H), 8.72 (s, 1H), 8.61 (d, 1H), 8.45-8.43 (m, 1H), 8.36 (d, 1H), 8.04 (dd, 1H), 7.98 (dd, 1H), 4.58 (s, 2H).
LC-MS (Methode 1): Rₜ = 2.12 min; MS (ESIpos): m/z = 269 [M+H]⁺.

Die in Tabelle 8 aufgeführten Verbindungen werden aus den entsprechenden Edukten analog zu Beispiel 23 hergestellt. Die Reinigung des jeweiligen Niederschlags kann alternativ mittels präparativer HPLC erfolgen (RP18-Säule; Laufmittel: Acetonitril/Wasser-Gradient, mit oder ohne Zusatz von 0.1% konz. Salzsäure).

**Tabelle 8**

| **Beispiel Nr.** | **Struktur** | **Edukte; Ausbeute (% d. Th.)** | **MS (ESI) [M+H]⁺; LC-MS - Rₜ [min] (Methode)** | **¹H-NMR** (400 MHz, DMSO-d₆) |
|---|---|---|---|---|
| **24** | 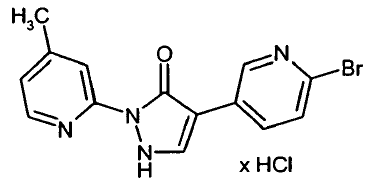 | 1A, 14A; 46% | m/z = 331; 2.24 min (4) | δ = 8.89 (d, 1H), 8.45 (s, 1H), 8.35 (d, 1H), 8.20 (dd, 1H), 8.11 (s, 1H), 7.62 (d, 1H), 7.22 (d, 1H), 2.44 (s, 3H). |
| **25** | 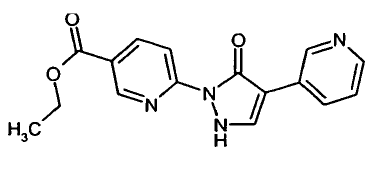 | 42A*; 25% | m/z=311; 1.42 min (3) | δ = 9.13 (s, 1H), 8.98 (s, 1H), 8.62-8.52 (m, 2H), 8.50-8.45 (m, 1H), 8.37 (d, 1H), 8.32 (d, 1H), 7.41 (dd, 1H), 4.37 (q, 2H), 1.35 (t, 3H). |
| **26** | 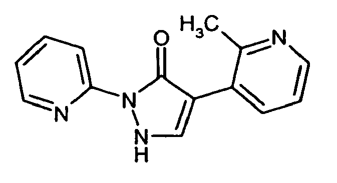 | 43A; 31% | m/z = 253; 2.16 min (1) | δ = 8.46 (d, 1H), 8.34-8.28 (m, 2H), 8.00 (dd, 1H), 7.75-7.69 (m, 1H), 7.52 (s, 1H), 7.06-6.96 (m, 2H), 2.56 (s, 3H). |
| **27** | 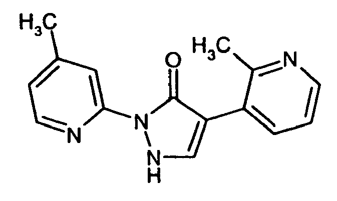 | 1A, 43A; 17% | m/z = 267; 1.13 min (5) | δ = 8.31-8.27 (m, 2H), 8.19 (d, 1H), 8.01 (dd, 1H), 7.52 (s, 1H), 7.04 (dd, 1H), 6.86 (d, 1H), 2.55 (s, 3H), 2.32 (s, 3H). |
| **28** | 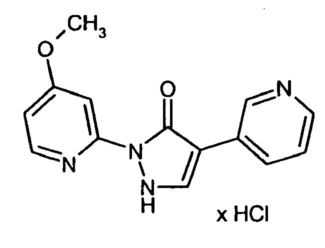 | 36A, 42A; 17% | m/z = 269; 1.25 min (5) | δ = 9.30 (d, 1H), 8.89 (d, 1H), 8.52 (s, 1H), 8.50 (d, 1H), 8.32 (d, 1H), 7.96 (dd, 1H), 7.85 (d, 1H), 7.06 (dd, 1H), 4.03 (s, 3H). |

| | | | | |
|---|---|---|---|---|
| [* 6-Hydrazinonicotinsäureethylester kann durch Veresterung von 6-Hydrazinonicotinsäure in Ethanol analog zu Beispiel 42 erhalten werden]. | | | | |

### Beispiel 29

### 2-(4-Cyanopyridin-2-yl)-4-pyridin-3-yl-1,2-dihydro-3H-pyrazol-3-on

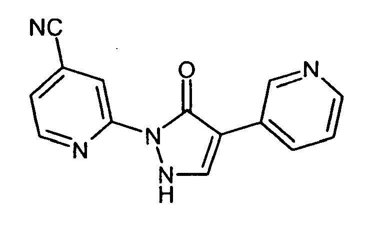

545 mg (4.06 mmol) der Verbindung aus Beispiel 33A und 1.07 g (4.88 mmol) der Verbindung aus Beispiel 42A werden in 15 ml Eisessig 2 h lang bei RT gerührt. Der Ansatz wird eingeengt, der Rückstand in 300 ml Essigsäureethylester aufgenommen und mehrfach mit gesättigter Natriumhydrogencarbonat-Lösung gewaschen. Die organische Phase wird über Natriumsulfat getrocknet, filtriert und eingeengt. Der Rückstand wird in 30 ml Ethanol aufgenommen, bei RT mit 1.33 g (4.88 mmol) einer 25%-igen Lösung von Natriumethanolat in Ethanol versetzt und 30 min gerührt. Durch Zugabe von 1 M Salzsäure stellt man einen pH-Wert von 5 ein, saugt den entstandenen Feststoff ab, wäscht ihn mit Diethylether und trocknet im Hochvakuum.
Ausbeute: 890 mg (83% d. Th.)
¹H-NMR (400 MHz, DMSO-d₆): δ = 9.01-8.98 (m, 2dz, 8.54 (dd, 1H), 8.18 (dt, 1H), 8.01 (dd, 1H), 7.85 (s, 1H), 7.39 (dd, 1H), 7.14 (dd, 1H).
LC-MS (Methode 5): Rₜ = 1.13 min; MS (ESIpos): m/z = 264 [M+H]⁺.

### Beispiel 30

### 2-(5-Chlorpyridin-2-yl)-4-pyridin-3-yl-1,2-dihydro-3H-pyrazol-3-on

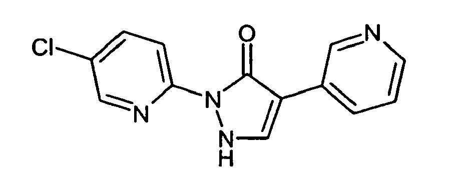

250 mg (1.74 mmol) der Verbindung aus Beispiel 30A und 460 mg (2.09 mmol) der Verbindung aus Beispiel 42A werden in 4 ml Eisessig 0.5 h lang bei RT gerührt. Der Ansatz wird eingeengt, der Rückstand in Essigsäureethylester aufgenommen und mehrfach mit gesättigter Natriumhydrogencarbonat-Lösung gewaschen. Die organische Phase wird über Magnesiumsulfat getrocknet, filtriert und eingeengt. Der Rückstand wird in 9 ml Ethanol aufgenommen, bei RT mit 664 mg (2.44 mmol) einer 25%-igen Lösung von Natriumethanolat in Ethanol versetzt und 1 h gerührt. Durch Zugabe von 1 M Salzsäure stellt man einen pH-Wert von 5 ein, rührt über Nacht bei RT, saugt den entstandenen Feststoff ab, wäscht ihn mit Wasser und trocknet im Hochvakuum.
Ausbeute: 239 mg (50% d. Th.)
¹H-NMR (400 MHz, DMSO-d₆): δ = 9.04 (d, 1H), 8.51 (d, 1H), 8.39 (d, 1H), 8.22 (dt, 1H), 8.10 (dd, 1H), 8.00 (s, 1H), 7.92 (dd, 1H), 7.20 (dd, 1 H).
LC-MS (Methode 5): Rₜ = 1.33 min; MS (ESIpos): m/z = 273 [M+H]⁺.

### Beispiel 31

### 2-(5-Iodpyridin-2-yl)-4-pyridin-3-yl-1,2-dihydro-3H-pyrazol-3-on

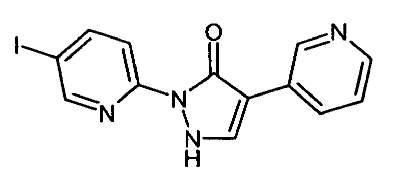

Die Synthese der Titelverbindung erfolgt analog zu Beispiel 30 aus 250 mg (1.06 mmol) der Verbindung aus Beispiel 32A und 281 mg (1.28 mmol) der Verbindung aus Beispiel 42A.
Ausbeute: 80 mg (21 % d. Th.)
¹H-NMR (400 MHz, DMSO-d₆): δ = 9.12 (s, 1H), 8.70 (s, 1H), 8.54-8.46 (m, 1H), 8.40-8.25 (m, 4H), 7.43-7.36 (m, 1H).
LC-MS (Methode 3): Rₜ = 1.44 min; MS (ESIpos): m/z = 365 [M+H]⁺.

### Beispiel 32

### 2-(5-Brompyridin-2-yl)-4-pyridin-3-yl-1,2-dihydro-3H-pyrazol-3-on

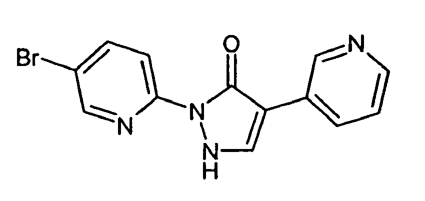

Die Synthese der Titelverbindung erfolgt analog zu Beispiel 30 aus 250 mg (1.33 mmol) der Verbindung aus Beispiel 31A und 351 mg (1.60 mmol) der Verbindung aus Beispiel 42A.
Ausbeute: 166 mg (39% d. Th.)
¹H-NMR (400 MHz, DMSO-d₆): δ = 9.06 (d, 1H), 8.50 (d, 1H), 8.46 (s, 1H), 8.24 (d, 1H), 8.16 (d, 1H), 8.13 (s, 1H), 8.08 (dd, 1H), 7.24 (dd, 1H).
LC-MS (Methode 5): Rₜ = 1.40 min; MS (ESIpos): m/z = 318 [M+H]⁺.

### Beispiel 33

### 2-(5-Brom-4-methylpyridin-2-yl)-4-pyridin-3-yl-1,2-dihydro-3H-pyrazol-3-on

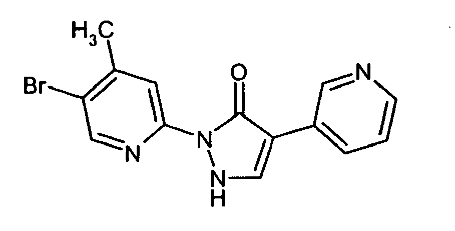

300 mg (1.49 mmol) der Verbindung aus Beispiel 29A und 392 mg (1.78 mmol) der Verbindung aus Beispiel 42A werden 36 h lang bei RT in 7 ml Eisessig gerührt. Der Ansatz wird eingeengt, der Rückstand in Essigsäureethylester aufgenommen und mehrfach mit gesättigter Natriumhydrogencarbonat-Lösung gewaschen. Die organische Phase wird über Magnesiumsulfat getrocknet, filtriert und eingeengt. Der Rückstand wird in 13 ml Ethanol aufgenommen, bei RT mit 566 mg (2.08 mmol) einer 25%-igen Lösung von Natriumethanolat in Ethanol versetzt und 1 h gerührt. Durch Zugabe von 1 M Salzsäure stellt man einen pH-Wert von 5 ein, engt ein und reinigt den Rückstand mittels präparativer HPLC (RP18-Säule; Laufmittel: Acetonitril/Wasser-Gradient unter Zusatz von 0.1% konz. Salzsäure).
Ausbeute: 55 mg (11% d. Th.)
¹H-NMR (400 MHz, DMSO-d₆): δ = 9.41 (s, 1H), 8.99 (d, 1H), 8.86 (s, 1H), 8.67 (d, 1H), 8.60 (s, 1H), 8.45 (s, 1H), 8.03 (dd, 1H), 2.47 (s, 3H).
LC-MS (Methode 3): Rₜ = 1.53 min; MS (ESIpos): m/z = 331 [M+H]⁺.

### Beispiel 34

### 4-(6-Cyanopyridin-3-yl)-2-(4-methylpyridin-2-yl)-1,2-dihydro-3H-pyrazol-3-on-Hydrochlorid

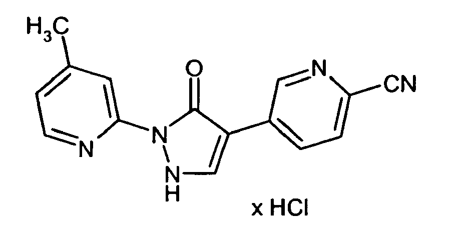

50 mg (136 µmol) der Verbindung aus Beispiel 24 werden in 0.6 ml 1-Methyl-2-pyrrolidon gelöst, mit 31.9 mg (272 µmol) Zinkcyanid und 15.7 mg (14 µmol) Tetrakis(triphenylphosphin)palladium(0) versetzt und 30 min lang bei 200°C in der Mikrowelle erhitzt. Die Reaktionsmischung wird über Kieselgur filtriert und mit Methanol eluiert. Das Filtrat stellt man durch Zugabe von 1 M Salzsäure auf einen leicht sauren pH-Wert ein, filtriert den Niederschlag ab und reinigt diesen mittels präparativer HPLC (RP18-Säule; Laufmittel: Acetonitril/Wasser-Gradient unter Zusatz von 0.1% konz. Salzsäure).
Ausbeute: 13 mg (31% d. Th.)
¹H-NMR (400 MHz, DMSO-d₆): δ = 9.25 (s, 1H), 8.57 (s, 1H), 8.44 (d, 1H), 8.36 (d, 1H), 8.15 (s, 1H), 7.95 (d, 1H), 7.25 (d, 1H), 2.47 (s, 3H).
LC-MS (Methode 4): Rₜ = 2.20 min; MS (ESIpos): m/z = 278 [M+H]⁺.

### Beispiel 35

### 2-[4-(Aminomethyl)pyridin-2-yl]-4-pyridin-3-yl-1,2-dihydro-3H-pyrazol-3-on-Dihydrochlorid

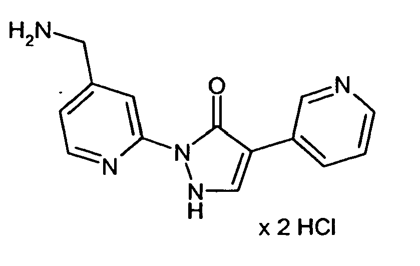

100 mg (380 µmol) der Verbindung aus Beispiel 29 werden in 10 ml Eisessig gelöst, mit 50.0 mg Katalysator (10% Palladium auf Kohle) versetzt und über Nacht unter Wasserstoffatmosphäre bei Normaldruck und RT gerührt. Anschließend wird die Reaktionsmischung filtriert, eingeengt und der Rückstand mittels präparativer HPLC (RP18-Säule; Laufmittel: Acetonitril/Wasser-Gradient unter Zusatz von 0.1% konz. Salzsäure) gereinigt.
Ausbeute: 64 mg (49% d. Th.)
¹H-NMR (400 MHz, DMSO-d₆): δ = 9.38 (s, 1H), 8.93 (d, 1H), 8.79 (s, 1H), 8.75 (s, 3H), 8.64 (d, 1H), 8.56 (d, 1H), 8.48 (s, 1H), 7.99 (dd, 1H), 7.54 (d, 1H), 4.21 (q, 2H).
LC-MS (Methode 1): Rₜ = 1.39 min; MS (ESIpos): m/z = 268 [M+H]⁺.

### Beispiel 36

### N-{[2-(5-Oxo-4-pyridin-3-yl-2,5-dihydro-1H-pyrazol-1-yl)pyridin-4-yl]methyl}butanamid-Hydrochlorid

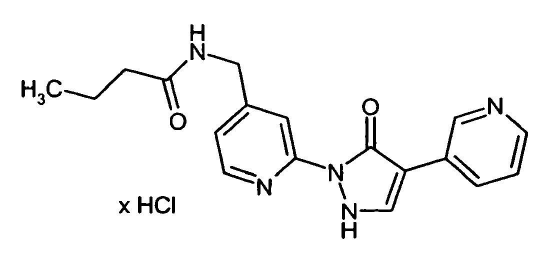

80.0 mg (235 µmol) der Verbindung aus Beispiel 35 und 22.8 mg (259 µmol) Buttersäure werden in 5 ml DMF gelöst, unter Eiskühlung mit 119 mg (1.18 mmol) Triethylamin und 90.2 mg (470 µmol) 1-(3-Dimethylaminopropyl)-3-ethylcarbodiimid-Hydrochlorid versetzt und über Nacht bei RT gerührt. Anschließend wird die Reaktionsmischung direkt mittels präparativer HPLC (RP18-Säule; Laufmittel: Acetonitril/Wasser-Gradient unter Zusatz von 0.1% konz. Salzsäure) aufgereinigt.
Ausbeute: 7 mg (7% d. Th.)
¹H-NMR (400 MHz, DMSO-d₆): δ = 9.33-9.31 (m, 1H), 8.87 (d, 1H), 8.66 (s, 1H), 8.61-8.56 (m, 2H), 8.43 (d, 1H), 8.25 (s, 1H), 7.96 (dd, 1H), 7.26 (d, 1H), 4.41 (d, 1H), 2.19 (t, 2H), 1.58 (sext, 2H), 0.90 (t, 3H).
LC-MS (Methode 1): Rₜ = 2.26 min; MS (ESIpos): m/z = 338 [M+H]⁺.

### Beispiel 37

### N-Isopropyl-N'-{[2-(5-oxo-4-pyridin-3-yl-2,5-dihydro-1H-pyrazol-1-yl)pyridin-4-yl]methyl}harnstoff-Hydrochlorid

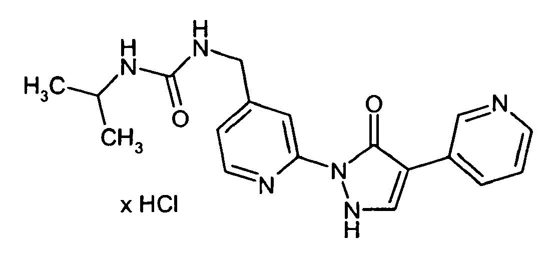

40.0 mg (470 µmol) Isopropylisocyanat werden unter Argon in 5 ml DMF gelöst, mit 80.0 mg (235 µmol) der Verbindung aus Beispiel 35 und 71.4 mg (705 µmol) Triethylamin versetzt und über Nacht bei RT gerührt. Anschließend engt man ein und reinigt den Rückstand mittels präparativer HPLC (RP18-Säule; Laufmittel: Acetonitril/Wasser-Gradient unter Zusatz von 0.1% konz. Salzsäure).
Ausbeute: 80 mg (85% d. Th.)
¹H-NMR (400 MHz, DMSO-d₆): δ = 9.35 (s, 1H), 8.95 (d, 1H), 8.66 (s, 1H), 8.59 (d, 1H), 8.42 (d, 1H), 8.24 (s, 1H), 8.02 (dd, 1H), 7.27 (d, 1H), 6.55 (s, 1H), 4.35 (s, 2H), 3.76-3.63 (m, 1H), 2.75 (s, 1H), 1.08-1.03 (m, 6H).
LC-MS (Methode 1): Rₜ = 2.81 min; MS (ESIpos): m/z = 353 [M+H]⁺.

### Beispiel 38

### N-{[2-(5-Oxo-4-pyridin-3-yl-2,5-dihydro-1H-pyrazol-1-yl)pyridin-4-yl]methyl}methansulfonamid-Hydrochlorid

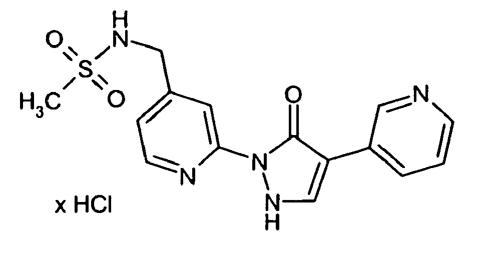

80.0 mg (235 µmol) der Verbindung aus Beispiel 35 und 53.9 mg (470 µmol) Methansulfonsäurechlorid werden unter Argon und Eiskühlung in 5 ml DMF gelöst, mit 152 mg (1.18 mmol) *N,N-*Diisopropylethylamin versetzt und über Nacht bei RT gerührt. Anschließend reinigt man die Reaktionsmischung direkt mittels präparativer HPLC (RP18-Säule; Laufmittel: Acetonitril/Wasser-Gradient unter Zusatz von 0.1% konz. Salzsäure).
Ausbeute: 31 mg (34% d. Th.)
¹H-NMR (400 MHz, DMSO-d₆): δ = 9.34 (s, 1H), 8.88 (d, 1H), 8.69 (s, 1H), 8.59 (d, 1H), 8.47 (d, 1H), 8.40 (s, 1H), 7.95 (dd, 1H), 7.89 (t, 1H), 7.36 (d, 1H), 4.36 (d, 2H), 2.99 (s, 3H).
LC-MS (Methode 1): Rₜ = 2.35 min; MS (ESIpos): m/z = 346 [M+H]⁺.

### Beispiel 39

### 6-(5-Oxo-4-pyridin-3-yl-2,5-dihydro-1H-pyrazol-1-yl)-nicotinsäure

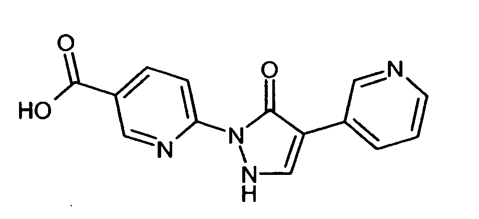

1.49 g (4.81 mmol) der Verbindung aus Beispiel 25 werden in 60 ml 1,4-Dioxan gelöst, mit 40 ml einer 1 M wässrigen Lithiumhydroxid-Lösung versetzt und 1 h unter Rückfluss erhitzt. Anschließend wird die Reaktionsmischung auf 0°C abgekühlt, mit 40 ml 1 M Salzsäure auf einen schwach sauren pH-Wert eingestellt und 2 h bei 0°C gerührt. Man saugt den entstandenen Niederschlag ab, wäscht ihn mit Wasser und Diethylether und trocknet im Hochvakuum.
Ausbeute: 1.20 g (88% d. Th.)
¹H-NMR (400 MHz, DMSO-d₆): δ = 9.40 (s, 1H), 9.00-8.94 (m, 2H), 8.90 (s, 1H), 8.66 (d, 1H), 8.60-8.54 (m, 1H), 8.49 (dd, 1H), 8.02 (dd, 1H).
LC-MS (Methode 3): Rₜ = 0.63 min; MS (ESIpos): m/z = 283 [M+H]⁺.

### Beispiel 40

### N-Benzyl-6-(5-oxo-4-pyridin-3-yl-2,5-dihydro-1H-pyrazol-1-yl)-nicotinamid-Hydrochlorid

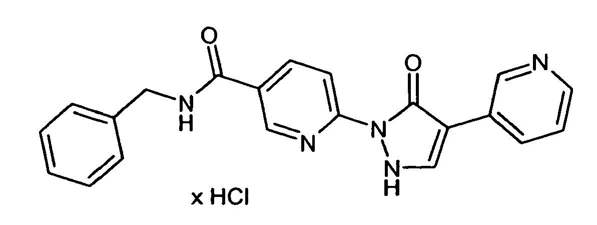

50 mg (177 µmol) der Verbindung aus Beispiel 39 werden in 2 ml DMF gelöst, mit 1.9 mg (16 µmol) 4-*N,N*-Dimethylaminopyridin, 71.0 mg (549 µmol) *N,N*-Diisopropylethylamin und 98.0 mg (188 µmol) (Benzotriazol-1-yloxy)-tripyrrolidinophosphonium-hexafluorophosphat versetzt und 15 min bei RT gerührt. Man gibt 25.2 mg (235 µmol) Benzylamin hinzu und rührt weitere 5 h bei RT. Zur Vervollständigung des Umsatzes setzt man weitere 25 mg (235 µmol) Benzylamin zu und rührt über Nacht bei RT. Man reinigt die Reaktionsmischung mittels präparativer HPLC (RP18-Säule; Laufmittel: Acetonitril/Wasser-Gradient unter Zusatz von 0.1% konz. Salzsäure) und nachfolgender Flash-Chromatographie an Kieselgel (Laufmittel: Dichlormethan/Methanol-Gradient) vor, fällt das Rohprodukt aus Methanol aus und reinigt den Niederschlag erneut mittels präparativer HPLC (RP18-Säule; Laufmittel: Acetonitril/Wasser-Gradient unter Zusatz von 0.1% konz. Salzsäure).
Ausbeute: 21 mg (30% d. Th.)
¹H-NMR (400 MHz, DMSO-d₆): δ = 9.41 (s, 1H), 9.38-9.33 (m, 1H), 9.01 (s, 1H), 8.95 (d, 1H), 8.87 (s, 1H), 8.64 (d, 1H), 8.56-8.49 (m, 2H), 7.99 (dd, 1H), 7.38-7.32 (m, 4H), 7.30-7.24 (m, 1H), 4.53 (d, 2H).
LC-MS (Methode 5): Rₜ = 1.50 min; MS (ESIpos): m/z = 372 [M+H]⁺.

### Beispiel 41

### 2-(5-Oxo-4-pyridin-3-yl-2,5-dihydro-1H-pyrazol-1-yl)-isonicotinsäure

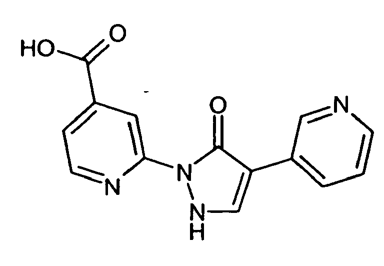

200 mg (760 µmol) der Verbindung aus Beispiel 29 werden in einer Mischung aus 6 ml Ethanol und 4 ml Wasser suspendiert, mit 0.6 ml 50%-iger Natronlauge versetzt und 1 h unter Rückfluss erhitzt. Nach dem Abkühlen wird mit 1 M Salzsäure auf einen schwach sauren pH-Wert eingestellt und der Niederschlag abgesaugt, mit Wasser gewaschen und getrocknet.
Ausbeute: 180 mg (84% d. Th.)
¹H-NMR (400 MHz, DMSO-d₆): δ = 9.11 (d, 1H), 8.86 (s, 1H), 8.62 (d, 1H), 8.46 (s, 1H), 8.32 (dd, 1H), 8.28 (dt, 1H), 7.69 (dd, 1H), 7.36 (dd, 1H).
LC-MS (Methode 1): Rₜ = 2.19 min; MS (ESIpos): m/z = 283 [M+H]⁺.

### Beispiel 42

### 2-(5-Oxo-4-pyridin-3-yl-2,5-dihydro-1H-pyrazol-1-yl)-isonicotinsäuremethylester

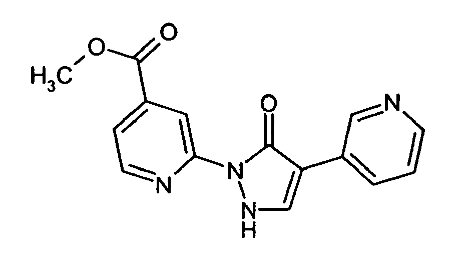

150 mg (531 µmol) der Verbindung aus Beispiel 41 werden in 20 ml Methanol gelöst, mit 1 ml konzentrierter Schwefelsäure versetzt und über Nacht unter Rückfluss erhitzt. Nach dem Abkühlen wird der entstandene Niederschlag abgesaugt, mit Methanol gewaschen und getrocknet.
Ausbeute: 117 mg (74% d. Th.)
¹H-NMR (400 MHz, DMSO-d₆): δ = 9.41 (d, 1H), 8.99-8.94 (m, 2H), 8.86 (s, 1H), 8.71 (d, 1H), 8.66 (d, 1H), 8.01 (dd, 1H), 7.78 (dd, 1H), 3.96 (s, 3H).
LC-MS (Methode 4): Rₜ = 1.03 min; MS (ESIpos): m/z = 297 [M+H]⁺.

### Beispiel 43

### 2-[4-(Hydroxymethyl)pyridin-2-yl]-4-pyridin-3-yl-1,2-dihydro-3H-pyrazol-3-on-Hydrochlorid

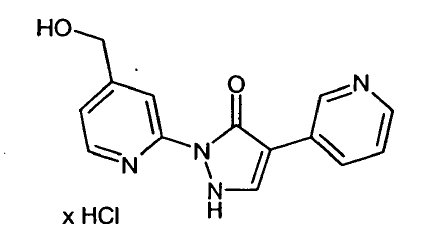

197 mg (1.77 mmol) Calciumchlorid und 319 mg (8.44 mmol) Natriumborhydrid werden in 26 ml Ethanol vorgelegt, bei 0°C portionsweise mit 50 mg (169 µmol) der Verbindung aus Beispiel 42 versetzt und über Nacht bei RT gerührt. Das Reaktionsgemisch stellt man durch Zugabe von 1 M Salzsäure auf einen leicht sauren pH-Wert ein, engt ein und reinigt den Rückstand mittels präparativer HPLC (RP18-Säule; Laufmittel: Acetonitril/Wasser-Gradient unter Zusatz von 0.1% konz. Salzsäure).
Ausbeute: 32 mg (63% d. Th.)
¹H-NMR (400 MHz, DMSO-d₆): δ = 9.34 (s, 1H), 8.93 (d, 1H), 8.66 (s, 1H), 8.58 (d, 1H), 8.42 (d, 1H), 8.36 (s, 1H), 8.00 (dd, 1H), 7.34 (d, 1H), 4.68 (s, 2H).
LC-MS (Methode 1): Rₜ = 2.14 min; MS (ESIpos): m/z = 269 [M+H]⁺.

### Beispiel 44

### 5-(3-Oxo-2-pyridin-2-yl-2,3-dihydro-1H-pyrazol-4-yl)-nicotinsäure

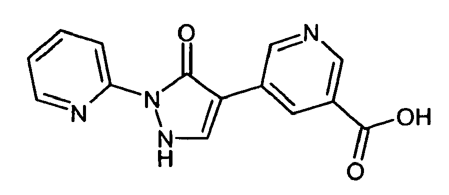

100 mg (380 µmol) der Verbindung aus Beispiel 14 werden in einer Mischung aus 3 ml Ethanol und 2 ml Wasser suspendiert, mit 0.3 ml 50%-iger Natronlauge versetzt und 2 h unter Rückfluss erhitzt. Nach dem Abkühlen wird der Niederschlag abgesaugt, mit Diethylether gewaschen und getrocknet.
Ausbeute: 50 mg (47% d. Th.)
¹H-NMR (400 MHz, DMSO-d₆): δ = 9.04 (d, 1H), 8.47-8.42 (m, 2H), 8.36-8.33 (m, 2H), 7.75-7.70 (m, 1H), 7.68 (s, 1H), 7.01-6.97 (m, 1H).
LC-MS (Methode 5): Rₜ = 1.28 min; MS (ESIpos): m/z = 283 [M+H]⁺.

### Beispiel 45

### N-Methyl-5-(3-oxo-2-pyridin-2-yl-2,3-dihydro-1H-pyrazol-4-yl)-nicotinamid

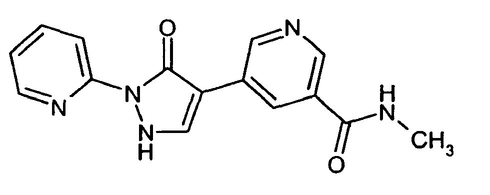

45.0 mg (159 µmol) der Verbindung aus Beispiel 44 werden in 1 ml DMF gelöst, mit 1.9 mg (16 µmol) 4-*N*,*N*-Dimethylaminopyridin, 24.7 mg (191 µmol) *N*,*N-*Diisopropylethylamin und 100 mg (191 µmol) (Benzotriazol-1-yloxy)-tripyrrolidinophosphonium-hexafluorophosphat versetzt und 15 min bei RT gerührt. Man gibt 120 µl (239 µmol) einer 2 M Lösung von Methylamin in THF hinzu und rührt über Nacht bei RT. Zur Vervollständigung des Umsatzes setzt man weitere 120 µl (239 µmol) der 2 M Lösung von Methylamin in THF zu und rührt erneut über Nacht bei RT. Man reinigt die Reaktionsmischung direkt mittels präparativer HPLC (RP18-Säule; Laufmittel: Acetonitril/Wasser-Gradient).
Ausbeute: 23 mg (49% d. Th.)
¹H-NMR (400 MHz, DMSO-d₆): δ = 9.18 (s, 1H), 8.74 (d, 1H), 8.64-8.57 (m, 2H), 8.53-8.44 (m, 2H), 8.40-8.25 (m, 1H), 8.09-8.03 (m, 1H), 7.40-7.34 (m, 1H), 2.83 (d, 3H).
LC-MS (Methode 3): R₁ = 1.21 min; MS (ESIpos): m/z = 296 [M+H]⁺.

### Beispiel 46

### N-{[2-(5-Oxo-4-pyridin-3-yl-2,5-dihydro-1H-pyrazol-1-yl)pyridin-4-yl]methyl}-2-phenylacetamid-Hydrochlorid

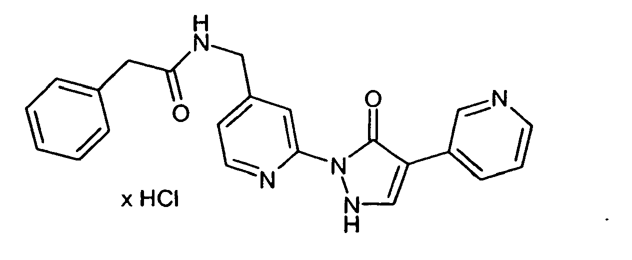

80.0 mg (235 µmol) der Verbindung aus Beispiel 35 und 35.2 mg (259 µmol) Phenylessigsäure werden in 5 ml DMF gelöst, unter Eiskühlung mit 119 mg (1.18 mmol) Triethylamin, 90.2 mg (470 µmol) 1-(3-Dimethylaminopropyl)-3-ethylcarbodiimid-Hydrochlorid sowie 127 mg (941 µmol) 1-Hydroxy-1*H*-benzotriazol-Hydrat versetzt und über Nacht bei RT gerührt. Der Niederschlag wird abfiltriert und das Filtrat mittels präparativer HPLC gereinigt (RP18-Säule; Laufmittel: Acetonitril/Wasser-Gradient unter Zusatz von 0.1 % konz. Salzsäure).
Ausbeute: 58 mg (57% d. Th.)
¹H-NMR (400 MHz, DMSO-d₆): δ = 9.38 (d, 1H), 8.97 (dt, 1H), 8.88 (t, 1H), 8.72 (s, 1H), 8.62 (d, 1H), 8.41 (d, 1H), 8.29 (s, 1H), 8.04 (dd, 1H), 7.36-7.29 (m, 4H), 7.26-7.21 (m, 2H), 4.42 (d, 2H), 3.56 (s, 2H).
LC-MS (Methode 3): Rₜ = 1.30 min; MS (ESIpos): m/z = 386 [M+H]⁺.

### Beispiel 47

### N-{[2-(5-Oxo-4-pyridin-3-yl-2,5-dihydro-1H-pyrazol-1-yl)pyridin-4-yl]methyl}acetamid-Hydrochlorid

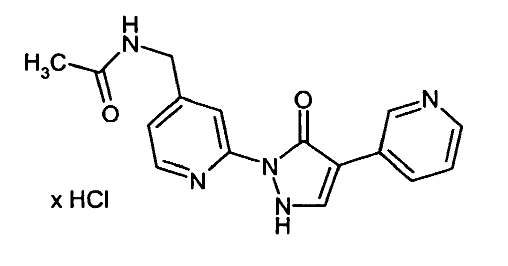

80.0 mg (235 µmol) der Verbindung aus Beispiel 35 werden in 5 ml DMF gelöst, unter Eiskühlung mit 71.4 mg (705 µmol) Triethylamin und 20.3 mg (259 µmol) Acetylchlorid versetzt und über Nacht bei RT gerührt. Die Reaktionsmischung wird direkt mittels präparativer HPLC (RP18-Säule; Laufmittel: Acetonitril/Wasser-Gradient unter Zusatz von 0.1 % konz. Salzsäure) aufgereinigt.
Ausbeute: 33 mg (41% d. Th.)
¹H-NMR (400 MHz, DMSO-d₆): δ = 9.37 (s, 1H), 8.97 (d, 1H), 8.71 (s, 1H), 8.68 (t, 1H), 8.61 (d, 1H), 8.43 (d, 1H), 8.26 (s, 1H), 8.03 (dd, 1H), 7.28 (d, 1H), 4.40 (d, 2H), 1.95 (s, 3H).
LC-MS (Methode 1): Rₜ = 2.09 min; MS (ESIpos): m/z = 310 [M+H]⁺.

### Beispiel 48

### N-{[2-(5-Oxo-4-pyridin-3-yl-2,5-dihydro-1H-pyrazol-1-yl)pyridin-4-yl]methyl}benzamid-Hydrochlorid

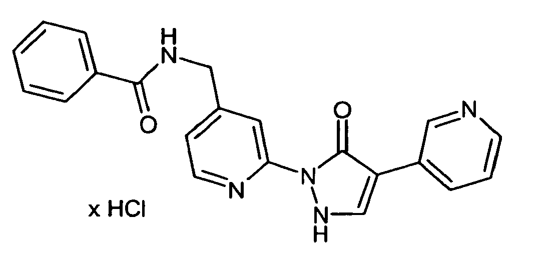

60.0 mg (176 µmol) der Verbindung aus Beispiel 35 werden in 4 ml DMF gelöst, unter Eiskühlung mit 53.5 mg (529 µmol) Triethylamin und 27.3 mg (194 µmol) Benzoylchlorid versetzt und über Nacht bei RT gerührt. Die Reaktionsmischung wird direkt mittels präparativer HPLC (RP 18-Säule; Laufmittel: Acetonitril/Wasser-Gradient unter Zusatz von 0.1% konz. Salzsäure) aufgereinigt.
Ausbeute: 36 mg (50% d. Th.)
¹H-NMR (400 MHz, DMSO-d₆): δ = 9.35-9.29 (m, 2H), 8.91 (d, 1H), 8.68 (s, 1H), 8.59 (d, 1H), 8.44 (d, 1H), 8.34 (s, 1H), 8.01-7.92 (m, 3H), 7.62-7.48 (m, 3H), 7.35 (d, 1H), 4.62 (d, 2H).
LC-MS (Methode 4): Rₜ = 1.07 min; MS (ESIpos): m/z = 372 [M+H]⁺.

### Beispiel 49

### N-Benzyl-2-(5-oxo-4-pyridin-3-yl-2,5-dihydro-1H-pyrazol-1-yl)-isonicotinamid

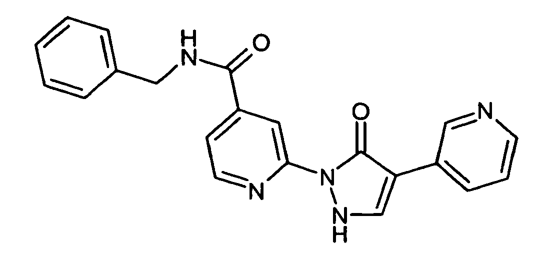

60.0 mg (213 µmol) der Verbindung aus Beispiel 41 werden in 24 ml DMF gelöst, mit 2.6 mg (21 µmol) 4-*N*,*N*-Dimethylaminopyridin, 65.9 mg (510 µmol) *N,N*-Diisopropylethylamin und 265 mg (510 µmol) (Benzotriazol-1-yloxy)-tripyrrolidinophosphonium-hexafluorophosphat versetzt und 45 min bei RT gerührt. Man gibt 68.3 mg (638 µmol) Benzylamin hinzu und rührt über Nacht bei RT. Zur Vervollständigung des Umsatzes setzt man weitere 65.9 mg (510 µmol) *N,N*-Diisopropylethylamin und 265 mg (510 µmol) (Benzotriazol-1-yloxy)-tripyrrolidinophosphonium-hexafluorophosphat zu, rührt für 45 min bei RT, fügt weitere 68.3 mg (638 µmol) Benzylamin hinzu und rührt dann über Nacht bei RT. Man engt ein und reinigt den Rückstand mittels präparativer HPLC (RP18-Säule; Laufmittel: Acetonitril/Wasser-Gradient unter Zusatz von 0.1% Ameisensäure).
Ausbeute: 35 mg (44% d. Th.)
¹H-NMR (400 MHz, DMSO-d₆): δ = 9.49 (t, 1H), 9.26 (s, 1H), 8.79 (s, 1H), 8.70-8.62 (m, 3H), 8.52 (d, 1H), 7.76-7.70 (m, 2H), 7.37-7.34 (m, 4H), 7.31-7.24 (m, 1H), 4.52 (d, 2H).
LC-MS (Methode 5): Rₜ = 1.57 min; MS (ESIpos): m/z = 372 [M+H]⁺.

### Beispiel 50

### 3-(4-Chlor-1H-pyrazol-1-yl)-N-{[2-(5-oxo-4-pyridin-3-yl-2,5-dihydro-1H-pyrazol-1-yl)pyridin-4-yl]methyl}propanamid

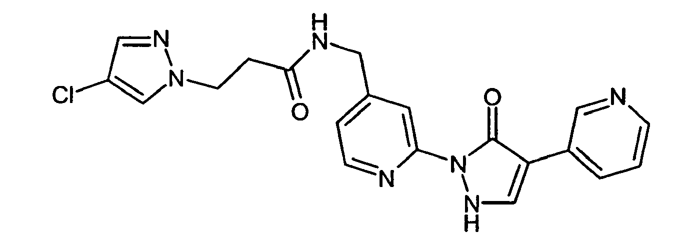

17.5 mg (100 µmol) 3-(4-Chlor-1*H*-pyrazol-1-yl)propansäure, 41.7 mg (130 µmol) O-(Benzotriazol-1-yl)-*N,N,N',N'*-tetramethyluronium-tetrafluoroborat und 20.2 mg (200 µmol) Triethylamin werden in 0.2 ml DMSO gelöst. Man gibt eine Lösung von 33.9 mg (100 µmol) der Verbindung aus Beispiel 35 in 0.2 ml DMSO hinzu und rührt die Reaktionsmischung über Nacht bei RT. Der entstandene Niederschlag wird abfiltriert und das Filtrat mittels HPLC (Methode 8) aufgereinigt.
Ausbeute: 5.8 mg (14% d. Th.)
LC-MS (Methode 8): Rₜ = 1.20 min; MS (ESIpos): m/z = 424 [M+H]⁺.

Die in Tabelle 9 aufgeführten Verbindungen werden analog zu Beispiel 50 aus Beispiel 35 und den entsprechenden Carbonsäuren hergestellt:

**Tabelle 9**

| **Beispiel Nr.** | **Struktur** | **MS (ESI) [M+H]⁺; LC-MS Rₜ [min] (Methode)** | **Ausbeute (% d. Th.)** |
|---|---|---|---|
| **51** | 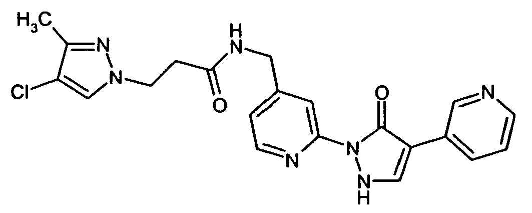 | m/z = 438; 1.27 min (8) | 12 |
| **52** | 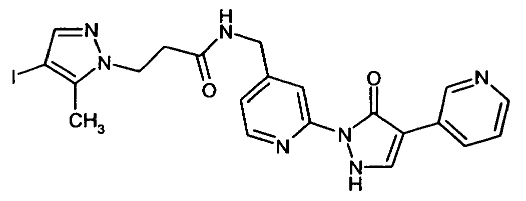 | m/z = 530; 1.32 min (8) | 11 |
| **53** | 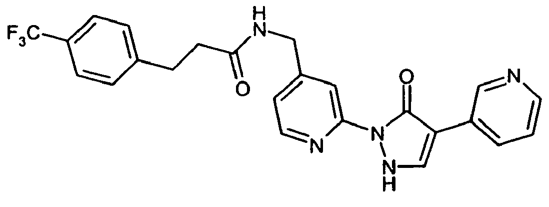 | m/z = 468; 1.48 min (8) | 18 |
| **54** | 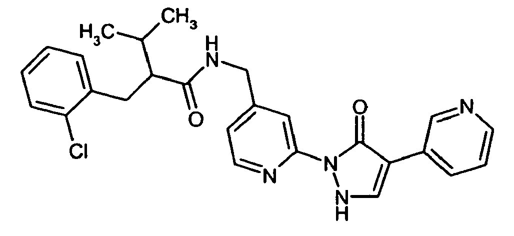 | m/z = 476; 1.60 min (8) | 9 |
| **55** | 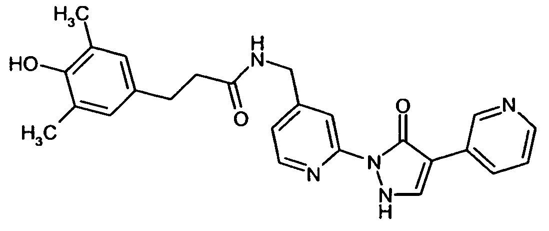 | m/z = 444; 1.28 min (8) | 16 |
| **56** | 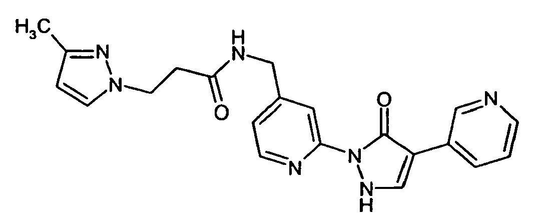 | m/z = 404; 1.13 min (8) | 14 |
| **57** | 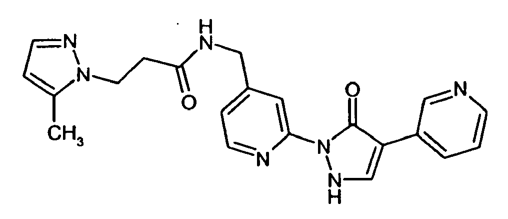 | m/z = 404; 1.11 min (8) | 13 |
| **58** | 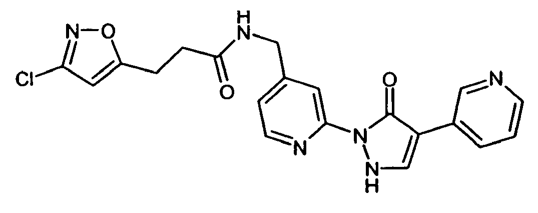 | m/z = 425; 1.21 min (8) | 15 |
| **59** | 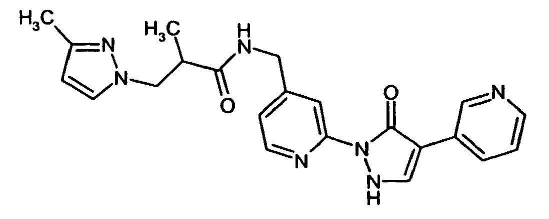 | m/z = 418; 1.16 min (8) | 19 |
| **60** | 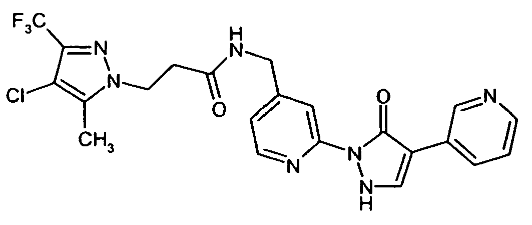 | m/z = 506; 1.48 min (8) | 9 |
| **61** | 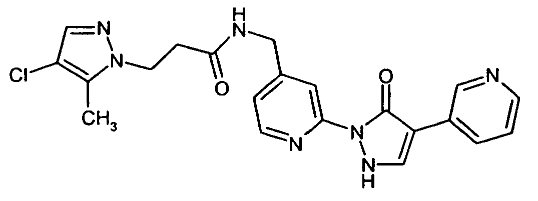 | m/z = 438; 1.27 min (8) | 14 |

| | | | |
|---|---|---|---|
| [zu Beispiel 55: Die Synthese des Ausgangsmaterials 3-(4-Hydroxy-3,5-dimethylphenyl)propionsäure ist beschrieben in J. Med. Chem. 1995, 38, 695-707]. | | | |

### B. Bewertung der pharmakologischen Wirksamkeit

Die pharmakologischen Eigenschaften der erfindungsgemäßen Verbindungen können in folgenden Assays gezeigt werden:

### Abkürzungen:

| | |
|---|---|
| DMEM | Dulbecco's Modified Eagle Medium |
| FCS | Fetal Calf Serum |
| TMB | 3,3',5,5'-Tetramethylbenzidin |
| Tris | Tris(hydroxymethyl)-aminomethan |

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgeführt [Oehme F., Jonghaus W., Narouz-Ott L., Huetter J., Flamme I., Anal. Biochem. 330 (1), 74-80 (2004)]:
Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYlPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCI, 1.5 mM MgCl₂, 1 µM-1 mM 2-Oxoglutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen. Die Testsubstanz wird bevorzugt, aber nicht ausschließlich bei Konzentrationen zwischen 1 nM und 100 µM eingesetzt. Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polyklonalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeitaufgelöste Fluoreszenz bestimmt. Außerdem ist die Verwendung von mit [³⁵S]-Methionin markiertem VBC-Komplex möglich. Hierfür kann der radioaktiv markierte VBC-Komplex durch *in vitro*-Transkription-Translation in Retikulozytenlysat hergestellt werden.

Die erfindungsgemäßen Verbindungen hemmen die Aktivität der HIF-Prolylhydroxylase in diesem Test mit einem IC₅₀-Wert von ≤ 10 µM. Repräsentative Ergebnisse sind in Tabelle 10 aufgeführt:

**Tabelle 10**

| **Beispiel Nr.** | **IC₅₀ [µM]** |
|---|---|
| 6 | 0.43 |
| 23 | 0.86 |
| 26 | 0.76 |
| 34 | 0.18 |
| 35 | 2.3 |
| 43 | 1.5 |
| 46 | 0.70 |
| 47 | 2.2 |
| 48 | 1.9 |
| 50 | 1.9 |

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-Zelllinie ab (A549, ATCC: American Type Culture Collection, Manassas, VA 20108, USA). Die Testzelllinie wird stabil mit einem Vektor transfiziert, der das Reportergen der *Photinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme I., Biochem. Biophys. Res. Commun. 296 (2), 343-9 (2002)]. Unter Einwirkung von Hypoxie (z.B. Kultivierung in Gegenwart von 1% Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder N-Oxalylglycindiethylester in einer Konzentration von 1 mM) produziert die Testzelllinie Luciferase, die mit Hilfe geeigneter Biolumineszenz-Reagenzien (z.B. Steady-Glo^{®} Luciferase Assay System, Promega Corporation, Madison, WI 53711, USA) und eines geeigneten Luminometers detektiert und quantifiziert werden kann.

Testablauf: Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (im folgenden als EC_{5O}-Wert bezeichnet) dient.

Die erfindungsgemäßen Verbindungen weisen in dem hier beschriebenen Test EC₅₀-Werte von ≤ 30 µM auf. Repräsentative Ergebnisse sind in Tabelle 11 aufgeführt:

**Tabelle 11**

| **Beispiel Nr.** | **EC₅₀ [µM]** |
|---|---|
| 6 | 4.9 |
| 23 | 13.4 |
| 26 | 6.0 |
| 34 | 4.7 |
| 35 | 17.2 |
| 43 | 7.6 |
| 46 | 7.4 |
| 47 | 12.4 |
| 48 | 18.9 |
| 50 | 7.1 |

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Lochplatten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol^{®}-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

Substanzen gemäß der vorliegenden Erfindung führen zu einem signifikanten, dosisabhängigen Anstieg der mRNA Hypoxie-induzierter Gene in Zellen menschlichen Ursprungs.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüfsubstanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Substanzen gemäß der vorliegenden Erfindung führen im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intraperitoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene 50 µl Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine^{®} mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Substanzen gemäß der vorliegenden Erfindung führen nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intraperitoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstriehen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Substanzen gemäß der vorliegenden Erfindung führen nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 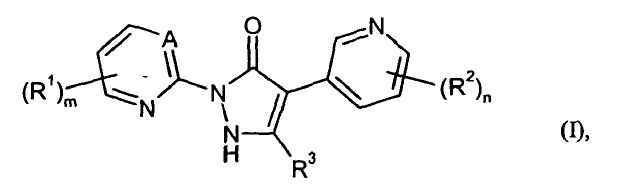 in welcher
A für CH oder N steht,
R¹ für einen Substituenten ausgewählt aus der Reihe (C₁-C₆)-Alkyl, Trifluormethyl, Halogen, Cyano, Nitro, Hydroxy, (C₁-C₆)-Alkoxy, Amino, (C₁-C₆)-Alkoxycarbonyl, Hydroxycarbonyl und -C(=O)-NH-R⁴ steht, worin
(C₁-C₆)-Alkyl seinerseits mit Hydroxy, (C₁-C₄)-Alkoxy, Amino, Mono-(C₁-C₄)-alkylamino, Di-(C₁-C₄)-alkylamino oder einer Gruppe der Formel -NH-C(=O)-R⁵, -NH-C(=O)-NH-R⁶ oder -NH-SO₂-R⁷ substituiert sein kann, worin
R₅ (C₁-C₆)-Alkyl, das mit Hydroxy, (C₁-C₄)-Alkoxy, Phenyl oder 5-oder 6-gliedrigem Heteroaryl substituiert sein kann, oder Phenyl bedeutet,
wobei Phenyl und Heteroaryl ihrerseits jeweils ein- bis dreifach, gleich oder verschieden, mit Halogen, Cyano, (C₁-C₄)-Alkyl, Hydroxy, (C₁-C₄)-Alkoxy, Trifluormethyl oder Trifluormethoxy substituiert sein können,
R₆ (C₁-C₆)-Alkyl, das mit Hydroxy oder (C₁-C₄)-Alkoxy substituiert sein kann, bedeutet
und
R⁷ (C₁-C₆)-Alkyl bedeutet,
und
R⁴ Wasserstoff oder (C₁-C₆)-Alkyl, das mit Hydroxy, (C₁-C₄)-Alkoxy oder Phenyl substituiert sein kann, bedeutet,
wobei Phenyl seinerseits mit Halogen, Cyano, (C₁-C₄)-Alkyl, (C₁-C₄)-Alkoxy, Trifluormethyl oder Trifluormethoxy substituiert sein kann,
R² für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Hydroxycarbonyl und -C(=O)-NH-R⁸ steht, worin
(C₁-C₆)-Alkyl und (C₁-C₆)-Alkoxy ihrerseits mit Hydroxy substituiert sein können
und
R⁸ Wasserstoff oder (C₁-C₄)-Alkyl bedeutet,
m für die Zahl 0, 1 oder 2 steht,
n für die Zahl 0, 1, 2 oder 3 steht,
wobei für den Fall, dass R¹ oder R² mehrfach auftreten, ihre Bedeutungen jeweils gleich oder verschieden sein können,
und
R³ für Wasserstoff, (C₁-C₆)-Alkyl oder (C₃-C₇)-Cycloalkyl steht,
sowie ihre Salze, Solvate und Solvate der Salze.

2. Verbindung der Formel (I) nach Anspruch 1, in welcher
A für CH oder N steht,
R¹ für einen Substituenten ausgewählt aus der Reihe (C₁-C₆)-Alkyl, Trifluormethyl, Cyano, Nitro, Hydroxy, (C₁-C₆)-Alkoxy, Amino, (C₁-C₆)-Alkoxycarbonyl und Hydroxycarbonyl steht,
R² für einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino und Hydroxycarbonyl steht, worin (C₁-C₆)-Alkyl und (C₁-C₆)-Alkoxy ihrerseits mit Hydroxy substituiert sein können,
m für die Zahl 0, 1 oder 2 steht,
n für die Zahl 0, 1, 2 oder 3 steht,
wobei für den Fall, dass R¹ oder R² mehrfach auftreten, ihre Bedeutungen jeweils gleich oder verschieden sein können,
und
R³ für Wasserstoff, (C₁-C₆)-Alkyl oder (C₃-C₇)-Cycloalkyl steht,
sowie ihre Salze, Solvate und Solvate der Salze.

3. Verbindung der Formel (I) nach Anspruch 1, in welcher
A für CH steht,
R¹ für einen Substituenten ausgewählt aus der Reihe (C₁-C₆)-Alkyl, Fluor, Chlor, Brom und -C(=O)-NH-R⁴ steht, worin
(C₁-C₆)-Alkyl seinerseits mit Hydroxy, (C₁-C₄)-Alkoxy, Amino, Mono-(C₁-C₄)-alkylamino, Di-(C₁-C₄)-alkylamino oder einer Gruppe der Formel -NH-C(=O)-R⁵, -NH-C(=O)-NH-R⁶ oder -NH-SO₂-R⁷ substituiert sein kann, worin
R⁵ (C₁-C₆)-Alkyl, das mit Hydroxy, Methoxy, Ethoxy, Phenyl oder 5-gliedrigem Heteroaryl substituiert sein kann, oder Phenyl bedeutet,
wobei Phenyl und Heteroaryl ihrerseits jeweils ein- bis dreifach, gleich oder verschieden, mit Fluor, Chlor, Brom, Cyano, Methyl, Hydroxy, Methoxy, Ethoxy, Trifluormethyl oder Trifluormethoxy substituiert sein können,
und
R⁶ und R⁷ unabhängig voneinander (C₁-C₆)-Alkyl bedeuten,
und
R⁴ (C₁-C₆)-Alkyl, das mit Hydroxy, Methoxy, Ethoxy oder Phenyl substituiert sein kann, bedeutet;
wobei Phenyl seinerseits mit Fluor, Chlor, Brom, Cyano, Methyl, Methoxy, Ethoxy, Trifluormethyl oder Trifluormethoxy substituiert sein kann,
R² für einen Substituenten ausgewählt aus der Reihe Fluor, Chlor, Brom, Cyano, (C₁-C₆)-Alkyl, Trifluormethyl, Hydroxycarbonyl und -C(=O)-NH-R⁸ steht, worin
(C₁-C₆)-Alkyl seinerseits mit Hydroxy substituiert sein kann
und
R⁸ (C₁-C₄)-Alkyl bedeutet,
m für die Zahl 0, 1 oder 2 steht,
n für die Zahl 0, 1 oder 2 steht,
wobei für den Fall, dass R¹ oder R² mehrfach auftreten, ihre Bedeutungen jeweils gleich oder verschieden sein können,
und
R³ für Wasserstoff steht,
sowie ihre Salze, Solvate und Solvate der Salze.

4. Verbindung der Formel (I) nach Anspruch 1 oder 2, in welcher
A für CH steht,
R¹ für einen Substituenten ausgewählt aus der Reihe (C₁-C₄)-Alkyl, Trifluormethyl, Nitro, (C₁-C₄)-Alkoxy, Amino und (C₁-C₄)-Alkoxycarbonyl steht,
R² für einen Substituenten ausgewählt aus der Reihe Chlor, Brom, Cyano, (C₁-C₄)-Alkyl, Trifluormethyl, Hydroxy, (C₁-C₄)-Alkoxy, Trifluormethoxy und Amino steht, worin (C₁-C₄)-Alkyl und (C₁-C₄)-Alkoxy ihrerseits mit Hydroxy substituiert sein können,
m für die Zahl 0 oder 1 steht,
n für die Zahl 0, 1, 2 oder 3 steht,
wobei für den Fall, dass R² mehrfach auftritt, seine Bedeutungen gleich oder verschieden sein können,
und
R³ für Wasserstoff oder Methyl steht,
sowie ihre Salze, Solvate und Solvate der Salze.

5. Verbindung der Formel (I) nach Anspruch 1 oder 3, in welcher
A für CH steht,
R¹ für einen Substituenten ausgewählt aus der Reihe (C₁-C₄)-Alkyl, Fluor, Chlor, Brom und -C(=O)-NH-R⁴ steht, worin
(C₁-C₄)-Alkyl seinerseits mit Hydroxy, Amino oder einer Gruppe der Formel -NH-C(=O)-R⁵ oder -NH-C(=O)-NH-R⁶ substituiert sein kann, worin
R⁵ (C₁-C₄)-Alkyl, das mit Phenyl oder Pyrazolyl substituiert sein kann, oder Phenyl bedeutet,
wobei Phenyl und Pyrazolyl ihrerseits jeweils ein- bis dreifach, gleich oder verschieden, mit Fluor, Chlor, Methyl oder Trifluormethyl substituiert sein können,
und
R⁶ (C₁-C₄)-Alkyl bedeutet,
und
R⁴ (C₁-C₄)-Alkyl, das mit Phenyl substituiert sein kann, bedeutet,
R² für einen Substituenten ausgewählt aus der Reihe Chlor, Brom, Cyano, (C₁-C₄)-Alkyl und Trifluormethyl steht, worin (C₁-C₄)-Alkyl seinerseits mit Hydroxy substituiert sein kann,
m für die Zahl 0, 1 oder 2 steht,
n für die Zahl 0, 1 oder 2 steht,
wobei für den Fall, dass R¹ oder R² mehrfach auftreten, ihre Bedeutungen jeweils gleich oder verschieden sein können,
und
R³ für Wasserstoff steht,
sowie ihre Salze, Solvate und Solvate der Salze.

6. Verbindung der Formel (I-A) nach Anspruch 1 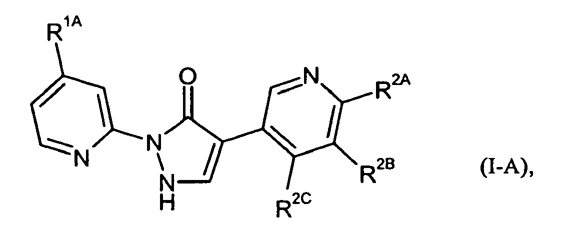 in welcher
R^{1A} für Wasserstoff, Methyl oder Trifluormethyl steht
und
R^{2A}, R^{2B} und R^{2C} gleich oder verschieden sind und unabhängig voneinander für Wasserstoff, Chlor, Brom, Cyano, Methyl, Hydroxymethyl, Methoxy oder Ethoxy stehen,
sowie ihre Salze, Solvate und Solvate der Salze.

7. Verbindung der Formel (I-B) nach Anspruch 1 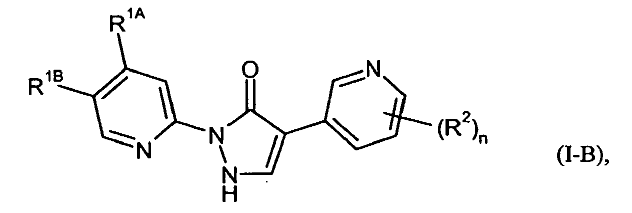 in welcher
R^{1A} und R^{1B} gleich oder verschieden sind und unabhängig voneinander für Wasserstoff Fluor, Chlor, (C₁-C₄)-Alkyl oder -C(=O)-NH-R⁴ stehen, worin
(C₁-C₄)-Alkyl seinerseits mit Hydroxy, Amino oder einer Gruppe der Formel -NH-C(=O)-R⁵ substituiert sein kann, worin
R⁵ (C₁-C₄)-Alkyl, das mit Phenyl oder Pyrazolyl substituiert sein kann, oder Phenyl bedeutet,
wobei Phenyl und Pyrazolyl ihrerseits jeweils ein- bis dreifach, gleich oder verschieden, mit Fluor, Chlor, Methyl oder Trifluormethyl substituiert sein können,
und
R⁴ (C₁-C₄)-Alkyl, das mit Phenyl substituiert sein kann, bedeutet,
R² für einen Substituenten ausgewählt aus der Reihe Chlor, Brom, Cyano, Methyl, Hydroxymethyl oder Trifluormethyl steht
und
n für die Zahl 0, 1 oder 2 steht,
wobei für den Fall, dass R² mehrfach auftritt, seine Bedeutungen gleich oder verschieden sein können,
sowie ihre Salze, Solvate und Solvate der Salze.

8. Verfahren zur Herstellung einer Verbindung der Formel (I), (I-A) bzw. (I-B), wie in den Ansprüchen 1 bis 7 definiert, **dadurch gekennzeichnet, dass** man Verbindungen der Formel (II) 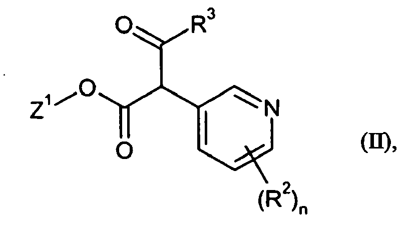 in welcher R², R³ und n jeweils die in den Ansprüchen 1 bis 7 angegebenen Bedeutungen haben und
Z¹ für Methyl oder Ethyl steht,
in einem inerten Lösungsmittel gegebenenfalls in Gegenwart einer Säure mit einer Verbindung der Formel (III) 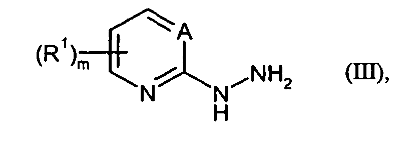 in welcher A, R¹ und m jeweils die in den Ansprüchen 1 bis 7 angegebenen Bedeutungen haben,
zu Verbindungen der Formel (IV) 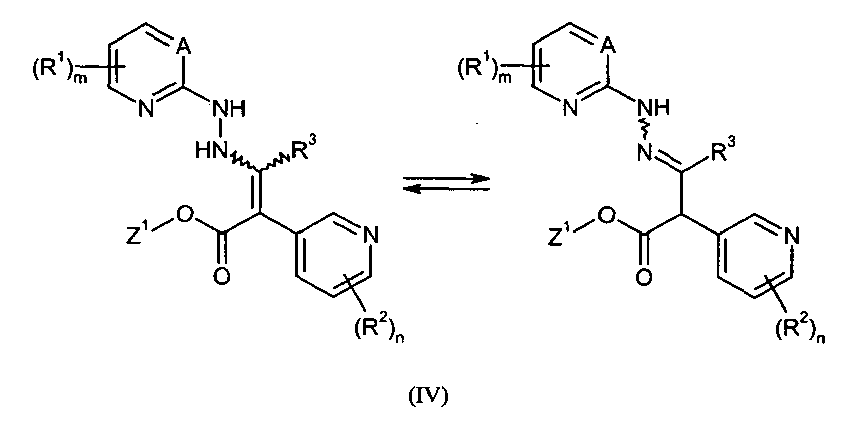 in welcher Z¹, A, R¹, R², R³, m und n die oben angegebenen Bedeutungen haben,
umsetzt und diese dann in einem inerten Lösungsmittel in Gegenwart einer Base cyclisiert
und die Verbindungen der Formel (I), (I-A) bzw. (I-B) gegebenenfalls mit den entsprechenden (i) Lösungsmitteln und/oder (ii) Basen oder Säuren in ihre Solvate, Salze und/oder Solvate der Salze überführt.

9. Verfahren zur Herstellung einer Verbindung der Formel (I), (I-A) bzw. (I-B), wie in den Ansprüchen 1 bis 7 definiert, in welcher R³ für Wasserstoff steht, **dadurch gekennzeichnet, dass** man zunächst Verbindungen der Formel (V) 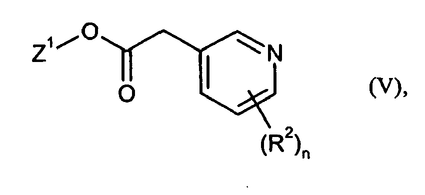 in welcher R² und n jeweils die in den Ansprüchen 1 bis 7 angegebenen Bedeutungen haben und
Z¹ für Methyl oder Ethyl steht,
mit einer Verbindung der Formel (VI) 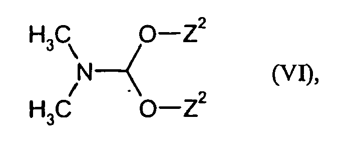 in welcher
Z² für Methyl oder Ethyl steht,
zu Verbindungen der Formel (VII) 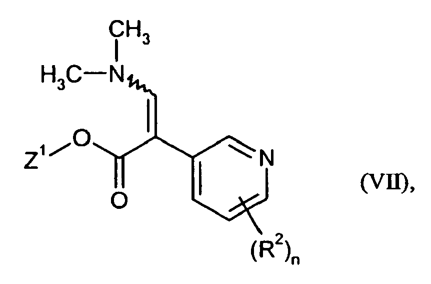
in welcher Z¹, R² und n die oben angegebenen Bedeutungen aufweisen,
kondensiert, anschließend in Gegenwart einer Säure mit einer Verbindung der Formel (III) 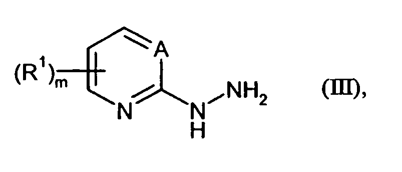 in welcher A, R¹ und m jeweils die in den Ansprüchen 1 bis 7 angegebenen Bedeutungen haben,
zu Verbindungen der Formel (IV-A) 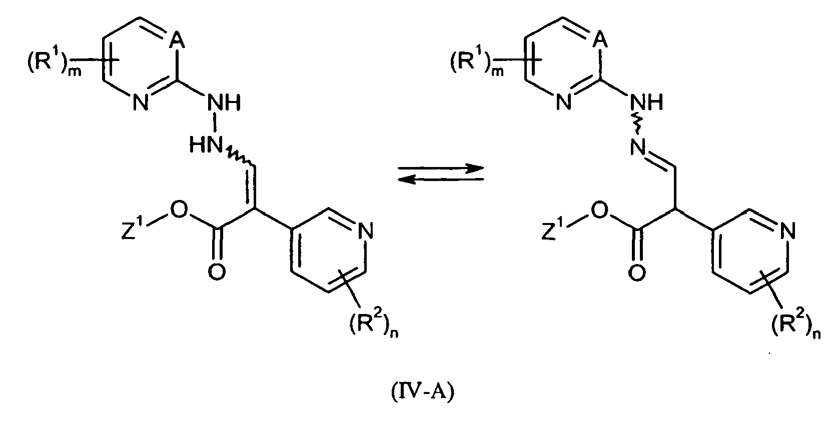 in welcher Z¹, A, R¹, R², m und n die oben angegebenen Bedeutungen aufweisen,
umsetzt und diese dann in einem inerten Lösungsmittel in Gegenwart einer Base cyclisiert.

10. Verbindung, wie in einem der Ansprüchen 1 bis 7 definiert, zur Behandlung und/oder Prophylaxe von Krankheiten.

11. Verwendung einer Verbindung, wie in einem der Ansprüche 1 bis 7 definiert, zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

12. Arzneimittel enthaltend eine Verbindung, wie in einem der Ansprüche 1 bis 7 definiert, in Kombination mit einem inerten, nicht-toxischen, pharmazeutisch geeigneten Hilfsstoff.

13. Arzneimittel enthaltend eine Verbindung, wie in einem der Ansprüche 1 bis 7 definiert, in Kombination mit einem weiteren Wirkstoff ausgewählt aus der Gruppe bestehend aus ACE-Inhibitoren, Angiotensin II-Rezeptor-Antagonisten, Beta-Rezeptor-Blocker, Mineralocorticoid-Rezeptor-Antagonisten, Aspirin, Diuretika, Eisen-Supplements, Vitamin B12-und Folsäure-Supplements, Calcium-Antagonisten, Statine und Digitalis (Digoxin)-Derivate.

14. Arzneimittel nach Anspruch 12 oder 13 zur Behandlung und/oder Prophylaxe von Herz-Kreislauf-Erkrankungen, Herzinsuffizienz, Anämie, chronischen Nierenerkrankungen und Niereninsuffizienz.

15. Verfahren zur Herstellung eines Arzneimittels zur Behandlung und/oder Prophylaxe von Herz-Kreislauf Erkrankungen, Herzinsuffizienz, Anämie, chronischen Nierenerkrankungen und Niereninsuffizienz in Menschen und Tieren unter Verwendung einer wirksamen Menge mindestens einer Verbindung, wie in einem der Ansprüche 1 bis 7 definiert, oder eines Arzneimittels, wie in einem der Ansprüche 12 bis 14 definiert.

## Claims

1. Compound of the formula (I) 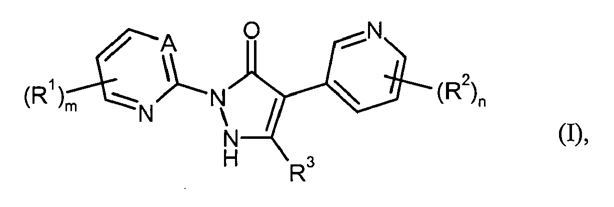 in which
A represents CH or N,
R¹ represents a substituent chosen from the series consisting of (C₁-C₆)-alkyl, trifluoromethyl, halogen, cyano, nitro, hydroxyl, (C₁-C₆)-alkoxy, amino, (C₁-C₆)-alkoxycarbonyl, hydroxycarbonyl and-C(=O)-NH-R⁴, wherein
(C₁-C₆)-alkyl in its turn can be substituted by hydroxyl, (C₁-C₄)-alkoxy, amino, mono-(C₁-C₄)-alkylamino, di-(C₁-C₄)-alkylamino or a group of the formula -NH-C(=O)-R⁵, -NH-C(=O)-NH-R⁶ or -NH-SO₂-R⁷ , wherein
R⁵ denotes (C₁-C₆)-alkyl, which can be substituted by hydroxyl, (C₁-C₄)-alkoxy, phenyl or 5- or 6-membered heteroaryl, or phenyl,
wherein phenyl and heteroaryl in their turn can in each case be substituted once to three times in an identical or different manner by halogen, cyano, (C₁-C₄)-alkyl, hydroxyl, (C₁-C₄)-alkoxy, trifluoromethyl or trifluoromethoxy,
R⁶ denotes (C₁-C₆)-alkyl, which can be substituted by hydroxyl or (C₁-C₄)-alkoxy,
and
R⁷ denotes (C₁-C₆)-alkyl,
and
R⁴ denotes hydrogen or (C₁-C₆)-alkyl, which can be substituted by hydroxyl, (C₁-C₄)-alkoxy or phenyl,
wherein phenyl in its turn can be substituted by halogen, cyano, (C₁-C₄)-alkyl, (C₁-C₄)-alkoxy, trifluoromethyl or trifluoromethoxy,
R² represents a substituent chosen from the series consisting of halogen, cyano, nitro, (C₁-C₆)-alkyl, trifluoromethyl, hydroxyl, (C₁-C₆)-alkoxy, trifluoromethoxy, amino, hydroxycarbonyl and -C(=O)-NH-R⁸, wherein
(C₁-C₆)-alkyl and (C₁-C₆)-alkoxy in their turn can be substituted by hydroxyl
and
R⁸ denotes hydrogen or (C₁-C₄)-alkyl,
m represents the number 0, 1 or 2,
n represents the number 0, 1, 2 or 3,
wherein, in the case where R¹ or R² occur more than once, their meanings can in each case be identical or different,
and
R³ represents hydrogen, (C₁-C₆)-alkyl or (C₃-C₇)-cycloalkyl,
and its salts, solvates and solvates of the salts.

2. Compound of the formula (I) according to claim 1, in which
A represents CH or N,
R¹ represents a substituent chosen from the series consisting of (C₁-C₆)-alkyl, trifluoromethyl, cyano, nitro, hydroxyl, (C₁-C₆)-alkoxy, amino, (C₁-C₆)-alkoxycarbonyl and hydroxycarbonyl,
R² represents a substituent chosen from the series consisting of halogen, cyano, nitro, (C₁-C₆)-alkyl, trifluoromethyl, hydroxyl, (C₁-C₆)-alkoxy, trifluoromethoxy, amino and hydroxycarbonyl, wherein (C₁-C₆)-alkyl and (C₁-C₆)-alkoxy in their turn can be substituted by hydroxyl,
m represents the number 0, 1 or 2,
n represents the number 0, 1, 2 or 3,
wherein, in the case where R¹ or R² occur more than once, their meanings can in each case be identical or different,
and
R³ represents hydrogen, (C₁-C₆)-alkyl or (C₃-C₇)-cycloalkyl,
and its salts, solvates and solvates of the salts.

3. Compound of the formula (I) according to claim 1, in which
A represents CH,
R¹ represents a substituent chosen from the series consisting of (C₁-C₆)-alkyl, fluorine, chlorine, bromine and -C(=O)-NH-R⁴, wherein
(C₁-C₆)-alkyl in its turn can be substituted by hydroxyl, (C₁-C₄)-alkoxy, amino, mono-(C₁-C₄)-alkylamino, di-(C₁-C₄)-alkylamino or a group of the formula -NH-C(=O)-R⁵, -NH-C(=O)-NH-R⁶ or -NH-SO₂-R⁷, wherein
R⁵ denotes (C₁-C₆)-alkyl, which can be substituted by hydroxyl, methoxy, ethoxy, phenyl or 5-membered heteroaryl, or phenyl,
wherein phenyl and heteroaryl in their turn can in each case be substituted once to three times in an identical or different manner by fluorine, chlorine, bromine, cyano, methyl, hydroxyl, methoxy, ethoxy, trifluoromethyl or trifluoromethoxy,
and
R⁶ and R⁷ independently of one another denote (C₁-C₆)-alkyl,
and
R⁴ denotes (C₁-C₆)-alkyl, which can be substituted by hydroxyl, methoxy, ethoxy or phenyl,
wherein phenyl in its turn can be substituted by fluorine, chlorine, bromine, cyano, methyl, methoxy, ethoxy, trifluoromethyl or trifluoromethoxy,
R² represents a substituent chosen from the series consisting of fluorine, chlorine, bromine, cyano, (C₁-C₆)-alkyl, trifluoromethyl, hydroxycarbonyl and -C(=O)-NH-R⁸, wherein
(C₁-C₆)-alkyl in its turn can be substituted by hydroxyl
and
R⁸ denotes (C₁-C₄)-alkyl,
m represents the number 0, 1 or 2,
n represents the number 0, 1 or 2,
wherein, in the case where R¹ or R² occur more than once, their meanings can in each case be identical or different,
and
R³ represents hydrogen,
and its salts, solvates and solvates of the salts.

4. Compound of the formula (I) according to claim 1 or 2, in which
A represents CH,
R¹ represents a substituent chosen from the series consisting of (C₁-C₄)-alkyl, trifluoromethyl, nitro, (C₁-C₄)-alkoxy, amino and (C₁-C₄)-alkoxycarbonyl,
R² represents a substituent chosen from the series consisting of chorine, bromine, cyano, (C₁-C₄)-alkyl, trifluoromethyl, hydroxyl, (C₁-C₄)-alkoxy, trifluoromethoxy and amino, wherein (C₁-C₄)-alkyl and (C₁-C₄)-alkoxy in their turn can be substituted by hydroxyl,
m represents the number 0 or 1,
n represents the number 0, 1, 2 or 3,
wherein, in the case where R² occurs more than once, its meanings can be identical or different,
and
R³ represents hydrogen or methyl,
and its salts, solvates and solvates of the salts.

5. Compound of the formula (I) according to claim 1 or 3, in which
A represents CH,
R¹ represents a substituent chosen from the series consisting of (C₁-C₄)-alkyl, fluorine, chlorine, bromine and -C(=O)-NH-R⁴, wherein
(C₁-C₄)-alkyl in its turn can be substituted by hydroxyl, amino or a group of the formula -NH-C(=O)-R⁵ or -NH-C(=O)-NH-R⁶, wherein
R⁵ denotes (C₁-C₄)-alkyl, which can be substituted by phenyl or pyrazolyl, or phenyl,
wherein phenyl and pyrazolyl in their turn can in each case be substituted once to three times in an identical or different manner by fluorine, chlorine, methyl or trifluoromethyl,
and
R⁶ denotes (C₁-C₄)-alkyl,
and
R⁴ denotes (C₁-C₄)-alkyl, which can be substituted by phenyl,
R² represents a substituent chosen from the series consisting of chlorine, bromine, cyano, (C₁-C₄)-alkyl and trifluoromethyl,
wherein (C₁-C₄)-alkyl in its turn can be substituted by hydroxyl,
m represents the number 0, 1 or 2,
n represents the number 0, 1 or 2,
wherein in the case where R¹ or R² occur more than once, their meanings can in each case be identical or different,
and
R³ represents hydrogen,
and its salts, solvates and solvates of the salts.

6. Compound of the formula (I-A) according to claim 1 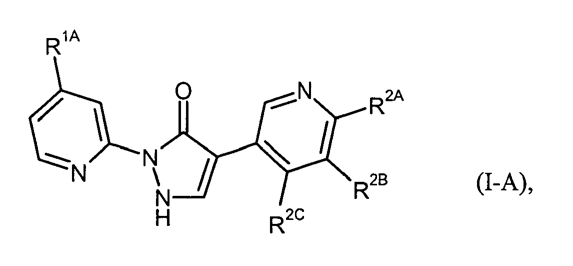 in which
R^{1A} represents hydrogen, methyl or trifluoromethyl
and
R^{2A}, R^{2B} and R^{2C} are identical or different and independently of one another represent hydrogen, chlorine, bromine, cyano, methyl, hydroxymethyl, methoxy or ethoxy,
and its salts, solvates and solvates of the salts.

7. Compound of the formula (I-B) according to claim 1 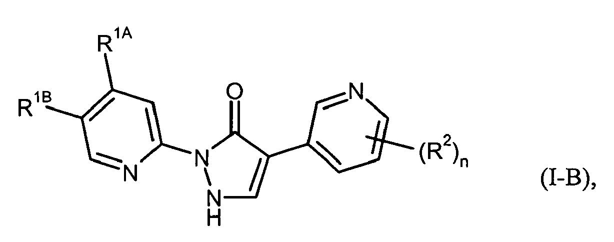 in which
R^{1A} and R^{1B} are identical or different and independently of one another represent hydrogen, fluorine, chlorine, (C₁-C₄)-alkyl or -C(=O)-NH-R⁴, wherein
(C₁-C₄)-alkyl in its turn can be substituted by hydroxyl, amino or a group of the formula -NH-C(=O)-R⁵, wherein
R⁵ denotes (C₁-C₄)-alkyl, which can be substituted by phenyl or pyrazolyl, or phenyl,
wherein phenyl and pyrazolyl in their turn can in each case be substituted once to three times in an identical or different manner by fluorine, chlorine, methyl or trifluoromethyl,
and
R⁴ denotes (C₁-C₄)-alkyl, which can be substituted by phenyl,
R² represents a substituent chosen from the series consisting of chlorine, bromine, cyano, methyl, hydroxymethyl or trifluoromethyl
and
n represents the number 0, 1 or 2,
wherein, in the case where R² occurs more than once, its meanings can be identical or different,
and its salts, solvates and solvates of the salts.

8. Process for the preparation of a compound of the formula (I), (I-A) or (I-B), as defined in claims 1 to 7, **characterized in that** compounds of the formula (II) 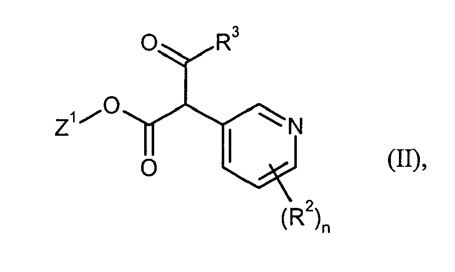 in which R², R³ and n in each case have the meanings given in claims 1 to 7 and
Z¹ represents methyl or ethyl,
are reacted in an inert solvent, optionally in the presence of an acid, with a compound of the formula (III) 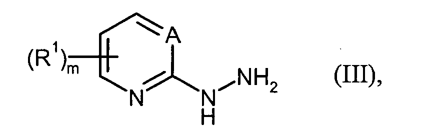 in which A, R¹ and m in each case have the meanings given in claims 1 to 7,
to give compounds of the formula (IV) 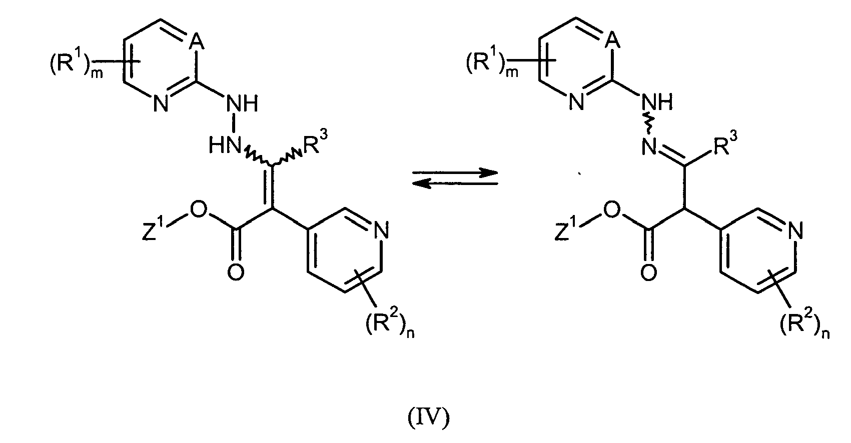 in which Z¹, A, R¹, R², R³, m and n have the abovementioned meanings,
and these are then cyclized in an inert solvent in the presence of a base
and the compounds of the formula (I), (I-A) or (I-B) are optionally converted with the corresponding (i) solvents and/or (ii) bases or acids into their solvates, salts and/or solvates of the salts.

9. Process for the preparation of a compound of the formula (I), (I-A) or (I-B), as defined in claims 1 to 7, in which R³ represents hydrogen, **characterized in that** compounds of the formula (V) 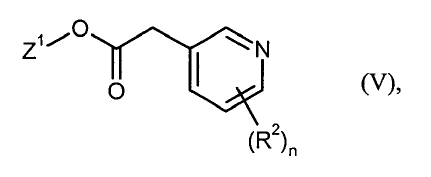 in which R² and n in each case have the meanings given in claims 1 to 7 and
Z¹ represents methyl or ethyl,
are subjected to a condensation reaction with a compound of the formula (VI) 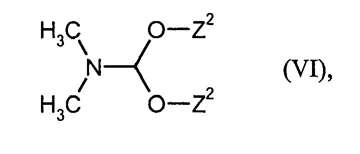 in which
Z² represents methyl or ethyl,
to give compounds of the formula (VII) 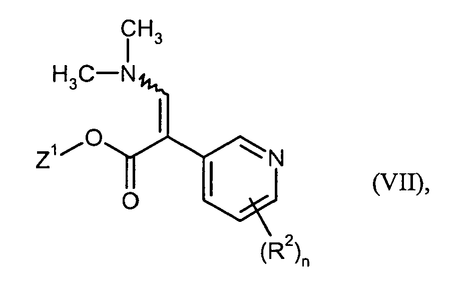 in which Z¹, R² and n have the abovementioned meanings,
which are subsequently reacted in the presence of an acid with a compound of the formula (III) 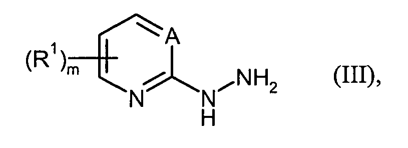 in which A, R¹ and m in each case have the meanings given in claims 1 to 7,
to give compounds of the formula (IV-A) 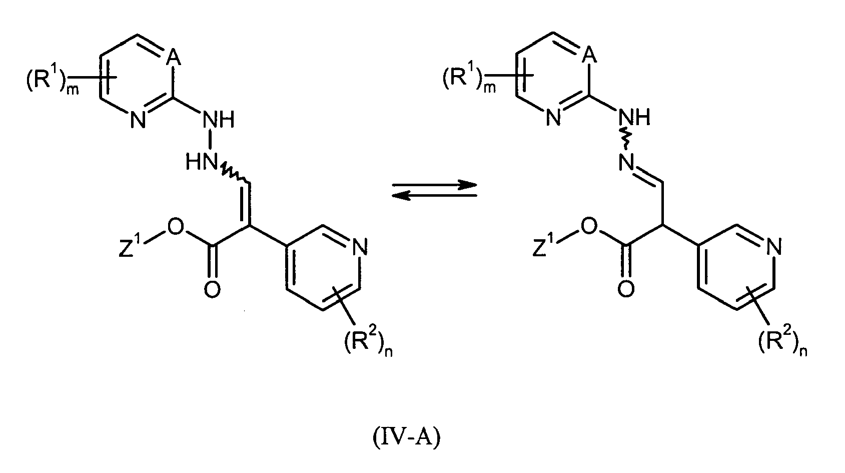 in which Z¹, A, R¹, R², m and n have the abovementioned meanings,
and these are then cyclized in an inert solvent in the presence of a base.

10. Compound as defined in one of claims 1 to 7, for treatment and/or prophylaxis of diseases.

11. Use of a compound as defined in one of claims 1 to 7 in the manufacture of a medicament for treatment and/or prophylaxis of cardiovascular diseases, cardiac insufficiency, anemia, chronic kidney diseases and renal insufficiency.

12. Medicament comprising a compound as defined in one of claims 1 to 7 in combination with an inert, non-toxic, pharmaceutically suitable auxiliary substance.

13. Medicament comprising a compound as defined in one of claims 1 to 7 in combination with a further active compound chosen from the group consisting of ACE inhibitors, angiotensin II receptor antagonists, beta receptor blockers, mineralocorticoid receptor antagonists, aspirin, diuretics, iron supplements, vitamin B12 and folic acid supplements, calcium antagonists, statins and digitalis (digoxin) derivatives.

14. Medicament according to claim 12 or 13 for treatment and/or prophylaxis of cardiovascular diseases, cardiac insufficiency, anemia, chronic kidney diseases and renal insufficiency.

15. Method for the manufacture of a medicament for treatment and/or prophylaxis of cardiovascular diseases, cardiac insufficiency, anemia, chronic kidney diseases and renal insufficiency in humans and animals using an active amount of at least one compound as defined in one of claims 1 to 7 or of a medicament as defined in one of claims 12 to 14.

## Revendications

1. Composé de formule (I) 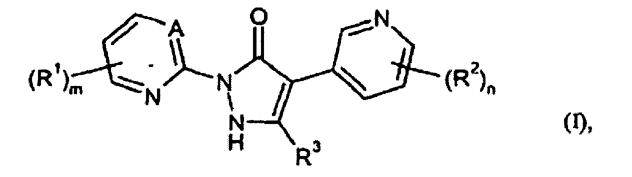 dans laquelle
A représente CH ou N,
R¹ représente un substituant choisi dans la série alkyle en C₁ à C₆, trifluorométhyle, halogéno, cyano, nitro, hydroxy, alkoxy en C₁ à C₆, amino, (alkoxy en C₁ à C₆)-carbonyle, hydroxycarbonyle ou -C(=O)-NH-R⁴, où alkyle en C₁ à C₆ peut lui-même être substitué avec un radical hydroxy, alkoxy en C₁ à C₄, amino, mono (alkyle en C₁ à C₄) amino, di(alkyle en C₁ à C₄) amino ou avec un groupe de formule -NH-C(=O)-R⁵, -NH-C(=O)-NH-R⁶ ou -NH-SO₂-R⁷, formule dans laquelle
R⁵ représente un reste alkyle en C₁ à C₆, qui peut être substitué avec un radical hydroxy, alkoxy en C₁ à C₄, phényle ou hétéroaryle penta- ou hexagonal, ou un reste phényle,
les radicaux phényle et hétéroaryle pouvant eux-mêmes être substitués chacun une à trois fois identiques ou différentes avec un radical halogéno, cyano, alkyle en C₁ à C₄ , hydroxy, alkoxy en C₁ à C₄, trifluorométhyle ou trifluorométhoxy,
R⁶ représente un reste alkyle en C₁ à C₆, qui peut être substitué avec un radical hydroxy ou alkoxy en C₁ à C₄,
et
R⁷ représente un reste alkyle en C₁ à C₆,
et
R⁴ représente l'hydrogène ou un reste alkyle en C₁ à C₆, qui peut être substitué avec un radical hydroxy, alkoxy en C₁ à C₄ ou phényle,
le reste phényle pouvant lui-même être substitué avec un radical halogéno, cyano, alkyle en C₁ à C₄, alkoxy en C₁ à C₄, trifluorométhyle ou trifluorométhoxy,
R² représente un substituant choisi dans la série halogéno, cyano, nitro, alkyle en C₁ à C₆, trifluorométhyle, hydroxy, alkoxy en C₁ à C₆, trifluorométhoxy, amino, hydroxycarbonyle et -C(=O)-NH-R⁸, où
alkyle en C₁ à C₆ et alkoxy en C₁ à C₆ peuvent eux-mêmes être substitués avec un radical hydroxy et
R⁸ représente l'hydrogène ou un reste alkyle en C₁ à C₄,
m représente le nombre 0, 1 ou 2,
n représente le nombre 0, 1, 2 ou 3,
au cas où R¹ ou R² apparaissent plusieurs fois, leurs définitions peuvent dans chaque cas être identiques ou différentes,
et
R³ représente l'hydrogène, un reste alkyle en C₁ à C₆ ou cycloalkyle en C₃ à C₇,
ainsi que ses sels, ses produits de solvatation et les produits de solvatation des sels.

2. Composé de formule (I) suivant la revendication 1, formule dans laquelle
A représente CH ou N,
R¹ représente un substituant choisi dans la série alkyle en C₁ à C₆, trifluorométhyle, cyano, nitro, hydroxy, alkoxy en C₁ à C₆, amino, (alkoxy en C₁ à C₆) carbonyle et hydroxycarbonyle,
R² représente un substituant choisi dans la série halogéno, cyano, nitro, alkyle en C₁ à C₆, trifluorométhyle, hydroxy, alkoxy en C₁ à C₆, trifluorométhoxy, amino et hydroxycarbonyle, où les restes alkyle en C₁ à C₆ et alkoxy en C₁ à C₆ peuvent eux-mêmes être substitués avec un radical hydroxy,
m représente le nombre 0, 1 ou 2,
n représente le nombre 0, 1, 2 ou 3, au cas où R¹ ou R² apparaissent plusieurs fois, leurs définitions peuvent dans chaque cas être identiques ou différentes,
et
R³ représente l'hydrogène, un reste alkyle en C₁ à C₆ ou cycloalkyle en C₃ à C₇,
ainsi que ses sels, ses produits de solvatation et les produits de solvatation des sels.

3. Composé de formule (I) suivant la revendication 1, formule dans laquelle
A représente CH,
R¹ représente un substituant choisi dans la série alkyle en C₁ à C₆, fluoro, chloro, bromo et -C(=O)-NH-R⁴, où alkyle en C₁ à C₆ peut lui-même être substitué avec un radical hydroxy, alkoxy en C₁ à C₄, amino, mono(alkyle en C₁ à C₄) amino, di(alkyle en C₁ à C₄)amino ou avec un groupe de formule -NH-C(=O)-R⁵, -NH-C(=O)-NH-R⁶ ou -NH-SO₂-R⁷, où
R⁵ représente un reste alkyle en C₁ à C₆, qui peut être substitué avec un radical hydroxy, méthoxy, éthoxy, phényle ou hétéroaryle pentagonal, ou un reste phényle,
les radicaux phényle et hétéroaryle pouvant eux-mêmes être substitués chacun une à trois fois identiques ou différentes avec un radical fluoro, chloro, bromo, cyano, méthyle, hydroxy, méthoxy, éthoxy, trifluorométhyle ou trifluorométhoxy,
et
R⁶ et R⁷ représentent indépendamment l'un de l'autre un reste alkyle en C₁ à C₆,
et
R⁴ représente un reste alkyle en C₁ à C₆, qui peut être substitué avec un radical hydroxy, méthoxy, éthoxy ou phényle,
le radical phényle pouvant lui-même être substitué avec un radical fluoro, chloro, bromo, cyano, méthyle, méthoxy, éthoxy, trifluorométhyle ou trifluorométhoxy,
R² représente un substituant choisi dans la série fluoro, chloro, bromo, cyano, alkyle en C₁ à C₆, trifluorométhyle, hydroxycarbonyle et -C(=O)-NH-R⁸, où
alkyle en C₁ à C₆ peut lui-même être substitué avec un radical hydroxy
et
R⁸ représente un reste alkyle en C₁ à C₄,
m représente le nombre 0, 1 ou 2,
n représente le nombre 0, 1 ou 2,
au cas où R¹ ou R² apparaissent plusieurs fois, leurs définitions peuvent dans chaque cas être identiques ou différentes,
et
R³ représente l'hydrogène,
ainsi que ses sels, ses produits de solvatation et les produits de solvatation des sels.

4. Composé de formule (I) suivant la revendication 1 ou 2, formule dans laquelle
A représente CH,
R¹ représente un substituant choisi dans la série alkyle en C₁ à C₄, trifluorométhyle, nitro, alkoxy en C₁ à C₄, amino et (alkoxy en C₁ à C₄)carbonyle,
R² représente un substituant choisi dans la série chloro, bromo, cyano, alkyle en C₁ à C₄, trifluorométhyle, hydroxy, alkoxy en C₁ à C₄, trifluorométhoxy et amino, où alkyle en C₁ à C₄ et alkoxy en C₁ à C₄ peuvent eux-mêmes être substitués avec un radical hydroxy
m représente le nombre 0 ou 1,
n représente le nombre 0, 1, 2 ou 3,
au cas où R² apparaît plusieurs fois, ses définitions peuvent être identiques ou différentes,
et
R³ représente l'hydrogène ou un reste méthyle,
ainsi que ses sels, ses produits de solvatation et les produits de solvatation des sels.

5. Composé de formule (I) suivant la revendication 1 ou 3, formule dans laquelle
A représente CH,
R¹ représente un substituant choisi dans la série alkyle en C₁ à C₄, fluoro, chloro, bromo et -C(=O)-NH-R⁴, où alkyle en C₁ à C₄ peut lui-même être substitué avec un radical hydroxy, amino, ou avec un groupe de formule -NH-C (=O) -R⁵ ou -NH-C (=O) -NH-R⁶, où
R⁵ représente un reste alkyle en C₁ à C₄, qui peut être substitué avec un radical phényle ou pyrazolyle, ou un reste phényle,
les radicaux phényle et pyrazolyle pouvant eux-mêmes être substitués chacun une à trois fois identiques ou différentes avec un radical fluoro, chloro, méthyle ou trifluorométhyle, et
R⁶ représente un reste alkyle en C₁ à C₄,
et
R⁴ représente un reste alkyle en C₁ à C₄, qui peut être substitué avec un radical phényle,
R² représente un substituant choisi dans la série chloro, bromo, cyano, alkyle en C₁ à C₄ et trifluorométhyle, où le reste alkyle en C₁ à C₄ peut être substitué avec un radical hydroxy,
m représente le nombre 0, 1 ou 2,
n représente le nombre 0, 1 ou 2,
au cas où R¹ ou R² apparaissent plusieurs fois, leurs définitions peuvent dans chaque cas être identiques ou différentes,
et
R³ représente l'hydrogène,
ainsi que ses sels, ses produits de solvatation et les produits de solvatation des sels.

6. Composé de formule (I-A) suivant la revendication 1, 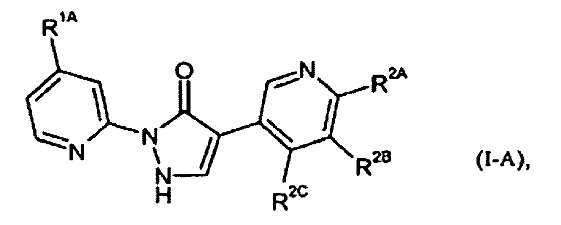 formule dans laquelle
R^{1A} représente l'hydrogène, un reste méthyle ou trifluorométhyle
et
R^{2A} R^{2B} et R^{2C} sont identiques ou différents et représentent, indépendamment les uns des autres, l'hydrogène, le chlore, le brome, un reste cyano, méthyle, hydroxyméthyle, méthoxy ou éthoxy,
ainsi que ses sels, ses produits de solvatation et les produits de solvatation des sels.

7. Composé de formule (I-B) suivant la revendication 1, 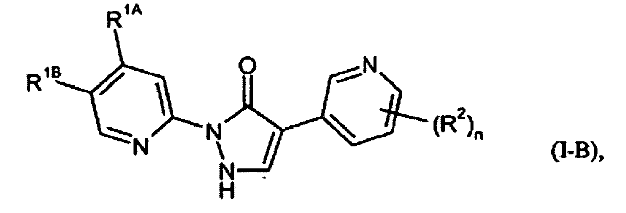 formule dans laquelle
R^{1A} et R^{1B} sont identiques ou différents et représentent, indépendamment l'un de l'autre, l'hydrogène, le fluor, le chlore, un reste alkyle en C₁ à C₄ ou -C(=O)-NH-R⁴, où
alkyle en C₁ à C₄ peut lui-même être substitué avec un radical hydroxy, amino ou un groupe de formule -NH-C(=O)-R⁵, où
R⁵ représente un reste alkyle en C₁ à C₄, qui peut être substitué avec un radical phényle ou pyrazolyle, ou un reste phényle,
les radicaux phényle et pyrazolyle pouvant eux-mêmes être substitués une à trois fois identiques ou différentes par un radical fluoro, chloro, méthyle ou trifluorométhyle,
et
R⁴ représente un reste alkyle en C₁ à C₄ qui peut être substitué avec un radical phényle,
R² représente un substituant choisi dans la série chlore, brome, cyano, méthyle, hydroxyméthyle ou trifluorométhyle,
et
n représente le nombre 0, 1 ou 2,
au cas où R² apparaît plusieurs fois, ses définitions peuvent être identiques ou différentes,
ainsi que ses sels, ses produits de solvatation et les produits de solvatation des sels.

8. Procédé de production d'un composé de formule (I), (I-A) ou (I-B), telle que définie dans les revendications 1 à 7, **caractérisé en ce que** des composés de formule (II) 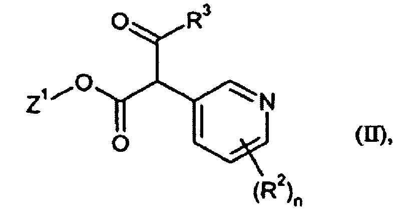 dans laquelle R², R³ et n ont chacun les définitions indiquées dans les revendications 1 à 7 et Z¹ représente un reste méthyle ou éthyle,
sont amenés à réagir, dans un solvant inerte, éventuellement en présence d'un acide, avec un composé de formule (III) 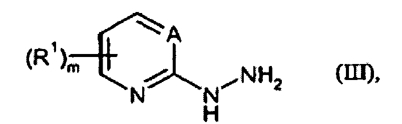 dans laquelle A, R¹ et m ont chacun les définitions indiquées dans les revendications 1 à 7, pour former des composés de formule (IV) 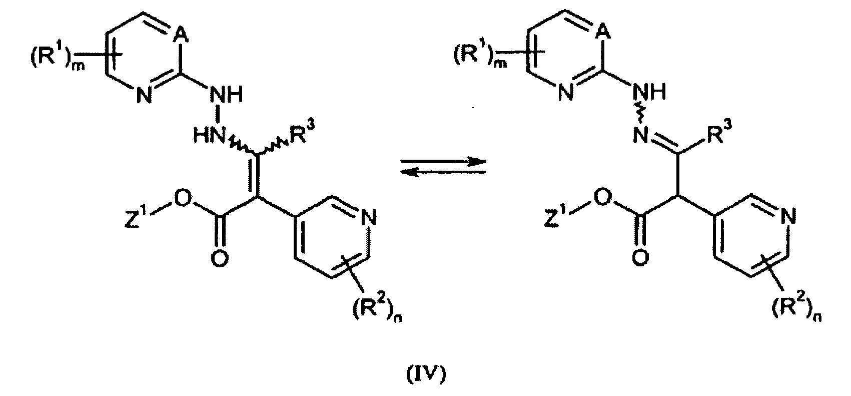 dans laquelle Z¹, A, R¹, R², R³, m et n ont les définitions indiquées ci-dessus,
puis ces composés sont cyclisés dans un solvant inerte en présence d'une base
et les composés de formule (I), (I-A) ou (I-B) sont éventuellement transformés, avec (i) les solvants correspondants et/ou (ii) les bases ou les acides correspondants, en leurs produits de solvatation, leurs sels et/ou les produits de solvatation des sels.

9. Procédé de production d'un composé de formule (I), (I-A) ou (I-B), telle que définie dans les revendications 1 à 7, dans laquelle R³ représente l'hydrogène, **caractérisé en ce qu'**on condense tout d'abord des composés de formule (V) 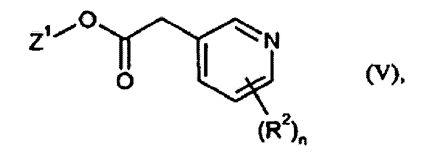 dans laquelle R² et n ont chacun les définitions indiquées dans les revendications 1 à 7 et Z¹ représente un reste méthyle ou éthyle, avec un composé de formule (VI) 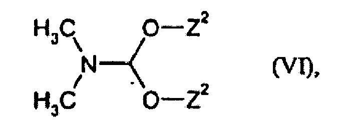 dans laquelle
Z² représente un reste méthyle ou éthyle, pour former des composés de formule (VII) 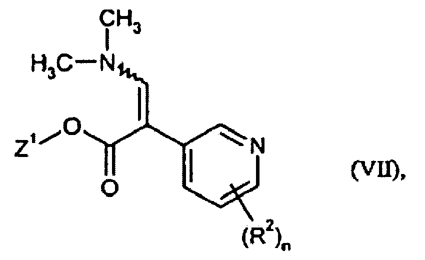
dans laquelle Z¹, R² et n ont les définitions indiquées ci-dessus,
qu'on fait ensuite réagir en présence d'un acide avec un composé de formule (III) 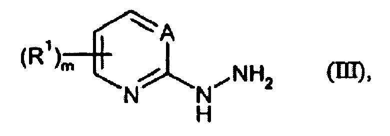 dans laquelle A, R¹ et m ont les définitions indiquées dans les revendications 1 à 7, pour obtenir des composés de formule (IV-A) 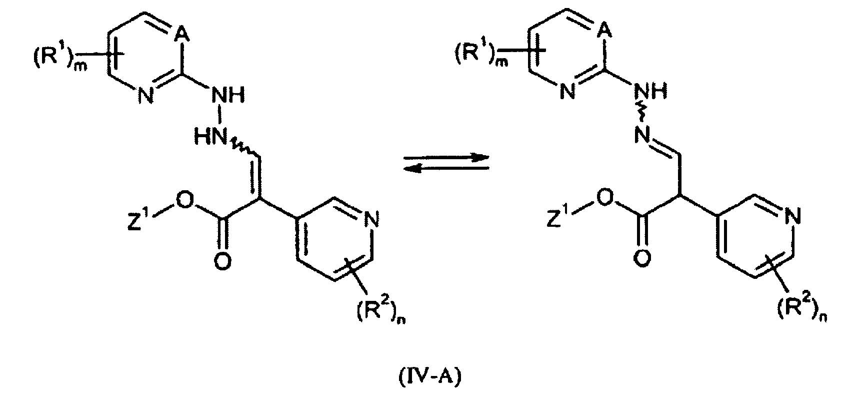 dans laquelle Z¹, A, R¹, R², m et n ont les définitions indiquées ci-dessus,
et on cyclise ensuite ces composés dans un solvant inerte en présence d'une base.

10. Composé tel que défini dans l'une des revendications 1 à 7, destiné au traitement et/ou à la prophylaxie de maladies.

11. Utilisation d'un composé tel que défini dans l'une des revendications 1 à 7 pour la préparation d'un médicament destiné au traitement et/ou à la prophylaxie de maladies cardio-vasculaires, d'insuffisance cardiaque, d'anémie, de maladies rénales chroniques et d'insuffisance rénale.

12. Médicament contenant un composé tel que défini dans l'une des revendications 1 à 7, en combinaison avec une substance auxiliaire inerte non toxique pharmaceutiquement acceptable.

13. Médicament contenant un composé tel que défini dans l'une des revendications 1 à 7, en combinaison avec une autre substance active choisie dans le groupe constitué d'inhibiteurs de ACE, d'antagonistes des récepteurs d'angiotensine II, d'inhibiteurs des bêtarécepteurs, d'antagonistes des récepteurs minéralocorticoïdes, de l'aspirine, des diurétiques, des suppléments en fer, des suppléments en vitamine B12 et en acide folique, des antagonistes du calcium, de la statine et des dérivés de digitale (digoxine).

14. Médicament suivant la revendication 12 ou 13, destiné au traitement et/ou à la prophylaxie de maladies cardio-vasculaires, de l'insuffisance cardiaque, de l'anémie, de maladies rénales chroniques et de l'insuffisance rénale.

15. Procédé de préparation d'un médicament destiné au traitement et/ou à la prophylaxie de maladies cardio-vasculaires, d'insuffisance cardiaque, d'anémie, de maladies rénales chroniques et d'insuffisance rénale en médecine humaine et en médecine vétérinaire, impliquant l'utilisation d'une quantité efficace d'au moins un composé tel que défini dans l'une des revendications 1 à 7, ou d'un médicament tel que défini dans l'une des revendications 12 à 14.
